# EUROPÄISCHE PATENTSCHRIFT

(11) **EP 4 482 466 B1**
(45) Veröffentlichungstag und Bekanntmachung des Hinweises auf die Patenterteilung: **05.11.2025**
(21) Anmeldenummer: 23716787.9
(22) Anmeldetag: 29.03.2023
(51) Int. Cl.: A61K 9/00, A61K 9/24, A61K 31/00

(54) **KOMBINATIONSTHERAPEUTIKUM**
COMBINED THERAPEUTIC AGENT
AGENT THÉRAPEUTIQUE COMBINÉ

(30) Priorität: 14.06.2022 DE 102022002132; 16.09.2022 DE 102022123723
(43) Veröffentlichungstag der Anmeldung: 01.01.2025
(73) Patentinhaber: Maria Clementine Martin Klosterfrau Vertriebsgesellschaft mbH, 50670 Köln (DE)
(72) Erfinder: GALÁN-SOÚSA, José, 50670 Köln (DE)
(74) Vertreter: Strehlke, Ingo Kurt
(86) Internationale Anmeldenummer: PCT/EP2023/058160
(87) Internationale Veröffentlichungsnummer: WO 2023/241838

(56) Entgegenhaltungen:
- WO-A1-2016/137411
- WO-A1-2021/263132
- US-A1- 2019 350 859

## Beschreibung

Die vorliegende Erfindung betrifft das Gebiet der Medizin und insbesondere das technische (d.h. medizinisch-pharmazeutische) Gebiet der Erkrankungen des Mund/Rachen-Raums, vorzugsweise der insbesondere schmerzbegleiteten entzündlichen Erkrankungen des Mund/Rachen-Raums.

Insbesondere betrifft die vorliegende Erfindung eine Darreichungsform, insbesondere pharmazeutische Darreichungsform, insbesondere für die orale Applikation, welche sich für die prophylaktische bzw. therapeutische Behandlung von insbesondere schmerzbegleiteten entzündlichen Erkrankungen des Mund/Rachen-Raums eignet bzw. hierbei verwendet wird.

Die vorliegende Erfindung betrifft darüber hinaus auch ein Verfahren zur Herstellung einer Darreichungsform, insbesondere pharmazeutischen Darreichungsform, insbesondere für die orale Applikation, wobei die Darreichungsform insbesondere zur Verwendung bei der prophylaktischen bzw. therapeutischen Behandlung von insbesondere schmerzbegleiteten entzündlichen Erkrankungen des Mund/Rachen-Raums vorgesehen ist.

Im Rahmen der vorliegenden Erfindung verhält es sich dabei insbesondere derart, dass eine spezielle Wirkstoffkombination auf Basis von Ibuprofen bzw. dessen pharmazeutisch verträglichen Salzen und Estern, bevorzugt Ibuprofen, einerseits und Lidocain bzw. dessen pharmazeutisch verträglichen Salzen und Estern, bevorzugt Lidocainhydrochlorid, andererseits bereitgestellt wird, und zwar in der sehr speziellen erfindungsgemäßen Darreichungsform, welche nämlich als mehrphasige, insbesondere mindestens zweiphasige, vorzugsweise zweiphasige Tablette, besonders bevorzugt als Zweischicht-Tablette, ausgebildet ist, insbesondere wie nachfolgend im Detail angeführt.

Im Allgemeinen treten entzündliche Erkrankungen des Mund/Rachen-Raums, d. h. Entzündungen im Bereich der Mundhöhle, des Rachens und des Halses bzw. im Mund- und Hals/Rachen-Raum, oftmals als Begleiterscheinung beispielsweise von Erkältungskrankheiten und grippalen Infekten sowie von Grippe (Influenza) aber auch als eigenständige Erkrankungen auf.

Nichtbeschränkende Beispiele für insbesondere schmerzbegleitete entzündliche Erkrankungen des Mund/Rachen-Raums (d. h. des Mundraums bzw. des Hals/Rachen-Raums) sind in diesem Zusammenhang Halsentzündungen, insbesondere Angina, Entzündungen des Kehlkopfs (Laryingitis), Entzündungen der Rachenschleimhaut (Pharyngitis) und Entzündungen der Rachenmandeln (Angina tonsillaris bzw. Tonsillitis). Auch Entzündungen im Bereich der Mundhöhle, wie z. B. Stomatitis, Gingivitis, Mundschleimhautläsionen, wie Aphthen, oder dergleichen, zählen zu den sogenannten Erkrankungen des Mund/Rachen-Raums.

Für weitergehende diesbezügliche Einzelheiten zu den vorgenannten Erkrankungen kann beispielsweise auf Roche-Lexikon Medizin, 3. Auflage, 1993, Urban & Schwarzenberg, München/Wien, Baltimore, sowie auf Pschyrembel, medizinisches Wörterbuch, 257. Auflage, 1993, Nikol Verlagsgesellschaft mbH, Hamburg, verwiesen werden.

Entzündliche Erkrankungen des Mund/Rachen-Raums, insbesondere wie zuvor angeführt, gehen oftmals mit einer unangenehmen, das Wohlbefinden beeinflussenden bzw. das Krankheitsgefühl verstärkenden Schmerz- und Entzündungssymptomatik einher, wobei neben schmerzhaften Schleimhautläsionen bzw. -entzündungen beispielsweise auch schmerzhafte Schluckbeschwerden oder dergleichen auftreten können, was nicht zuletzt auch die Aufnahme von Nahrung und die Abfuhr von Speichel erschwert. Entzündliche Erkrankungen des Mund/Rachen-Raums gehen auch oftmals mit einer Beeinträchtigung der Phonation einher, so dass auch eine Heiserkeit vorliegen kann, welche gleichermaßen als äußerst störend bzw. unangenehm und oftmals als schmerzhaft empfunden wird. Auch Verletzungen im Mund/Rachen-Raum, insbesondere im Bereich der dortigen Schleimhäute, können mit Schmerzen einhergehen.

Im Allgemeinen sind Halsschmerzen ein häufig auftretendes Symptom, und zwar auch im Zusammenhang mit entzündlichen Erkrankungen des Mund/Rachen-Raums. Häufig liegt dabei eine akute Infektion insbesondere im Rachenbereich vor, wie es beispielsweise für eine akute Pharyngitis der Fall ist. Dabei kann die zugrundeliegende akute Infektion viralen Ursprungs sein, wobei jedoch auch bakteriell hervorgerufene bzw. begleitete Infektionen vorliegen können. Die Entzündung kann beispielsweise mit einem grippalen Infekt oder dergleichen einhergehen. Im Allgemeinen können auch Infektionen des Mundraums sowie des Hals/Rachen-Raums zu entsprechenden Schmerzsymptomen führen bzw. hiermit einhergehen.

Infolge der Infektion zeigt sich insbesondere eine Rötung und Schwellung der Schleimhäute, welche oftmals von Schmerzen, Schluckbeschwerden und einem Räusperzwang begleitet sind, wobei oftmals auch Heiserkeit auftritt, wie zuvor angeführt. Darüber hinaus kann es insbesondere im Rahmen einer oftmals bakteriell bedingten Superinfektion auch zu einer akuten Mandelentzündung kommen. Halsschmerzen können zudem auch bei schwerwiegenderen Erkrankungen, wie z. B. Scharlach, EBV-Infektionen (Pfeiffersches Drüsenfieber), Influenza oder Diphtherie auftreten.

Oftmals beschränkt sich die Behandlung von entzündlichen Erkrankungen des Mund/Rachen-Raums bzw. der damit einhergehenden Schmerzen auf die Gabe von sogenannten "Hausmitteln", wie beispielsweise heißer Milch mit Honig oder Gurgellösungen auf Basis von Kochsalz oder Salbei. Derartige Behandlungsmethoden gewährleisten jedoch oftmals keine zufriedenstellende Linderung der Beschwerden. Einige der bekannten Hausmittel, wie insbesondere heiße Milch mit Honig, sind sogar abträglich, da sie eine verstärkte Schleimbildung im Hals bewirken, was aber dem Heilungsprozess bzw. der Regeneration entgegenstehen kann.

Im Stand der Technik liegt ein Schwerpunkt bei der Behandlung von entzündlichen Erkrankungen des Mund/Rachen-Raums bzw. hiermit einhergehender Schmerzen - insbesondere in Abhängigkeit von der Schwere des Krankheitsbildes bzw. der Erkrankung - zudem in der topischen Therapie bzw. in der topischen Applikation entsprechender Wirkstoffe. Die Wirkstoffe können beispielsweise in Form von Rachenspülungen, Sprays oder lutschbaren Zusammensetzungen verabreicht werden.

Insbesondere kommen häufig handelsübliche lutschbare Zusammensetzungen zum Einsatz, welche mitunter zwar eine kurzzeitige Linderung verschaffen können, deren Wirkung jedoch üblicherweise äußerst schnell nachlässt. Zudem gehen die im Stand der Technik bekannten Zusammensetzungen zur Behandlung von insbesondere schmerzbegleiteten entzündlichen Erkrankungen des Mund/Rachen-Raums oftmals mit einer zwar zeitlich begrenzten, dennoch äußerst unangenehmen Veränderung des Geschmackssinns bzw. mit Geschmacksirritationen einher. Zudem kann es zu Verfärbungen an Zähnen kommen. Auch liegt mitunter kein Fokus auf eine kausale Schmerz- bzw. Entzündungstherapie.

Als topisches Antiseptikum bzw. Desinfektionsmittel für die Mund-, Hals- und Rachenschleimhaut wird oftmals die Wirksubstanz 1,3-Bis(2-ethylhexyl)-hexahydro-5-methyl-5-pyridinamin (internationaler Freiname: "Hexetidin") verabreicht. Bei längerer Anwendung und starker Dosierung kann es insbesondere zu Magen/Darm-Beschwerden und darüber hinaus zu Geschmacksirritationen sowie zu Verfärbungen an den Zähnen kommen, wobei zudem auch keine unmittelbare entzündungshemmende bzw. schmerzlindernde Wirkung vorliegt.

Im Stand der Technik kommt zur topischen Behandlung von entzündlichen Prozessen bzw. Erkrankungen des Mund/Rachen-Raums beispielsweise der Wirkstoff 1-Hexadecylpyridiniumchlorid (internationaler Freiname: "Cetylpyridiniumchlorid" (CPC)) zum Einsatz, wobei es sich hierbei um eine quartäre Ammoniumverbindung mit bakterizider und fungizider Wirkung handelt. Dieser Wirkstoff kommt unter anderem in Form von lutschbaren Zusammensetzungen zum Einsatz. Bei hoher Dosierung und übermäßiger systemischer Aufnahme des Wirkstoffs kann es jedoch zu unerwünschten Magen/Darm-Beschwerden, Atemnot sowie zu einer vermehrten Methämoglobinbildung, insbesondere bei Kindern, kommen. Zudem liegt nicht immer eine ausreichende Wirksamkeit und Wirkdauer vor. Insbesondere kann auf dieser Basis die Schmerz- und Entzündungssymptomatik nur begrenzt behandelt bzw. verringert werden.

Darüber hinaus kommt zur Behandlung der in Rede stehenden entzündlichen Erkrankungen des Mund/Rachen-Raums oftmals auch der Wirkstoff Flurbiprofen zum Einsatz, und zwar insbesondere in Form von lutschbaren Zusammensetzungen. Flurbiprofen weist dabei zwar grundsätzlich schmerzlindernde Eigenschaften auf, jedoch liegen nicht immer zufriedenstellende Wirkeigenschaften vor, und zwar auch im Hinblick auf eine unzureichende Wirkeffizienz und Wirkdauer. Zudem kann es auch hier zu Nebenwirkumgen in Form von Störungen im Magen/Darm-Bereich kommen, wie Bauchschmerzen, Übelkeit oder dergleichen.

Insgesamt gehen die zuvor genannten und insbesondere topisch verabreichten Wirkstoffe, nämlich Cetylpyridiniumchlorid und Flurbiprofen, im Rahmen ihrer lokalen Anwendung bzw. ihres lokalen Einsatzes zur lokalen Behandlung von entzündlichen Erkrankungen des Mund/Rachenraums nicht immer mit einem optimalen Therapieerfolg einher, und zwar auch was die Verbesserung der zugrundeliegenden Schmerz- bzw. Entzündungssymptome anbelangt. Zwar tritt bei den vorgenannten Wirkstoffen mit der diesbezüglich topischen bzw. lokalen Verabreichung mitunter ein relativ schneller Wirkeintritt nach Applikation einher, jedoch ist die Wirkdauer sowie die Wirkeffizienz bzw. -stärke mitunter nicht optimal.

Was die topische bzw. lokale Verabreichung von Wirkstoffen zur Behandlung von insbesondere schmerzbegleiteten entzündlichen Erkrankungen des Mund/Rachen-Raums weiterhin anbelangt, so ist es auf dieser Basis oftmals nicht möglich, ein breites Wirkspektrum insbesondere im Hinblick auf eine gleichzeitige Behandlung entzündlicher Prozesse sowie einer schmerzstillenden bzw. analgetischen bzw. (lokal-)anästhetischen Wirkung zu ermöglichen.

Darüber hinaus wird im Stand der Technik insbesondere bei schwereren Erkrankungsverläufen der Einsatz systemischer Wirkstoffe in Betracht gezogen.

So kommen beispielsweise systemische Wirkstoffe mit analgetischen, antipyretischen bzw. antiphlogistischen Wirkeigenschaften zum Einsatz, wie beispielsweise Acetylsalicylsäure, Ibuprofen sowie Paracetamol oder dergleichen. Auf dieser Basis kann zwar eine gewisse Schmerzlinderung erreicht werden, jedoch liegt diesbezüglich aufgrund der vorrangig systemischen Wirkweise oftmals ein verzögerter Wirkeintritt vor, so dass eine schmerzstillende Wirkung erst nach einem längeren Zeitraum nach Einnahme einsetzt, beispielsweise erst nach mehreren Stunden nach Beginn der Verabreichung, wobei zudem auch die Wirkdauer nicht immer zufriedenstellend ist. Insbesondere kann auf Basis systemisch wirkender Substanzen der lokale Entzündungsreiz im Mund/Rachen-Raum oftmals nicht zufriedenstellend verringert bzw. behandelt werden, und zwar auch vor dem Hintergrund, dass die Menge bzw. Dosis des systemisch aufgenommenen Wirkstoffs am lokalen Wirkort mitunter nicht ausreichend bzw. nicht optimal ist. Darüber hinaus können bei länger andauernder Verabreichung sowie höheren Mengen bzw. höherer Dosierung auch systemische Nebenwirkungen, wie Magen/Darm-Probleme, auftreten.

Auch vor diesem Hintergrund hat es im Stand der Technik weitere Ansätze gegeben, welche auf eine topische Verabreichung von lokal wirksamen Schmerzmitteln bzw. Lokalanästhetika, wie beispielsweise Polydocanol, Benzocain, Lidocain sowie Ambroxol oder dergleichen, abzielen. Diesbezüglich liegt aber oftmals keine ausreichende entzündungshemmende Wirkung vor, und auch der lokalanästhetische Effekt ist oftmals unzureichend, so dass es nicht immer zu einer nachhaltigen Schmerzlinderung kommt. Derartige Lokalanästhetika zeichnen sich zwar durch einen relativ schnellen Wirkeintritt nach deren Applikation aus, jedoch ist das Wirkprofil sowohl hinsichtlich der Wirkeffizienz bzw. -stärke sowie der Wirkdauer mitunter nicht optimal..

Darüber hinaus hat es auch nicht an Versuchen gefehlt, Kombinationszusammensetzungen bzw. -präparate bereitzustellen. So betrifft die WO 2016/137411 A1 eine topische, für die rein äußerliche Anwendung vorgesehene und insbesondere in flüssiger Form bzw. als Sprühformulierung vorliegende pharmazeutische Zusammensetzung auf Basis von Ibuprofen und Lidocain. Dabei wird vorrangig auf die Behandlung von Muskelschmerzen und Verstauchungen sowie insbesondere nach Sportverletzungen auftretenden Rückenschmerzen und darüber hinaus auch auf eine Anwendung im Rahmen der Wundbehandlung abgestellt. Zwar wird beiläufig unter anderem auch auf Halsschmerzen hingewiesen, dies jedoch ohne weiterführenden Fokus und ohne zielgerichtete Optimierung der Formulierung, welche vielmehr universell eingesetzt werden soll. Darüber hinaus wird nur auf den Einsatz relativ geringer Mengen bzw. Dosen an Ibuprofen abgestellt, wobei auch keine weiterführende (Wirk-) Abstimmung mit der weiteren Wirkkomponente vorliegt. Insbesondere fehlt es an einer kontrollierten Freisetzung des Lidocains über einen längeren Zeitraum.

Die WO 2016/137411 A1 betrifft eine topische pharmazeutische Zusammensetzung, welche Ibuprofen oder pharmazeutisch akzeptable Salze oder Enantiomere davon und Lidocain oder pharmazeutisch akzeptable Salze davon in Kombination mit mindestens einem pharmazeutisch akzeptablen Hilfsstoff umfasst, wobei die Zusammensetzung maßgeblich als Spray ausgebildet sein soll.

Weiterhin betrifft die US 2019/350859 A1 eine orale Tablette, welche für pharmazeutische Wirkstoffe geeignet ist, umfassend eine Population von Teilchen, wobei die Population von Teilchen direkt komprimierbare (DC) und nicht direkt komprimierbare (nicht-DC) Zuckeralkoholteilchen umfasst, wobei die nicht-DC-Teilchen die Tablette mit einer Vielzahl von diskreten nicht-DC-Bereichen versehen und die nicht-DC-Bereiche beim Kauen der Tablette zu einer induzierten Speichelproduktion führen.

Die WO 2021/263132 A1 betrifft eine schnell orodispergierbare Dosierungsform, umfassend einen porösen, dauerhaften Körper, der ein gebundenes Pulvermaterial umfasst, wobei der Körper einen oder mehrere innere Hohlräume aufweist, wobei das gebundene Pulvermaterial vorzugsweise eine miteinander verbundene Matrix aus mindestens einem einnehmbaren Pulvermaterial und mindestens einem einnehmbaren Bindemittelmaterial umfasst.

Insgesamt besteht im Stand der Technik ein großer Bedarf an der Bereitstellung eines effizienten Konzepts zur prophylaktischen bzw. therapeutischen Behandlung von insbesondere schmerzbegleiteten entzündlichen Erkrankungen des Mund/Rachen-Raums, wobei bei gleichzeitig hoher Wirkeffizienz bzw. -stärke auch eine möglichst schnell einsetzende und langanhaltende Wirkung insbesondere im Hinblick auf eine Schmerzlinderung und eine Verbesserung der Entzündungssymptome bei gleichzeitig guter Verträglichkeit bzw. geringen Nebenwirkungen gewährleistet sein soll. Dabei soll auch eine leichte und sichere Anwendung bzw. Applikation (d. h. eine insbesondere einfache und Fehldosierungen vermeidende Verabreichung) ermöglicht werden, und dies bei gleichzeitig guten bzw. verbesserten organoleptischen Eigenschaften. Nicht zuletzt sollen entsprechende Zusammensetzungen auch eine hohe Stabilität, insbesondere Lagerstabilität, aufweisen.

Vor dem Hintergrund des zuvor geschilderten Standes der Technik liegt der vorliegenden Erfindung somit die Aufgabe zugrunde, ein effizientes Konzept bzw. eine diesbezügliche Darreichungsform bereitzustellen, welche(s) eine effiziente Behandlung von insbesondere schmerzbegleiteten entzündlichen Erkrankungen des Mund/Rachen-Raums ermöglicht, wobei in diesem Zusammenhang die zuvor angeführten Nachteile des Standes der Technik überkommen oder zumindest abgeschwächt werden sollen.

Insbesondere liegt der vorliegenden Erfindung die Aufgabe zugrunde, entsprechende Darreichungsformen bereitzustellen, welche im Rahmen der Behandlung von insbesondere schmerzbegleiteten entzündlichen Erkrankungen des Mund/Rachen-Raums eine hervorragende Wirkeffizienz sowie durch einen schnellen Wirkeintritt und eine langanhaltende Wirkdauer bei gleichzeitig guter Verträglichkeit aufweisen. Dabei soll auch eine einfache und sichere Anwendung gewährleistet sein.

Eine weiterführende Aufgabe der vorliegenden Erfindung ist vor diesem Hintergrund insbesondere auch darin zu sehen, bei der Anwendung der erfindungsgemäß bereitgestellten Darreichungsform eine in Bezug auf die zugrundeliegenden insbesondere schmerzbegleiteten entzündlichen Erkrankungen des Mund/Rachen-Raums nachhaltige (d.h. schnell einsetzende und lang andauernde) Verringerung der zugrundeliegende Schmerzsymptomatik zu ermöglichen, wobei weiterführend auch die mit den Erkrankungen einhergehenden entzündlichen Prozesse abgeschwächt bzw. verringert werden sollen. Insbesondere soll erfindungsgemäß ein effizientes Konzept zur wirksamen Schmerz- bzw. Entzündungstherapie bei den zugrundeliegenden Erkrankungen des Mund/Rachen-Raums bereitgestellt werden.

Folglich soll im Rahmen der der vorliegenden Erfindung zugrundeliegenden Aufgabe eine spezielle Darreichungsform zur Behandlung von insbesondere schmerzbegleiteten entzündlichen Erkrankungen des Mund/Rachen-Raums bereitgestellt werden, wobei die Zusammensetzung bei einfacher und sicherer Anwendung bzw. Handhabbarkeit über hervorragende schmerzlindernde Eigenschaften bzw. entzündungshemmende Eigenschaften verfügen soll, wobei zudem ein rascher Wirkeintritt nach Verabreichung bei gleichzeitig verlängerter bzw. langanhaltender Wirkdauer gewährleistet sein soll. Dabei soll auch das Auftreten von Nebenwirkungen verringert und zudem eine stabile, insbesondere lagerstabile, Zusammensetzung bzw. Darreichungsform bereitgestellt werden.

Eine nochmals weitere Aufgabe der vorliegenden Erfindung ist zudem auch darin zu sehen, ein entsprechendes Verfahren zur Herstellung der erfindungsgemäßen Darreichungsform bereitzustellen, wobei das Verfahren bei einfacher Verfahrensführung zu entsprechenden Darreichungsformen mit optimierten Produkt- bzw. Wirkeigenschaften führen soll.

In vollkommen überraschender Weise hat die Anmelderin nunmehr gefunden, dass sich eine sehr spezielle Darreichungsform, insbesondere zur oralen Applikation, in hervorragender Weise für die prophylaktische bzw. therapeutische Behandlung von insbesondere schmerzbegleiteten entzündlichen Erkrankungen des Mund/Rachen-Raums eignet, und zwar insbesondere im Hinblick auf eine schmerzstillende und/oder entzündungsverringernde Wirkung, und dies bei raschem Wirkeintritt und langer Wirkdauer. Dabei weist die erfindungsgemäße Darreichungsform eine spezielle Kombination und Anordnung mindestens eines NSAR-Wirkstoffs, bevorzugt Ibuprofen, und mindestens eines Lokalanästhetikums, bevorzugt Lidocainhydrochlorid, auf. Dabei ist es erfindungsgemäß insbesondere vorgesehen, dass die erfindungsgemäß bereitgestellte Darreichungsform als mehrphasige und insbesondere mindestens zweiphasige, vorzugsweise zweiphasige Tablette ausgebildet ist, und zwar besonders bevorzugt als Zweischicht-Tablette, wobei die mehrphasige Tablette bzw. Zweischicht-Tablette mindestens zwei voneinander verschiedene Phasen bzw. Schichten mit bei oraler Applikation voneinander verschiedenen Zerfallszeiten bzw. Wirkstofffreisetzungsgeschwindigkeiten aufweist bzw. hieraus besteht. Dabei verhält es sich erfindungsgemäß insbesondere derart, dass eine erste Phase bzw. erste Schicht der erfindungsgemäßen Darreichungsform als orodispersible Phase bzw. orodispersible Schicht mit schnellem bzw. unmittelbarem Zerfall bzw. Wirkstofffreisetzung ausgebildet ist, wobei diese erste Phase bzw. erste Schicht den speziellen NSAR-Wirkstoff, wie nachfolgend definiert, aufweist. Zudem verhält es sich erfindungsgemäß insbesondere derart, dass die zweite Phase bzw. zweite Schicht der erfindungsgemäßen Darreichungsform als oral-transmukosale Phase bzw. oral-transmukosale Schicht mit langsamem bzw. retardiertem Zerfall bzw. Wirkstofffreisetzung ausgebildet ist, wobei die zweite Phase bzw. zweite Schicht das spezielle Lokalanästhetikum, wie nachfolgend beschrieben, aufweist.

Zur Lösung der zuvor geschilderten Problemstellung schlägt die vorliegende Erfindung somit - gemäß einem **ersten** Aspekt der vorliegenden Erfindung - eine Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, gemäß Anspruch 1, vor. Jeweils vorteilhafte Weiterbildungen und Ausgestaltungen dieses Erfindungsaspekts sind Gegenstand der diesbezüglichen Neben- bzw. Unteransprüche.

Ebenfalls Gegenstand der vorliegenden Erfindung - gemäß einem **zweiten** Aspekt der vorliegenden Erfindung - ist zudem das Verfahren nach der Erfindung zur Herstellung einer Darreichungsform, insbesondere pharmazeutischen Darreichungsform, gemäß dem das Herstellungsverfahren betreffenden unabhängigen Anspruch. Jeweils vorteilhafte Weiterbildungen und Ausgestaltungen dieses Erfindungsaspekts sind Gegenstand der betreffenden Unteransprüche.

Es versteht sich von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Hinblick auf das herangezogene Bezugssystem (z. B. Darreichungsform bzw. zugrundeliegende Phasen bzw. Schichten) vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Darüber hinaus gilt für die Beschreibung der vorliegenden Erfindung, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Insbesondere gilt für die die Erfindung charakterisierenden Merkmale zudem, dass auch beliebige Kombinationen dieser Merkmale als offenbart gelten, wobei Ausführungsformen gleicher Präferenz der verschiedenen Merkmale in ihrer Kombination bevorzugt sind (z.B. Mengen bzw. Mengenbereiche der betreffenden Wirk- und Inhaltsstoffe gleicher Präferenz, z.B. Zerfallszeiten bzw. Wirkstofffreisetzungsgeschwindigkeiten gleicher Präferenz sowie z.B. diesbezügliche Kombinationen von Mengen bzw. Mengenbereiche der betreffenden Wirk- und Inhaltsstoffe und Zerfallszeiten bzw. Wirkstofffreisetzungsgeschwindigkeiten gleicher Präferenz).

Weiterhin gilt in diesem Zusammenhang insbesondere auch, dass in Bezug auf die nachfolgend angeführten, die verschiedenen Inhaltsstoffe, insbesondere Wirkstoffe, der erfindungsgemäßen Darreichungsform betreffenden Mengenangaben, insbesondere relativen Mengenangaben oder absoluten Mengenangaben, jeweilige und die verschiedenen Inhaltsstoffe, insbesondere Wirkstoffe, betreffende Kombinationen gleicher Präferenz bzw. gleicher Bevorzugungsebene mit gleicher Präferenz bzw. Bevorzugung mitoffenbart sind. Ebenfalls sind auch sämtliche anderweitige Kombinationen (d.h. Kombinationen auf Basis unterschiedlicher Präferenzen bzw. unterschiedlicher Bevorzugungsebenen) mitoffenbart.

Dies vorausgeschickt, wird die vorliegende Erfindung nunmehr nachfolgend im Detail erläutert:
Gegenstand der vorliegenden Erfindung - gemäß einem **ersten** Aspekt der vorliegenden Erfindung - ist somit eine Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

In diesem Zusammenhang betrifft die vorliegende Erfindung insbesondere auch eine Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schnellem und/oder unmittelbarem (sofortigem) Zerfall und/oder Wirkstofffreisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
   - wobei eine zweite Schicht als oral-transmukosale Schicht (Lutschtabletten-Schicht) mit langsamem und/oder retardiertem (kontrolliertem) Zerfall und/oder Wirkstofffreisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

Im Rahmen der vorliegenden Erfindung ist es auf Grundlage der erfindungsgemäßen Darreichungsform mit der speziellen Ausbildung als mehrphasige, insbesondere mindestens zweiphasige, vorzugsweise zweiphasige, Tablette und mit spezieller Wirkstoffzuordnung - wonach nämlich der spezielle NSAR-Wirkstoff (nur) in der ersten Phase bzw. ersten Schicht mit schnellem bzw. unmittelbarem Zerfall und/oder Wirkstofffreisetzung vorliegt und wonach das spezielle Lokalanästhetikum (nur) in der zweiten Phase bzw. zweiten Schicht mit langsamem bzw. retardiertem Zerfall und/oder Wirkstofffreisetzung vorliegt - in völlig überraschender Weise gelungen, einen insgesamt optimierten Zerfall und auf die Wirkstoffe speziell abgestimmte Wirkstofffreisetzung zu ermöglichen. Hierdurch kann insgesamt eine besonders effiziente schmerz- bzw. entzündungsspezifische Behandlung von insbesondere schmerzbegleiteten entzündlichen Erkrankungen des Mund/Rachen-Raums erfolgen.

In diesem Zusammenhang wird im Rahmen der vorliegenden Erfindung nämlich sowohl ein schneller Wirkeintritt als auch eine langanhaltende Wirkdauer gewährleistet, und dies sowohl in Bezug auf eine Linderung von Schmerzen als auch im Hinblick auf eine Verringerung von Entzündungsprozessen, wie sie mit den zugrundeliegenden Erkrankungen einhergehen.

Erfindungsgemäß verhält es sich im Hinblick auf die Darreichungsform insbesondere derart, dass bei deren orale Applikation (d. h. bei Kontakt der Darreichungsform mit Flüssigkeit, insbesondere Speichel) eine schnelle bzw. unmittelbare Freisetzung des NSAR-Wirkstoffs aus der ersten Phase bzw. ersten Schicht, welche hierfür in orodispersibler Form ausgebildet ist, vorliegt. Zudem liegt eine langsame bzw. retardierte Freisetzung des Lokalanästhetikums aus der zweiten Phase bzw. zweiten Schicht vor, welche hierfür in oral-transmukosaler Form ausgebildet ist. Auf dieser Basis wird insgesamt eine das jeweilige Wirkstoffprofil bzw. die zugrundeliegende Wirkweise der jeweiligen Wirkstoffe berücksichtigende Gesamtfreisetzung in Abstimmung zueinander ermöglicht, was zu den verbesserten (Gesamt-)Wirkeigenschaften führt.

Ohne sich auf diese Theorie beschränken oder berufen zu wollen, verhält es sich erfindungsgemäß dabei insbesondere derart, dass der schnelle bzw. unmittelbare Zerfall der ersten Phase bzw. ersten Schicht mit der schnellen Wirkstofffreisetzung zu einer raschen und insbesondere systemischen Aufnahme (insbesondere durch Herunterschlucken) des im Allgemeinen systemisch wirkenden NSAR-Wirkstoffs führt, so dass eine relativ schnell einsetzende systemische Wirkung auf Basis des NSAR-Wirkstoffs erfolgen kann, welche zudem langanhaltend ist. Durch die Freisetzung des NSAR-Wirkstoffs bereits im Mund kann - gleichermaßen ohne sich auf diese Theorie berufen oder beschränken zu wollen, zudem in einem gewissen Maß eine lokale schmerzstillende und/oder entzündungshemmende durch den NSAR-Wirkstoff Wirkung im Mund/Rachen-Raum vorliegen bzw. hervorgerufen werden. Denn der NSAR-Wirkstoff kann - ohne sich auf diese Theorie beschränken oder berufen zu wollen - (in geringen Mengen) grundsätzlich auch lokal durch Schleimhäute bzw. entzündetem Gewebe aufgenommen werden. So kann zudem eine (lokale) Wirkung entfaltet werden bzw. vorliegen, welche die (vorrangig) systemische Wirkung des NSAR-Wirkstoffs ergänzen kann.

Weiterhin führt der abgestimmte langsame bzw. retardierte Zerfall der zweiten Phase bzw. zweiten Schicht nach oraler Applikation der Darreichungsform und bei Kontakt mit Flüssigkeit, insbesondere Speichel, zu einer langsamen bzw. bzw. retardierten und insbesondere über einen langen Zeitraum gleichmäßigen Freisetzung des insbesondere topisch bzw. lokal wirkenden Lokalanästhetikums unmittelbar am Wirkort, was gleichermaßen insbesondere im Hinblick auf eine Schmerzlinderung zu einer verlängerten Wirkdauer bei gleichzeitig rasch einsetzendem Wirkeintritt führt. Erfindungsgemäß verhält es sich insbesondere derart, dass das Lokalanästhetikum über einen längeren Zeitraum nach oraler Aufnahme der erfindungsgemäßen Darreichungsform mit dem langsameren Zerfall Wirkstofffreisetzung aus der zweiten bzw. oral-transmukosalen Phase freigesetzt wird, so dass eine entsprechend langanhaltende lokalanästhetische Wirkung vorliegt, insbesondere infolge der Aufnahme des Lokalanästhetikums im Bereich der Schleimhäute im Mund/Rachen-Raum bzw. des diesbezüglichen Gewebes. Insbesondere wird der Zerfall der zweiten Phase bzw. die Wirkstofffreisetzung aus der zweiten Phase gleichermaßen durch Kontakt auch der zweiten Phase mit Flüssigkeit, insbesondere Speichel, nach oraler Applikation der Darreichungsform hervorgerufen.

Insbesondere liegt auch am Wirkort selbst aufgrund der aufeinander abgestimmten Freisetzung die Wirkstoffe in Form des NSAR-Wirkstoffs einerseits und des Lokalanästhetikums andererseits ein optimal aufeinander abgestimmtes Wirkstoffverhältnis vor, und dies auch über einen langen Zeitraum, so dass eine optimale Ergänzung der Wirkeigenschaften vorliegt.

Im Rahmen der vorliegenden Erfindung ist es dabei insgesamt gelungen, die Wirkprofile und -eigenschaften des NSAR-Wirkstoffs einerseits und des Lokalanästhetikums andererseits durch eine differenzierte und aufeinander abgestimmte Freisetzung aus den jeweiligen Phasen bzw. Schichten der erfindungsgemäßen Darreichungsform in optimaler Beziehung zueinander zu stellen, um hierdurch eine insgesamt verbesserte Wirksamkeit bei der Behandlung von insbesondere schmerzbegleiteten entzündlichen Erkrankungen des Mund/Rachen-Raums zu gewährleisten, wobei diesbezüglich in überraschender Weise auch ein (Wirk-)Synergismus vorliegt.

Im Rahmen der vorliegenden Erfindung liegt insbesondere auch eine zu einer Wirkverbesserung führende Kombination der Wirkstoffe unter Einbezug der jeweiligen Pharmakodynamik und der jeweiligen Pharmakokinetik vor, wobei die jeweilige Wirkstofffreisetzung speziell hierauf angepasst und abgestimmt ist.

Erfindungsgemäß wird für die symptomatische Schmerzbehandlung bzw. Entzündungshemmung sowohl ein hierfür kausaler lokalanästhetischer und insgesamt rasch einsetzender Effekt als auch eine hierfür kausale und insbesondere verzögert einsetzende systemische Analgesie effektiv kombiniert. Dabei kann erfindungsgemäß zudem die Verzögerung des Wirkeintritts des NSAR-Wirkstoffs weiter vermindert werden, nämlich insbesondere durch die erfindungsgemäße Ausbildung der Darreichungsform, wonach die erste Phase mit dem NSAR-Wirkstoff als orodispersible Phase einen schnellen bzw. unmittelbaren Zerfall bzw. eine schnelle bzw. unmittelbare Wirkstofffreisetzung aufweist (was neben der systemischen Aufnahme des NSAR-Wirkstoffs auch eine schnelle lokale Wirkstoffresorption ermöglicht). Zudem liegt eine verlängerte Wirkung auch für das Lokalanästhetikum vor, nämlich dadurch, dass die zweite Phase bzw. Schicht als oral-transmukosale Phase bzw. Schicht mit langsamem bzw. retardiertem Zerfall bzw. Freisetzung ausgebildet ist.

Erfindungsgemäß kann dabei durch die erfindungsgemäße spezielle Kombination des NSAR-Wirkstoffs und des Lokalanästhetikums sozusagen eine sequentielle analgetische und lokalanästhetische Wirkung mit raschem Wirkeintritt (d. h. mit früherem Einsetzen der Wirkung) und mit insgesamt längerer Wirkdauer sowie mit weiterführender Wirkverstärkung bereitgestellt werden.

Erfindungsgemäß wird somit den Schmerz- und Entzündungszuständen des Mund/Rachen-Raums sozusagen multifaktoriell auf mehreren Ebenen begegnet und entgegengewirkt, nämlich durch eine unmittelbare lokal-symptomatische Behandlung des Schmerzzustands durch das Lokalanästhetikum, welche durch den retardierten bzw. kontrollierten Zerfall über einen verlängerten Zeitraum bewirkt wird, daneben durch eine unmittelbare lokal-analgetische Behandlung durch das Ibuprofen am Ort der Freisetzung (Mund/Rachen-Raum) und schließlich durch eine überwiegend kausal-systemische Behandlung durch das systemisch resorbierte Ibuprofen.

Insbesondere wird auf Basis der erfindungsgemäßen Darreichungsform mit der sehr speziellen Wirkstoffkombination und der gezielten unterschiedlichen Freisetzung der jeweiligen Wirkstoffe auch eine Vereinfachung hinsichtlich der Anwendung ermöglicht, so dass auch hierdurch die Verträglichkeit und Compliance vergrößert ist.

Zudem wird erfindungsgemäß durch die spezielle Wirkstoffabstimmung mit den speziellen Freisetzungsprofilen auch eine entsprechende Optimierung bzw. Verringerung der jeweiligen Dosen an zu verabreichendem NSAR-Wirkstoff einerseits und Lokalanästhetikum andererseits ermöglicht, was mit einer nochmals verbesserten Verträglichkeit bzw. mit geringeren Nebenwirkungen einhergeht.

Im Rahmen der vorliegenden Erfindung wird insgesamt somit eine Darreichungsform mit voneinander verschiedenen Phasen bzw. Schichten mit jeweils speziell abgestimmtem Zerfall bzw. Wirkstofffreisetzung und spezieller Wirkstoffzuordnung bereitgestellt, welche insgesamt für die orale Applikation und Wirkstofffreisetzung insbesondere im Mund/Rachen-Raum, insbesondere im Mundbereich, optimiert ist, wobei der Zerfall bzw. die Wirkstofffreisetzung durch den Kontakt mit Flüssigkeit und insbesondere mit Speichel induziert bzw. hervorgerufen wird.

Erfindungsgemäß ist, wie zuvor angeführt, die erste Phase der Darreichungsform im Speziellen als orodispersible Phase bzw. Schmelztabletten-Phase ausgebildet. Auch auf dieser Basis ist ein schneller Zerfall mit schneller Wirkstofffreisetzung gewährleistet. Weiterführend ist die zweite Phase bzw. Schicht als oral-transmukosale Phase bzw. Lutschtabletten-Phase ausgebildet. Hierdurch ist insgesamt gewährleistet, dass ein Zerfall bzw. eine Auflösung der Darreichungsform insgesamt im Bereich des Mundes erfolgen kann, so dass ein Schlucken der nicht zerfallenen Darreichungsform bzw. eine diesbezügliche Einnahme mit Wasser nicht erforderlich ist. Dies verbessert nochmals die Compliance und bietet entsprechende Vorteile bei der Anwendung der erfindungsgemäßen Darreichungsform.

Im Rahmen der vorliegenden Erfindung werden nicht zuletzt auch durch die nachfolgend noch beschriebene Verpressung der Wirk- bzw. Inhaltsstoffe zur Ausbildung der Darreichungsform bzw. der Phasen und/oder den Einsatz des NSAR-Wirkstoffs in Granulatform mit gegebenenfalls vorliegender Beschichtung insgesamt auch besonders abriebfeste und stabile, insbesondere lagerstabile, Darreichungsformen mit definiertem Zerfall bzw. Wirkstofffreisetzung in Bezug auf die jeweiligen Phasen bzw. Schichten bereitgestellt.

Der Begriff "Darreichungsform" bzw. "pharmazeutische Darreichungsform", wie er im Rahmen der vorliegenden Erfindung verwendet wird, ist dabei sehr breit zu verstehen und umfasst nicht nur pharmazeutische Präparate bzw. Pharmazeutika und Arzneimittel als solche, sondern auch sogenannte Medizinprodukte, Lebensmittel oder Nahrungsergänzungsmittel.

Weiterführend ist auch der Begriff "Mund/Rachen-Raum" im Rahmen der vorliegenden Erfindung breit zu verstehen. Insbesondere umfasst der Begriff den Bereich des Mundraums sowie den Hals/Rachen-Raum mit den diesbezüglichen biologischen Strukturen bzw. Geweben. Dabei umfasst der Bereich des Mundraums insbesondere den Bereich der Mundhöhle, und der Hals/Rachen-Raum umfasst den Hals- und Rachenbereich, insbesondere die Bereiche des Nasenrachens, Mundrachens sowie des Kehlkopfrachens, sowie weiterführend auch den Halsbereich und insbesondere den oberen Halsbereich.

Was den Begriff "orodispersibel" anbelangt, wie er erfindungsgemäß für die erste Phase, insbesondere erste Schicht, verwendet wird, so ist dieser Begriff im Rahmen der vorliegenden Erfindung breit zu verstehen. Insbesondere bezieht sich dieser Begriff auf die Eigenschaft der ersten Phase bzw. ersten Schicht, bei oraler Applikation, insbesondere in der Mundhöhle nach Kontakt mit Flüssigkeit, insbesondere Speichel, relativ schnell zu zerfallen, was mit einer gleichermaßen relativ schnellen Freisetzung des NSAR-Wirkstoffs, bevorzugt Ibuprofen, einhergeht. Dabei kann der Wirkstoff als solcher bzw. in Form partikulärer Strukturen bzw. in Teilchenform freigesetzt werden. Durch nachfolgendes Schlucken des freigesetzten NSAR-Wirkstoffs kann dieser systemisch aufgenommen werden, wobei grundsätzlich teilweise auch eine lokale Aufnahme bzw. Resorption durch und in das entzündete Gewebe erfolgen kann, wo dann eine unmittelbare Wirkung eintreten kann. Der maßgebliche Anteil des NSAR-Wirkstoffs wird jedoch systemisch resorbiert, insbesondere über den Gastrointestinaltrakt.

Was darüber hinaus den Begriff "oral-transmukosal", wie er erfindungsgemäß für die zweite Phase bzw. zweite Schicht verwendet wird, anbelangt, so ist dieser Begriff im Rahmen der vorliegenden Erfindung breit zu verstehen. Insbesondere bezieht sich dieser Begriff auf die Ausgestaltung der zweiten Phase bzw. Schicht derart, dass vorrangig eine langsame lokale Wirkstofffreisetzung mit einhergehender Wirkstoffaufnahme des Lokalanästhetikums im Mund/Rachen-Raum und insbesondere über die Schleimhäute ermöglicht wird, so dass hierdurch die lokalanästhetische Wirkung erreicht bzw. bewirkt wird. Dabei kann die zweite Phase bzw. Schicht insbesondere als Lutschtabletten-Schicht ausgebildet sein, einhergehend mit einem relativ langsamen bzw. retardierten Zerfall, insbesondere Auflösung, bzw. einer relativ langsamen bzw. retardierten Wirkstofffreisetzung. Durch die nahezu ausschließliche lokale Aufnahme des Wirkstoffs kann ein unerwünschter systemischer Wirkstoffabbau, beispielsweise im Gastrointestinaltrakt, bzw. eine Verstoffwechselung in der Leber (hepatischer First-Pass-Effekt) für das Lokalanästhetikum zumindest weitestgehend verringert bzw. verhindert werden.

Bei dem Wirkstoff Ibuprofen handelt es sich im Allgemeinen um einen Wirkstoff bzw. Arzneistoff aus der Gruppe der nichtsteroidalen Antirheumatika (NSAR-Wirkstoff), welcher eine Wirksamkeit im Hinblick auf die Behandlung von Schmerzen, Entzündungen und Fieber aufweist (analgetische, antiphlogistische und antipyretische Wirkung). Im Allgemeinen führt der Wirkstoff Ibuprofen zu einer nichtselektiven und reversiblen Inhibition der Cyclooxygenasen I und II (COX-1 und COX-2), was auch zu einer Verringerung der Bildung von entzündungsvermittelnden Prostaglandinen führt. Die Wirkung von Ibuprofen basiert dabei auf der Hemmung der Cyclooxygenasen. Im Allgemeinen liegt Ibuprofen als Racemat vor, wobei dem S-(+)-Enantiomer die pharmakologische Hauptwirkung zugesprochen wird. Zu weiteren Ausführungen zu Ibuprofen kann verwiesen werden auf Monographie Ibuprofen, Europäische Pharmakopöe (European Pharmacopoeia) (Ph. Eur.), 9. Auflage, Januar 2017*.* Zudem kann verwiesen werden auf RÖMPP Chemielexikon, 10. Auflage, Bd. 3, 1997, Georg Thieme-Verlag, Stuttgart/New York, Stichwort: "Ibuprofen" sowie auf die dort jeweils in Bezug genommene Literatur, deren gesamter Inhalt hiermit durch Bezugnahme vollumfänglich eingeschlossen ist.

Bei Lidocain handelt es sich um ein Lokalanästhetikum vom Amidtyp, insbesondere vom Aminoamidtyp, welches eine gute lokale Wirksamkeit bei gleichzeitig schnellem Wirkeintritt und guter Verträglichkeit aufweist. Lidocain blockiert reversibel spannungsabhängige Natriumkanäle in den Zellmembranen von Nervenzellen. Insbesondere hemmt Lidocain den Einstrom von Natriumionen über spannungsabhängige Natriumkanäle in die Nervenzellen, was zu einer verminderten Erregbarkeit von Nervenfasern führt, da der zur Ausbildung eines Aktionspotentials erforderliche Anstieg der Natriumpermeabilität verringert ist. Hierdurch wird das Schmerzempfinden herabgesetzt. Für weitere Ausführungen zu Lidocain kann beispielsweise auf RÖMPP Chemielexikon, 10. Auflage, Bd. 3, 1997, Georg Thieme-Verlag, Stuttgart/New York, Stichwort: "Lidocain" sowie auf die dort jeweils in Bezug genommene Literatur verwiesen werden, deren gesamter Inhalt hiermit durch Bezugnahme vollumfänglich eingeschlossen ist. Zudem kann verwiesen werden auf die Ausführungen in Lidocaine Hydrochloride, Europäische Pharmakopöe (European Pharmacopoeia) (Ph. Eur.), 9. Auflage (Januar 2017*).* Zudem kann in Bezug auf Lidocain auf die Ausführungen in European Pharmacopoeia 8.0, European Directorate for the Quality of Medicines and Healthcare, Seiten 2620/2621, Stichwort: "Lidocaine" verwiesen werden. Darüber hinaus kann in Bezug auf Lidocainhydrochlorid auf European Pharmacopoeia 8.0, European Directorate for the Quality of Medicines and Healthcare, Seiten 2620/2621, Stichwort: "Lidocaine Hydrochloride" verwiesen werden.

Nachfolgend wird die vorliegende Erfindung weiterführend und im Detail beschrieben, insbesondere im Hinblick auf weitere bevorzugte Ausgestaltungen bzw. bevorzugte Ausführungsformen.

Erfindungsgemäß ist der NSAR-Wirkstoff ausgewählt aus der Gruppe von Ibuprofen sowie dessen pharmazeutisch verträglichen Salzen und Estern. In bevorzugter Weise wird Ibuprofen verwendet.

Zudem ist das Lokalanästhetikum ausgewählt aus der Gruppe von Lidocain sowie dessen pharmazeutisch verträglichen Salzen und Estern. Gemäß einer erfindungsgemäß bevorzugten Ausführungsform wird das Lokalanästhetikum in Form von Lidocainhydrochlorid eingesetzt. Lidocainhydrochlorid weist im Vergleich zu Lidocain insbesondere eine höhere Wasserlöslichkeit auf. Hierdurch wird - ohne sich auf diese Theorie berufen oder beschränken zu wollen - die Wirkstofffreisetzung bei Kontakt mit Flüssigkeit bzw. Speichel nochmals verbessert, was auch zu verbesserten Wirkeigenschaften führt. Hinzu kommt, dass - gleichermaßen ohne sich auf diese Theorie berufen oder beschränken zu wollen - das mit dem vorliegenden Erkrankungen des Mund/Rachen-Raums vorliegende entzündete Gewebe, insbesondere infolge einer lokalen Lactatacidose, einen niedrigeren pH-Wert gegenüber normalen bzw. nichtentzündetem Gewebe aufweist. Auch von daher weist Lidocainhydrochlorid ein gutes Penetrationsvermögen auf, da diesbezüglich bereits die protonierte Form vorliegt.

Insbesondere kann es erfindungsgemäß vorgesehen sein, dass der NSAR-Wirkstoff Ibuprofen ist und dass das Lokalanästhetikum Lidocainhydrochlorid ist. Auf Basis dieser Kombination können besonders gute Wirksamkeiten erreicht werden, und dies sowohl im Hinblick auf einen schnellen Wirkeintritt als auch einer langen Wirkdauer. Wie zuvor angeführt, weist Lidocainhydrochlorid eine relativ hohe Wasserlöslichkeit auf, was der Wirkstofffreisetzung insbesondere im Mund zuträglich ist. Zudem liegt für Lidocainhydrochlorid eine hohe Bioverfügbarkeit im entzündeten Gewebe mit dem dort vorliegenden sauren pH-Wert vor, wie zuvor angeführt.

Insbesondere kann es sich erfindungsgemäß derart verhalten, dass die erste Phase, insbesondere die erste Schicht, Ibuprofen als NSAR-Wirkstoff aufweist und wobei die zweite Phase, insbesondere die zweite Schicht, Lidocainhydrochlorid als Lokalanästhetikum aufweist.

Weiterführend kann es sich erfindungsgemäß insbesondere auch derart verhalten, dass die erste Phase, insbesondere die erste Schicht, außer Ibuprofen keinen weiteren NSAR-Wirkstoff aufweist und dass die zweite Phase, insbesondere die zweite Schicht, außer Lidocainhydrochlorid kein weiteres Lokalanästhetikum aufweist. In diesem Zusammenhang fokussiert die vorliegende Erfindung folglich auf eine spezielle Optimierung und Abstimmung im Hinblick auf die Wirkprofile bzw. die zugrundeliegenden Pharmakokinetik bzw. -dynamik von Ibuprofen einerseits und Lidocainhydrochlorid andererseits.

Gemäß einer erfindungsgemäßen Ausführungsform kann der NSAR-Wirkstoff zudem Ibuprofenlysinat sein bzw. in Form von Ibuprofenlysinat vorliegen. Ibuprofenlysinat stellt dabei das Salz der Aminosäure Lysin mit Ibuprofen dar. Infolge der guten Wasserlöslichkeit von Ibuprofenlysinat kann eine schnellere Absorption erfolgen, einhergehend mit einem schnelleren Wirkeintritt. Vor diesem Hintergrund kann es erfindungsgemäß gleichermaßen auch vorgesehen sein, dass der NSAR-Wirkstoff Ibuprofenlysinat ist und dass das Lokalanästhetikum Lidocainhydrochlorid ist. Insbesondere kann somit die erste Phase, insbesondere die erste Schicht, Ibuprofenlysinat als NSAR-Wirkstoff aufweisen und kann die zweite Phase, insbesondere die zweite Schicht, Lidocainhydrochlorid als Lokalanästhetikum aufweisen.

Was die erfindungsgemäße Darreichungsform weiterhin anbelangt, so kommt auch den jeweiligen Wirkstoffmengen eine große Bedeutung zu, und zwar auch in deren Abstimmung zueinander. Dabei kann die Darreichungsform nach der Erfindung insbesondere die nachfolgend angeführten Wirkstoffmengen aufweisen, wobei einzelfallbedingt von den nachfolgend angegebenen Werten abgewichen werden kann, ohne dass der Rahmen der vorliegenden Erfindung verlassen ist.

So kann die Darreichungsform, insbesondere die erste Phase, bevorzugt die erste Schicht, den NSAR-Wirkstoff in einer (absoluten) Menge im Bereich von 50 mg bis 800 mg, insbesondere im Bereich von 100 mg bis 400 mg, vorzugsweise im Bereich von 150 mg bis 300 mg, bevorzugt im Bereich von 190 mg bis 210 mg, berechnet als Ibuprofen, aufweisen.

Zudem kann die Darreichungsform, insbesondere die zweite Phase, bevorzugt die zweite Schicht, das Lokalanästhetikum in einer (absoluten) Menge im Bereich von 2 mg bis 50 mg, insbesondere im Bereich von 4 mg bis 20 mg, vorzugsweise im Bereich von 5 mg bis 10 mg, bevorzugt im Bereich von 6 mg bis 9 mg, berechnet als Lidocain, aufweisen.

In diesem Zusammenhang verhält es sich erfindungsgemäß insbesondere derart, dass der NSAR-Wirkstoff in der ersten Phase, bevorzugt in der ersten Schicht, vorliegt und dass das Lokalanästhetikum in der zweiten Phase, insbesondere in der zweiten Schicht, vorliegt.

Gemäß einer erfindungsgemäß bevorzugten Ausführungsform kann es insbesondere vorgesehen sein, dass die Darreichungsform, insbesondere die erste Phase, bevorzugt die erste Schicht, den NSAR-Wirkstoff in einer (absoluten) Menge im Bereich von 190 mg bis 210 mg, berechnet als Ibuprofen, aufweist; und dass die Darreichungsform, insbesondere die zweite Phase, bevorzugt die zweite Schicht, das Lokalanästhetikum in einer (absoluten) Menge im Bereich von 6 mg bis 9 mg, berechnet als Lidocain, aufweist. Denn bei den vorgenannten speziellen Mengen an NSAR-Wirkstoff einerseits und Lokalanästhetikum andererseits liegt eine besonders gute Abstimmung in Bezug auf die Wirkstoffe vor, einhergehend mit einer weiterführenden Wirkoptimierung.

Darüber hinaus kann es erfindungsgemäß vorgesehen sein, dass die Darreichungsform, insbesondere die erste Phase, bevorzugt die erste Schicht, den NSAR-Wirkstoff in einer (relativen) Menge im Bereich von 2,5 Gew.-% bis 50 Gew.-%, insbesondere im Bereich von 5 Gew.-% bis 40 Gew.-%, vorzugsweise im Bereich von 7,5 Gew.-% bis 30 Gew.-%, bevorzugt im Bereich von 10 Gew.-% bis 20 Gew.-%, berechnet als Ibuprofen und bezogen auf die Darreichungsform (Trockengewicht), aufweist.

Erfindungsgemäß kann es zudem vorgesehen sein, dass die Darreichungsform, insbesondere die zweite Phase, bevorzugt die zweite Schicht, das Lokalanästhetikum in einer (relativen) Menge im Bereich von 0,05 Gew.-% bis 5 Gew.-%, insbesondere im Bereich von 0,1 Gew.-% bis 3 Gew.-%, vorzugsweise im Bereich von 0,2 Gew.-% bis 2 Gew.-%, bevorzugt im Bereich von 0,25 Gew.-% bis 0,75 Gew.-%, berechnet als Lidocain und bezogen auf die Darreichungsform (Trockengewicht), aufweist.

In diesem Zusammenhang verhält es sich insbesondere derart, dass der NSAR-Wirkstoff (nur) in der ersten Phase, insbesondere (nur) in der ersten Schicht, vorliegt und dass das Lokalanästhetikum (nur) in der zweiten Phase, insbesondere (nur) in der zweiten Schicht, der erfindungsgemäßen Darreichungsform vorliegt.

Im Speziellen hat es sich dabei als besonders vorteilhaft erwiesen, wenn die Darreichungsform, insbesondere die erste Phase, bevorzugt die erste Schicht, den NSAR-Wirkstoff in einer (relativen) Menge im Bereich von 10 Gew.-% bis 20 Gew.-%, berechnet als Ibuprofen und bezogen auf die Darreichungsform (Trockengewicht), aufweist; und wenn die Darreichungsform, insbesondere die zweite Phase, bevorzugt die zweite Schicht, das Lokalanästhetikum in einer (relativen) Menge im Bereich von 0,25 Gew.-% bis 0,75 Gew.-%, berechnet als Lidocain und bezogen auf die Darreichungsform (Trockengewicht), aufweist.

Weiterführend kann es erfindungsgemäß auch vorgesehen sein, dass die erste Phase, insbesondere die erste Schicht, den NSAR-Wirkstoff in einer (relativen) Menge im Bereich von 5 Gew.-% bis 90 Gew.-%, insbesondere im Bereich von 10 Gew.-% bis 50 Gew.-%, vorzugsweise im Bereich von 15 Gew.-% bis 40 Gew.-%, bevorzugt im Bereich von 20 Gew.-% bis 30 Gew.-%, berechnet als Ibuprofen und bezogen auf die erste Phase (Trockengewicht), insbesondere auf die erste Schicht (Trockengewicht), aufweist.

Diesbezüglich kann es auch vorgesehen sein, dass die zweite Phase, insbesondere die zweite Schicht, das Lokalanästhetikum in einer (relativen) Menge im Bereich von 0,1 Gew.-% bis 20 Gew.-%, insbesondere im Bereich von 0,2 Gew.-% bis 10 Gew.-%, vorzugsweise im Bereich von 0,3 Gew.-% bis 5 Gew.-%, bevorzugt im Bereich von 0,5 Gew.-% bis 1,5 Gew.-%, berechnet als Lidocain und bezogen auf die zweite Phase (Trockengewicht), insbesondere auf die zweite Schicht (Trockengewicht), aufweist.

Weiterhin ist es von besonderem Vorteil, wenn die erste Phase, insbesondere die erste Schicht, den NSAR-Wirkstoff in einer (relativen) Menge im Bereich von 20 Gew.-% bis 30 Gew.-%, berechnet als Ibuprofen und bezogen auf die erste Phase (Trockengewicht), insbesondere auf die erste Schicht (Trockengewicht), aufweist; und wenn die zweite Phase, insbesondere die zweite Schicht, das Lokalanästhetikum in einer (relativen) Menge im Bereich von 0,5 Gew.-% bis 1,5 Gew.-%, berechnet als Lidocain und bezogen auf die zweite Phase (Trockengewicht), insbesondere auf die zweite Schicht (Trockengewicht), aufweist.

Erfindungsgemäß hat es sich zudem als besonders vorteilhaft erwiesen, wenn der NSAR-Wirkstoff zumindest im Wesentlichen nur in der ersten Phase, insbesondere ersten Schicht, vorliegt. Erfindungsgemäß verhält es sich somit insbesondere derart, dass die zweite Phase, insbesondere die zweite Schicht, zumindest im Wesentlichen keinen NSAR-Wirkstoff aufweist bzw. zumindest im Wesentlichen frei von dem NSAR-Wirkstoff ist.

Weiterhin ist es erfindungsgemäß gleichermaßen von besonderem Vorteil, wenn das Lokalanästhetikum zumindest im Wesentlichen nur in der zweiten Phase, insbesondere zweiten Schicht, vorliegt. Erfindungsgemäß ist es somit bevorzugt, dass die erste Phase, insbesondere die erste Schicht, zumindest im Wesentlichen kein Lokalanästhetikum aufweist bzw. zumindest im Wesentlichen frei von dem Lokalanästhetikum ist.

Erfindungsgemäß wird somit insbesondere auf eine Trennung der jeweiligen Wirkstoffe abgestellt, wonach nämlich der NSAR-Wirkstoff zumindest im Wesentlichen nur in der orodispersiblen Phase und das Lokalanästhetikum zumindest im Wesentlichen nur in der oral-transmukosalen Phase vorliegt. Dies ermöglicht ein optimales Freisetzungsprofil und Wirkeigenschaften. Zudem wird etwaigen Inkompatibilitäten entgegengewirkt, was auch zu einer verbesserten Lagerstabilität der erfindungsgemäßen Darreichungsform führt.

Erfindungsgemäß kann die Darreichungsform, insbesondere die Tablette, ein NSAR-Wirkstoff/Lokalanästhetikum-Mengenverhältnis von ≥ 1 : 1, insbesondere ≥ 5 : 1, vorzugsweise ≥ 15 : 1, bevorzugt ≥ 20 : 1, berechnet als Ibuprofen/Lidocain-Mengenverhältnis, aufweisen. Insbesondere kann die Darreichungsform, insbesondere die Tablette, ein NSAR-Wirkstoff/Lokalanästhetikum-Mengenverhältnis, im Bereich von 1 : 1 bis 400 : 1, insbesondere im Bereich von 5 : 1 bis 100 : 1, vorzugsweise im Bereich von 15 : 1 bis 60 : 1, bevorzugt im Bereich von 20 : 1 bis 35 : 1, berechnet als Ibuprofen/Lidocain-Mengenverhältnis, aufweisen.

Erfindungsgemäß kann es weiterhin vorgesehen sein, dass die Darreichungsform nach der Erfindung neben dem NSAR-Wirkstoff und dem Lokalanästhetikum keinen weiteren NSAR-Wirkstoff und kein weiteres Lokalanästhetikum, insbesondere keinen weiteren und/oder hiervon verschiedenen Wirkstoff, aufweist.

Erfindungsgemäß ist es darüber hinaus besonders bevorzugt, wenn die erste Phase, bevorzugt die erste Schicht, den NSAR-Wirkstoff, bevorzugt Ibuprofen, in Form eines Granulats, insbesondere auf Basis vorzugsweise diskreter Teilchen und/oder Partikel, aufweist. Insbesondere kann es erfindungsgemäß vorgesehen sein, dass der NSAR-Wirkstoff, bevorzugt Ibuprofen, granulatförmig ist bzw. in Form eines Granulats, insbesondere auf Basis vorzugsweise diskreter Teilchen und/oder Partikel, eingesetzt ist bzw. vorliegt. Durch die Verwendung des NSAR-Wirkstoffs in Form eines Granulats können die Zerfallszeiten sowie die Wirkstofffreisetzungsgeschwindigkeiten weiterführend vorgegeben bzw. maßgeschneidert werden. Weiterhin kann hierdurch die systemische Wirkstoffaufnahme verbessert werden (wobei der NSAR-Wirkstoff in bereits im Mund aufgelöster Form oder in Form des beim Zerfall der ersten Phase freigesetzten Granulats bzw. der diesbezüglichen Teilchen und/oder Partikel systemisch aufgenommen bzw. geschluckt werden kann). Zudem ergeben sich durch die Verwendung eines Granulats verarbeitungstechnische Vorteile bei der Herstellung der erfindungsgemäßen Darreichungsform. Der Einsatz eines Granulats geht zudem mit einer Stabilisierung des NSAR-Wirkstoffs einher, so dass auch von daher eine erhöhte Lagerstabilität vorliegt.

Erfindungsgemäß kann es insbesondere vorgesehen sein, dass das Granulat eine mittlere Teilchen- und/oder Partikelgröße D₅₀ im Bereich von 5 µm bis 500 µm, insbesondere im Bereich von 10 µm bis 200 µm, vorzugsweise im Bereich von 50 µm bis 100 µm, bevorzugt im Bereich von 60 µm bis 85 µm, aufweist. Mit den vorgenannten mittleren Teilchen- bzw. Partikelgrößen D₅₀ können beispielsweise besonders definierte Zerfallszeiten bzw. Wirkstofffreisetzungseigenschaften eingestellt werden, insbesondere auch was das Eindringen von Flüssigkeit bzw. Speichel in die das Granulat aufweisende erste Phase bzw. Schicht anbelangt /was den raschen Zerfall beschleunigt).

Darüber hinaus kann es erfindungsgemäß vorgesehen sein, dass das Granulat, insbesondere die einzelnen Teilchen bzw. Partikel des Granulats, eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist. Erfindungsgemäß kann das Granulat somit in beschichteter Form eingesetzt sein bzw. vorliegen. Erfindungsgemäß ist es dabei bevorzugt, dass die Beschichtung eine wasserlösliche Beschichtung ist. Insbesondere kann es sich erfindungsgemäß um eine vorzugsweise cellulosebasierte Beschichtung, bevorzugt auf Basis von Hydroxypropylmethylcellulose (HPMC), handeln. Insbesondere kann das Granulat folglich mit einer wasserlöslichen Beschichtung, vorzugsweise cellulosebasierten Beschichtung, bevorzugt auf Basis von Hydroxypropylmethylcellulose (HPMC), ausgerüstet sein. Insbesondere ist es dabei vorgesehen, dass das Granulat, insbesondere die einzelnen Teilchen bzw. Partikel des Granulats, zumindest im Wesentlichen vollständig beschichtet und/oder zumindest im Wesentlichen vollständig von der Beschichtung umhüllt sind.

Durch die ziel- und zweckgerichtete Verwendung einer Beschichtung des NSAR-Wirkstoff- bzw. Ibuprofengranulats, können zum einen die Zerfallszeiten bzw. die Wirkstofffreisetzungsgeschwindigkeiten weiterführend vorgegeben bzw. eingestellt werden. Zum anderen können mit der Beschichtung auch die organoleptischen sowie optischen Eigenschaften der Darreichungsform nach der Erfindung vorgegeben bzw. eingestellt werden. Insbesondere kann der oftmals als unangenehm empfundene Geschmack von Ibuprofen bzw. des NSAR-Wirkstoffs sozusagen maskiert werden. Zudem kann die Beschichtung einen Farbstoff aufweisen, so dass auch eine farbliche Vorgabe der Darreichungsform insbesondere im Hinblick auf den NSAR-Wirkstoff in der ersten Phase bzw. ersten Schicht bereitgestellt werden kann. Die Beschichtung stellt zudem sozusagen eine Schutzschicht für den NSAR-Wirkstoff dar, so dass dieser bzw. die Darreichungsform als solche weiterführen stabilisiert werden kann.

Was die Verwendung von Hydroxypropylmethylcellulose (HPMC) als Beschichtungsmaterial anbelangt, so ist dies zudem mit dem Vorteil verbunden, dass bei der Herstellung bzw. bei der Beschichtung des Granulats keine organischen Komponenten bzw. Lösemittel erforderlich sind, da Hydroxypropylmethylcellulose eine gute Wasserlöslichkeit aufweist. Zudem weist Hydroxypropylmethylcellulose gegenüber dem Substrat in Form des NSAR-Wirkstoffs eine gute Anhaftung und somit auch von daher eine hohe Kompatibilität auf.

Erfindungsgemäß weist die Darreichungsform insbesondere folgende Zerfalls- bzw. Wirkstofffreisetzungseigenschaften auf:
Insbesondere kann die erste Phase, insbesondere die erste Schicht, eine Zerfallszeit, insbesondere bestimmt gemäß Ph. Eur., 10. Ausgabe, Methode 2.9.1, im Bereich von 1 s bis 120 s, insbesondere im Bereich von 5 s bis 60 s, vorzugsweise im Bereich von 10 s bis 30 s, aufweisen. Zudem kann die zweite Phase, insbesondere die zweite Schicht, eine Zerfallszeit, insbesondere bestimmt gemäß Ph. Eur., 10. Ausgabe, Methode 2.9.1, im Bereich von 3 min bis 30 min, insbesondere im Bereich von 5 min bis 20 min, vorzugsweise im Bereich von 8 min bis 15 min, aufweisen. Somit kann die zweite Phase, insbesondere die zweite Schicht, eine Zerfallszeit, insbesondere bestimmt gemäß Ph. Eur., 10. Ausgabe, Methode 2.9.1, im Bereich von 180 s bis 1.800 s, insbesondere im Bereich von 300 s bis 1.200 s, vorzugsweise im Bereich von 480 s bis 900 s, aufweisen.

Erfindungsgemäß verhält es sich insbesondere derart, dass die erste Phase, insbesondere die erste Schicht, eine Zerfallszeit, insbesondere bestimmt gemäß Ph. Eur., 10. Ausgabe, Methode 2.9.1, im Bereich von 10 s bis 30 s aufweist und dass die zweite Phase, insbesondere die zweite Schicht, eine Zerfallszeit, insbesondere bestimmt gemäß Ph. Eur., 10. Ausgabe, Methode 2.9.1, im Bereich von 8 min bis 15 min (d. h. 480 s bis 900 s) aufweist. Auf Basis der vorgenannten Werte liegt eine besonders gute Freisetzungsabstimmung bzw. -koordination im Hinblick auf die jeweiligen Wirkstoffe vor.

Erfindungsgemäß kann das Verhältnis der Zerfallszeit der zweiten Phase, insbesondere der zweiten Schicht, zur Zerfallszeit der ersten Phase, insbesondere der ersten Schicht, im Bereich von 1,5 : 1 bis 1.500 : 1, insbesondere im Bereich von 5 : 1 bis 500 : 1, vorzugsweise im Bereich von 10 : 1 bis 100 : 1, liegen. Insbesondere sind dabei die Zerfallszeiten gemäß Ph. Eur., 10. Ausgabe, Methode 2.9.1 bestimmt.

Auf Basis der vorgenannten Verhältnisse der Zerfallszeiten wird insbesondere eine schnelle Freisetzung des NSAR-Wirkstoffs mit einer raschen systemischen Aufnahme sowie eine im Vergleich hierzu verlangsamte bzw. retardierte Freisetzung des Lokalanästhetikums gewährleistet, wobei das Lokalanästhetikum somit über einen längeren Zeitraum in definierter Weise am Wirkort, beispielsweise der Mund- bzw. Rachenschleimhaut, vorliegt, so dass auf lokaler Ebene ein langanhaltender Wirkeffekt auf Basis des Lokalanästhetikums erreicht wird, welcher durch den insbesondere systemisch (sowie zudem gegebenenfalls auch lokal) wirkenden NSAR-Wirkstoff entsprechend ergänzt bzw. verstärkt wird.

Im Allgemeinen verhält es sich im Rahmen der vorliegenden Erfindung insbesondere derart, dass die Wirkstofffreisetzungsgeschwindigkeit der ersten Phase (NSAR-Wirkstofffreisetzungsgeschwindigkeit) größer ist als die Wirkstofffreisetzungsgeschwindigkeit der zweiten Phase (Lokalanästhetikum-Wirkstofffreisetzungsgeschwindigkeit).

In diesem Zusammenhang kann es erfindungsgemäß insbesondere vorgesehen sein, dass die erste Phase, insbesondere die erste Schicht, eine Wirkstofffreisetzungsgeschwindigkeit, insbesondere bestimmt gemäß Ph. Eur., 10. Ausgabe, Methode 2.9.3, im Bereich von 2 mg/s bis 200 mg/s, insbesondere im Bereich von 3 mg/s bis 100 mg/s, vorzugsweise im Bereich von 5 mg/s bis 50 mg/s, aufweist.

Zudem kann die zweite Phase, insbesondere die zweite Schicht, eine Zerfallszeit, insbesondere bestimmt gemäß Ph. Eur., 10. Ausgabe, Methode 2.9.1, im Bereich von 4·10⁻³ mg/s bis 40·10⁻³ mg/s, insbesondere im Bereich von 5·10⁻³ mg/s bis 20·10⁻³ mg/s, vorzugsweise im Bereich von 6·10⁻³ mg/s bis 15·10⁻³ mg/s, aufweisen.

Gemäß einer erfindungsgemäß bevorzugten Ausführungsform verhält es sich zudem derart, dass die erste Phase, insbesondere die erste Schicht, eine Wirkstofffreisetzungsgeschwindigkeit, insbesondere bestimmt gemäß Ph. Eur., 10. Ausgabe, Methode 2.9.3, im Bereich von 5 mg/s bis 50 mg/s, aufweist; und dass die zweite Phase, insbesondere die zweite Schicht, eine Zerfallszeit, insbesondere bestimmt gemäß Ph. Eur., 10. Ausgabe, Methode 2.9.1, im Bereich von 6·10⁻³ mg/s bis 15·10⁻³ mg/s, aufweist.

In diesem Zusammenhang kommt auch dem Verhältnis der Wirkstofffreisetzungsgeschwindigkeiten eine große Bedeutung zu: So kann das Verhältnis der Wirkstofffreisetzungsgeschwindigkeit der ersten Phase, insbesondere ersten Schicht, zur Wirkstofffreisetzungsgeschwindigkeit der zweiten Phase, insbesondere zur Wirkstofffreisetzungsgeschwindigkeit der zweiten Schicht, im Bereich von 50 : 1 bis 10.000 : 1, insbesondere im Bereich von 100 : 1 bis 5.000 : 1, vorzugsweise im Bereich von 500 : 1 bis 2.000 : 1, liegen. Die Wirkstofffreisetzungsgeschwindigkeit kann insbesondere gemäß Ph. Eur., 10. Ausgabe, Methode 2.9.3, bestimmt sein.

Erfindungsgemäß ist es dabei insbesondere vorgesehen, dass die erste Phase, insbesondere die erste Schicht, vorzugsweise nur und/oder ausschließlich die erste Phase, insbesondere die erste Schicht, mindestens ein Spreng- bzw. Zerfallsmittel aufweist. Das Spreng- bzw. Zerfallsmittel führt dabei zu einem schnelleren Zerfall bzw. zu einer höheren Wirkstofffreisetzungsgeschwindigkeit in Bezug auf die das Spreng- bzw. Zerfallsmittel aufweisende Phase bzw. Schicht, insbesondere erste Phase bzw. Schicht.

Das Spreng- bzw. Zerfallsmittel führt somit insbesondere bei Kontakt mit einer Flüssigkeit bzw. mit Speichel bei oraler Verabreichung zu einem definierten Zerfall der damit ausgerüsteten (ersten) Phase, wobei der Zerfall rasch einsetzt und zudem mit hoher Geschwindigkeit verläuft, so dass der (NSAR-)Wirkstoff schnell aus der ersten Phase bzw. ersten Schicht freigesetzt wird.

Bei dem Spreng- bzw. Zerfallsmittel handelt es sich insbesondere um einen pharmazeutischen Hilfsstoff, welcher den kontrollierten Zerfall bei oraler Applikation bzw. Kontakt der Darreichungsform mit Flüssigkeit bzw. Speichel ermöglicht. Die Zerfalls- bzw. Wirkstofffreisetzungseigenschaften können insbesondere durch die gezielte Verwendung eines Spreng- bzw. Zerfallsmittels eingestellt werden. Die Verwendung eines Spreng- bzw. Zerfallsmittels insbesondere für die erste Phase bzw. Schicht führt insgesamt zu einem definierten Zerfall bzw. Desintegration bei Kontakt mit einer Flüssigkeit, insbesondere Speichel (d. h. bei oraler Applikation).

Erfindungsgemäß kann es in diesem Zusammenhang vorgesehen sein, dass das Spreng- und/oder Zerfallsmittel der ersten Phase, insbesondere der ersten Schicht, ausgewählt ist aus der Gruppe von Polyvinylpolypyrrolidon (Crospovidon); Cellulosen, insbesondere mikrokristallinen Cellulosen, Methylcelllosen, Croscarmellose-Salz, vorzugsweise Croscarmellose-Alkali und -Erdalkali, bevorzugt Croscarmellose-Natrium; Stärken, insbesondere Maisstärke, Kartoffelstärke, vorverkleisterten Stärken; Alginsäure und deren Salzen, insbesondere Alkali- und Erdalkalisalzen, vorzugsweise Calciumalginat und Natriumalginat; Carboxyalkylstärken und deren Salze, insbesondere Alkali- und Erdalkalicarboxyalkylstärken, vorzugsweise Natriumcarboxmethylstärke; Alkali- und Erdalkalihydrogencarbonat, vorzugsweise Natriumhydrogencarbonat, insbesondere in Kombination mit mindestens einer vorzugsweise organischen Säure, vorzugsweise Natriumhydrogencarbonat in Kombination mit Citronensäure und/oder Weinsäure; oberflächenaktiven Substanzen, insbesondere zur Hydrophilisierung; und deren Mischungen oder Kombinationen, besonders bevorzugt Polyvinylpolypyrrolidon (Crospovidon) und/oder Croscarmellose-Natrium.

Im Rahmen der vorliegenden Erfindung werden besonders gute Zerfallseigenschaften bzw. definierte Wirkstofffreisetzungsgeschwindigkeiten erreicht, wenn das Spreng- und/oder Zerfallsmittel der ersten Phase, insbesondere der ersten Schicht, Croscarmellose-Alkali oder -Erdalkali, insbesondere Croscarmellose-Natrium, und/oder Polyvinylpolypyrrolidon (Crospovidon), insbesondere Polyvinylpolypyrrolidon (Crospovidon), ist.

Bei Polyvinylpolypyrrolidon bzw. Crospovidon handelt es sich im Allgemeinen um ein stark verzweigtes Polymer auf Basis von Vinylpyrrolidon. Polyvinylpolypyrrolidon ist zumindest im Wesentlichen nicht wasserlöslich. Bei Kontakt mit Wasser bzw. Speichel kommt es insbesondere zu einer Quellung, so dass auf dieser Basis ein kontrollierter Zerfall der ersten Phase bzw. der ersten Schicht ermöglicht wird. Dabei verhält es sich insbesondere derart, dass die erste Phase bzw. die erste Schicht der erfindungsgemäßen Darreichungsform sozusagen auch von innen "gesprengt" wird, sobald sie mit Flüssigkeit bzw. Speichel in Kontakt kommt. Dies führt zu einem definierten und schnellen Zerfall mit hoher Geschwindigkeit, einhergehend mit einer schnellen Wirkstofffreisetzung und -aufnahme. Die Verwendung von Polyvinylpolypyrrolidon ist zudem mit dem Vorteil verbunden, dass die Darreichungsform eine hohe Abriebfestigkeit aufweist und zudem bei Wasser- bzw. Speichelkontakt rasch und zumindest im Wesentlichen vollständig ohne Schleimbildung zerfällt. Polyvinylpolypyrrolidon bzw. Crospovidon weist dabei besonders definierte Eigenschaften im Hinblick auf seine Funktion als Spreng- bzw. Zerfallsmittel auf.

Erfindungsgemäß kann es insbesondere vorgesehen sein, dass die erste Phase, insbesondere die erste Schicht, das Spreng- und/oder Zerfallsmittel, insbesondere Polyvinylpolypyrrolidon (Crospovidon), in einer (absoluten) Menge im Bereich von 20 mg bis 750 mg, insbesondere im Bereich von 50 mg bis 350 mg, vorzugsweise im Bereich von 100 mg bis 250 mg, bevorzugt im Bereich von 140 mg bis 180 mg, aufweist.

Insbesondere kann die Darreichungsform, insbesondere die erste Phase, bevorzugt die erste Schicht, das Spreng- und/oder Zerfallsmittel, insbesondere Polyvinylpolypyrrolidon (Crospovidon), in einer (relativen) Menge im Bereich von 2 Gew.-% bis 45 Gew.-%, insbesondere im Bereich von 3 Gew.-% bis 35 Gew.-%, vorzugsweise im Bereich von 4 Gew.-% bis 25 Gew.-%, bevorzugt im Bereich von 5 Gew.-% bis 15 Gew.-%, bezogen auf die Darreichungsform (Trockengewicht), aufweisen.

Zudem kann die erste Phase, insbesondere die erste Schicht, das Spreng- und/oder Zerfallsmittel, insbesondere Polyvinylpolypyrrolidon (Crospovidon), in einer (relativen) Menge im Bereich von 3 Gew.-% bis 90 Gew.-%, insbesondere im Bereich von 5 Gew.-% bis 70 Gew.-%, vorzugsweise im Bereich von 10 Gew.-% bis 50 Gew.-%, bevorzugt im Bereich von 15 Gew.-% bis 25 Gew.-%, bezogen auf die erste Phase (Trockengewicht), insbesondere auf die erste Schicht (Trockengewicht), aufweisen.

Erfindungsgemäß verhält es sich dabei insbesondere derart, dass das Spreng- bzw. Zerfallsmittel, insbesondere Polyvinylpolypyrolidon (Crospovidon), zumindest im Wesentlichen nur in der ersten Phase, insbesondere ersten Schicht, vorliegt.

Erfindungsgemäß kann es dabei vorgesehen sein, dass die zweite Phase, insbesondere die zweite Schicht, zumindest im Wesentlichen kein wie zuvor definiertes Spreng- und/oder Zerfallsmittel (d.h. beispielsweise zumindest im Wesentlichen kein Croscarmellose-Natrium und/oder zumindest im Wesentlichen kein Polyvinylpolypyrrolidon (Crospovidon)), insbesondere zumindest im Wesentlichen kein Spreng- und/oder Zerfallsmittel, aufweist bzw. zumindest im Wesentlichen frei von einem wie zuvor definierten Spreng- und/oder Zerfallsmittel, insbesondere zumindest im Wesentlichen frei von einem Spreng- und/oder Zerfallsmittel, ist.

Mit der Verwendung eines Spreng- bzw. Zerfallsmittels nur für die erste Phase bzw. erste Schicht können die unterschiedlichen Zerfalls- bzw. Wirkstofffreisetzungseigenschaften in Bezug auf die erste Phase bzw. Schicht und die zweite Phase bzw. Schicht weiterführend eingestellt bzw. maßgeschneidert werden.

Die Vermeidung der Verwendung eines Spreng- bzw. Zerfallsmittels für die als oral-transmukosale Phase bzw. Schicht ausgebildete zweite Phase bzw. Schicht begünstigt den für diese Phase vorgesehenen langsamen bzw. retardierten Zerfall bzw. Wirkstofffreisetzung des Lokalanästhetikums. Dies ermöglicht wiederum die langanhaltende Exposition der Schleimhaut im Mund/Rachen-Raum mit dem Lokalanästhetikum, einhergehend mit einer entsprechend optimierten lokal hervorgerufenen schmerzstillenden Wirkung durch das Lokalanästhetikum.

Die erfindungsgemäß bevorzugte Ausgestaltung der Darreichungsform, wonach die erste Phase bzw. Schicht ein Spreng- bzw. Zerfallsmittel aufweist und die zweite Phase zumindest im Wesentlichen frei von einem Spreng- bzw. Zerfallsmittel ist, führt insgesamt zu einer nochmals verbesserten Abstimmung der Freisetzung der in den jeweiligen Phasen vorliegenden Wirkstoffe.

Demgegenüber kann es aber gemäß einer weiteren erfindungsgemäßen Ausführungsform mit einer alternativen Ausgestaltung der Darreichungsform nach der Erfindung jedoch vorgesehen sein, dass (auch) die zweite Phase, insbesondere die zweite Schicht, mindestens ein Spreng- und/oder Zerfallsmittel aufweist. In diesem Zusammenhang kann es vorgesehen sein, dass das Spreng- bzw. Zerfallsmittel der zweiten Phase, insbesondere der zweiten Schicht, ausgewählt ist aus der Gruppe von Polyvinylpolypyrrolidon (Crospovidon); Cellulosen, insbesondere mikrokristallinen Cellulosen, Methylcelllosen, Croscarmellose-Salz, vorzugsweise Croscarmellose-Alkali und -Erdalkali, bevorzugt Croscarmellose-Natrium; Stärken, insbesondere Maisstärke, Kartoffelstärke, vorverkleisterten Stärken; Alginsäure und deren Salzen, insbesondere Alkali- und Erdalkalisalzen, vorzugsweise Calciumalginat und Natriumalginat; Carboxyalkylstärken und deren Salze, insbesondere Alkali- und Erdalkalicarboxyalkylstärken, vorzugsweise Natriumcarboxymethylstärke; Alkali- und Erdalkalihydrogencarbonat, vorzugsweise Natriumhydrogencarbonat, insbesondere in Kombination mit mindestens einer vorzugsweise organischen Säure, vorzugsweise Natriumhydrogencarbonat in Kombination mit Citronensäure und/oder Weinsäure; oberflächenaktiven Substanzen, insbesondere zur Hydrophilisierung; und deren Mischungen oder Kombinationen, besonders bevorzugt Polyvinylpolypyrrolidon (Crospovidon) und/oder Croscarmellose-Natrium. Insbesondere kann das Spreng- und/oder Zerfallsmittel der zweiten Phase, insbesondere der zweiten Schicht, Croscarmellose-Alkali bzw. -Erdalkali, insbesondere Croscarmellose-Natrium, und/oder Polyvinylpolypyrrolidon (Crospovidon), insbesondere Polyvinylpolypyrrolidon (Crospovidon) sein.

Die erfindungsgemäße Ausführungsform, wonach die zweite Phase, insbesondere die zweite Schicht, mindestens ein Spreng- bzw. Zerfallsmittel aufweist, gilt jedoch mit der Maßgabe, dass die Menge an Spreng- und/oder Zerfallsmittel in der zweiten Phase, insbesondere zweiten Schicht, geringer ist als die Menge an Spreng- und/oder Zerfallsmittel in der ersten Phase, insbesondere ersten Schicht. Diesbezüglich ist es erfindungsgemäß insbesondere vorgesehen, dass die Menge an Spreng- und/oder Zerfallsmittel in der zweiten Phase, insbesondere zweiten Schicht, höchstens 60 %, insbesondere höchstens 40 %, vorzugsweise höchstens 20 %, bevorzugt höchstens 10 %, der absoluten Menge an Spreng- und/oder Zerfallsmittel in der ersten Phase, insbesondere ersten Schicht, beträgt.

In diesem Zusammenhang kann es erfindungsgemäß insbesondere vorgesehen sein, dass das Verhältnis der Menge an Spreng- und/oder Zerfallsmittel in der zweiten Phase, insbesondere zweiten Schicht, zu der Menge an Spreng- und/oder Zerfallsmittel in der ersten Phase, insbesondere ersten Schicht, höchstens 6 : 10, insbesondere höchstens 4 : 10, vorzugsweise höchstens 2 : 10, bevorzugt höchstens 1 : 10, beträgt. Insbesondere kann das Verhältnis der Menge an Spreng- und/oder Zerfallsmittel in der zweiten Phase, insbesondere zweiten Schicht, zu der Menge an Spreng- und/oder Zerfallsmittel in der ersten Phase, insbesondere ersten Schicht, im Bereich von 0,01 : 10 bis 6 : 10, insbesondere im Bereich von 0,05 : 10 bis 4 : 10, vorzugsweise im Bereich von 0,1 : 10 bis 2 : 10, bevorzugt im Bereich von 0,2 : 10 bis 1 : 10, liegen.

Hierdurch können die Zerfallseigenschaften bzw. Wirkstofffreisetzung aus der zweiten Phase insgesamt weiterführend gesteuert bzw. eingestellt werden, auch in Abstimmung mit der ersten Phase bzw. Schicht, insbesondere mit der Maßgabe, dass die zweite Phase bzw. Schicht einen langsameren bzw. retardierten Zerfall bzw. Wirkstofffreisetzung aufweist, insbesondere wie zuvor definiert.

Darüber hinaus hat es sich im Rahmen der vorliegenden Erfindung als vorteilhaft erwiesen, wenn die erste Phase, insbesondere die erste Schicht, mindestens einen (ersten) Füll- und/oder Trägerstoff, insbesondere zerfallsunterstützenden Füll- und/oder Trägerstoff, vorzugsweise auf Basis von Zuckeralkoholen, bevorzugt Mannitol, aufweist. Durch den ziel- und zweckgerichteten Einsatz eines Füll- bzw. Trägerstoffs insbesondere mit zerfallsunterstützenden Eigenschaften, können die Zerfalls- und Wirkstofffreisetzungen der erfindungsgemäßen Darreichungsform hinsichtlich der ersten Phase bzw. ersten Schicht weiterführend eingestellt bzw. maßgeschneidert werden. Der insbesondere zerfallsunterstützende Füll- und Trägerstoff geht mit einer ergänzenden bzw. unterstützenden Wirkung in Bezug auf das Spreng- bzw. Zerfallsmittel in der ersten Phase bzw. ersten Schicht einher. Insbesondere führt der Füll- bzw. Trägerstoff, welcher insbesondere zerfallsunterstützende Eigenschaften aufweist, zu einer Unterstützung bzw. Modulierung des Zerfalls der ersten Phase bzw. ersten Schicht der erfindungsgemäßen Darreichungsform. Die Verwendung eines Füll- bzw. Trägerstoffs geht auch mit verbesserten organoleptischen Eigenschaften der erfindungsgemäßen Darreichungsform einher. So liegt insgesamt ein verbessertes Mundgefühl, insbesondere einhergehend mit meinem kühlenden Effekt, bei oraler Verabreichung der erfindungsgemäßen Darreichungsform vor ("frischer Geschmack", "Kühleffekt").

Erfindungsgemäß kann die erste Phase, vorzugsweise die erste Schicht, den (ersten) Füll- und/oder Trägerstoff, insbesondere zerfallsunterstützenden Füll- und/oder Trägerstoff, bevorzugt Mannitol, in einer (absoluten) Menge im Bereich von 25 mg bis 1.500 mg, insbesondere im Bereich von 50 mg bis 1.000 mg, vorzugsweise im Bereich von 100 mg bis 800 mg, bevorzugt im Bereich von 150 mg bis 500 mg, aufweisen.

In diesem Zusammenhang kann die Darreichungsform, insbesondere die erste Phase, bevorzugt die erste Schicht, den (ersten) Füll- und/oder Trägerstoff, insbesondere zerfallsunterstützenden Füll- und/oder Trägerstoff, bevorzugt Mannitol, in einer (relativen) Menge im Bereich von 4 Gew.-% bis 75 Gew.-%, insbesondere im Bereich von 8 Gew.-% bis 50 Gew.-%, vorzugsweise im Bereich von 10 Gew.-% bis 40 Gew.-%, bevorzugt im Bereich von 15 Gew.-% bis 30 Gew.-%, bezogen auf die Darreichungsform (Trockengewicht), aufweisen.

Weiterhin kann die Darreichungsform nach der Erfindung, insbesondere die erste Phase, insbesondere die erste Schicht, den (ersten) Füll- und/oder Trägerstoff, insbesondere zerfallsunterstützenden Füll- und/oder Trägerstoff, bevorzugt Mannitol, in einer (relativen) Menge im Bereich von 5 Gew.-% bis 90 Gew.-%, insbesondere im Bereich von 10 Gew.-% bis 70 Gew.-%, vorzugsweise im Bereich von 20 Gew.-% bis 60 Gew.-%, bevorzugt im Bereich von 30 Gew.-% bis 45 Gew.-%, bezogen auf die erste Phase (Trockengewicht), insbesondere auf die erste Schicht (Trockengewicht), aufweisen.

Erfindungsgemäß kann es grundsätzlich vorgesehen sein, dass die zweite Phase, insbesondere die zweite Schicht, zumindest im Wesentlichen kein Mannitol, insbesondere zumindest im Wesentlichen keinen zerfallsunterstützenden Füll- und/oder Trägerstoff, aufweist bzw. zumindest im Wesentlichen frei von Mannitol, insbesondere zumindest im Wesentlichen frei von einem zerfallsunterstützenden Füll- und/oder Trägerstoff, ist. Hierdurch kann der insgesamt langsame bzw. retardierte Zerfall bzw. Wirkstofffreisetzung der zweiten Phase bzw. zweiten Schicht weiter unterstützt bzw. vorgegeben werden.

Demgegenüber kann es aber grundsätzlich aber auch vorgesehen sein, dass die zweite Phase, insbesondere die zweite Schicht, mindestens einen (zweiten) Füll- und/oder Trägerstoff, insbesondere nichtzerfallsunterstützenden Füll- und/oder Trägerstoff, vorzugsweise auf Basis von Zuckeralkoholen, bevorzugt Sorbitol bzw. Isomalt, aufweist. Diesbezüglich kann der (zweite) Füll- bzw. Trägerstoff, insbesondere nichtzerfallsunterstützender Füll- bzw. Trägerstoff, maßgeblich (nur) als Matrixmaterial zur Aufnahme bzw. Integration des Lokalanästhetikums fungieren.

In diesem Zusammenhang kann es erfindungsgemäß insbesondere vorgesehen sein, dass die zweite Phase, vorzugsweise die zweite Schicht, den (zweiten) Füll- und/oder Trägerstoff, insbesondere nichtzerfallsunterstützenden Füll- und/oder Trägerstoff, bevorzugt Sorbitol und/oder Isomalt, in einer (absoluten) Menge im Bereich von 50 mg bis 2.000 mg, insbesondere im Bereich von 75 mg bis 1.500 mg, vorzugsweise im Bereich von 100 mg bis 1.000 mg, bevorzugt im Bereich von 200 mg bis 800 mg, aufweist.

Zudem kann es erfindungsgemäß vorgesehen sein, dass die Darreichungsform, insbesondere die zweite Phase, bevorzugt die zweite Schicht, den (zweiten) Füll- und/oder Trägerstoff, insbesondere nichtzerfallsunterstützenden Füll- und/oder Trägerstoff, bevorzugt Sorbitol und/oder Isomalt, in einer (relativen) Menge im Bereich von 5 Gew.-% bis 80 Gew.-%, insbesondere im Bereich von 10 Gew.-% bis 75 Gew.-%, vorzugsweise im Bereich von 15 Gew.-% bis 70 Gew.-%, bevorzugt im Bereich von 20 Gew.-% bis 60 Gew.-%, bezogen auf die Darreichungsform (Trockengewicht), aufweist.

In diesem Zusammenhang kann es insbesondere auch vorgesehen sein, dass die zweite Phase, insbesondere die zweite Schicht, den (zweiten) Füll- und/oder Trägerstoff, insbesondere nichtzerfallsunterstützenden Füll- und/oder Trägerstoff, bevorzugt Sorbitol und/oder Isomalt, in einer (relativen) Menge im Bereich von 10 Gew.-% bis 98 Gew.-%, insbesondere im Bereich von 15 Gew.-% bis 95 Gew.-%, vorzugsweise im Bereich von 20 Gew.-% bis 90 Gew.-%, bevorzugt im Bereich von 30 Gew.-% bis 85 Gew.-%, bezogen auf die zweite Phase (Trockengewicht), insbesondere auf die zweite Schicht (Trockengewicht), aufweist.

Insgesamt ermöglicht bzw. unterstützt die Verwendung insbesondere von Sorbitol bzw. Isomalt den langsamen bzw. retardierten Zerfall der zweiten Phase bzw. zweiten Schicht.

Darüber hinaus kann es erfindungsgemäß vorgesehen sein, dass die erfindungsgemäße Darreichungsform, insbesondere die erste Phase und/oder die zweite Phase, vorzugsweise die erste Schicht und/oder die zweite Schicht, insbesondere unabhängig voneinander, mindestens ein Säuerungsmittel, insbesondere in Form einer organischen Säure oder deren Salze und Ester, vorzugsweise Citronensäure oder deren Salze und Ester, bevorzugt Citronensäure, aufweist. Hierdurch können auch die organoleptischen Eigenschaften weiterführend verbessert bzw. eingestellt werden. Zudem kann das Säuerungsmittel, insbesondere in Form von Citronensäure, der Induktion bzw. Steigerung des Speichelflusses bzw. der Speichelproduktion bei oraler Applikation dienen. Hierdurch kann der Zerfall bzw. die Wirkstofffreisetzung nochmals verbessert bzw. positiv beeinflusst werden. Zudem führt eine erhöhte Menge an Speichel zu einem häufigeren Schlucken, was insbesondere zu einer verbesserten systemischen Aufnahme des NSAR-Wirkstoffs aus der ersten Phase bzw. ersten Schicht führt. Zudem kann das Säuerungsmittel, insbesondere Citronensäure, zu einer Stabilisierung insbesondere des Lokalanästhetikums in Form von Lidocainhydrochlorid führen. Was die Verwendung von Citronensäure weiterhin anbelangt, so kann auf dieser Basis auch der Geschmack der erfindungsgemäßen Darreichungsform verbessert bzw. eingestellt werden, und zwar auch im Hinblick auf eine Überlagerung des mitunter schlechten Geschmacks der eingesetzten Wirkstoffe, insbesondere des NSAR-Wirkstoffs.

Gemäß einer erfindungsgemäß bevorzugten Ausführungsform weist sowohl die erste Phase bzw. die erste Schicht als auch die zweite Phase bzw. zweite Schicht ein Säuerungsmittel, insbesondere wie zuvor definiert, auf. Erfindungsgemäß kann es insbesondere vorgesehen sein, dass die erste Phase, insbesondere die erste Schicht, und die zweite Phase, insbesondere die zweite Schicht, insbesondere unabhängig voneinander, jeweils mindestens ein Säuerungsmittel, insbesondere in Form einer organischen Säure oder deren Salze und Ester, vorzugsweise Citronensäure oder deren Salze und Ester, bevorzugt Citronensäure, aufweisen.

Erfindungsgemäß kann es im Allgemeinen vorgesehen sein, dass die erste Phase, insbesondere die erste Schicht, und/oder die zweite Phase, insbesondere die zweite Schicht, insbesondere unabhängig voneinander, das Säuerungsmittel, insbesondere Citronensäure, jeweils in einer (absoluten) Menge im Bereich von 0,5 mg bis 100 mg, insbesondere im Bereich von 1 mg bis 50 mg, vorzugsweise im Bereich von 2 mg bis 25 mg, bevorzugt im Bereich von 5 mg bis 20 mg, aufweisen.

Zudem kann es erfindungsgemäß vorgesehen sein, dass die Darreichungsform, das Säuerungsmittel, insbesondere Citronensäure, in einer (relativen) Menge im Bereich von 0,01 Gew.-% bis 10 Gew.-%, insbesondere im Bereich von 0,05 Gew.-% bis 5 Gew.-%, vorzugsweise im Bereich von 0,1 Gew.-% bis 3 Gew.-%, bevorzugt im Bereich von 0,5 Gew.-% bis 2,5 Gew.-%, bezogen auf die Darreichungsform (Trockengewicht), aufweist.

Zudem kann es erfindungsgemäß auch vorgesehen sein, dass die erste Phase, insbesondere die erste Schicht, und/oder die zweite Phase, insbesondere die zweite Schicht, insbesondere unabhängig voneinander, das Säuerungsmittel, insbesondere Citronensäure, jeweils in einer (relativen) Menge im Bereich von 0,01 Gew.-% bis 10 Gew.-%, insbesondere im Bereich von 0,05 Gew.-% bis 5 Gew.-%, vorzugsweise im Bereich von 0,1 Gew.-% bis 3 Gew.-%, bevorzugt im Bereich von 0,5 Gew.-% bis 2,5 Gew.-%, bezogen auf die erste Phase und/oder die zweite Phase (Trockengewicht), insbesondere auf die erste Schicht und/oder die zweite Schicht (Trockengewicht), aufweisen.

Darüber hinaus kann die Darreichungsform, insbesondere die erste Phase und/oder die zweite Phase, vorzugsweise die erste Schicht und/oder die zweite Schicht, insbesondere unabhängig voneinander, mindestens ein geschmacksmodifizerendes und/oder geschmacksmaskierendes Mittel, insbesondere schärfemaskierendes Mittel und/oder bittermaskierendes Mittel, vorzugsweise Cyclodextrin und/oder Erdalkali- bzw. Alkalilaurylsulfat, insbesondere Natriumlaurylsulfat, aufweisen. Als geschmacksmodifizierendes bzw. geschmacksmaskierendes Mittel, insbesondere bittermaskierendes Mittel, kann insbesondere Cyclodextrin eingesetzt sein.

Die Verwendung eines geschmacksmodifizierenden bzw. geschmacksmaskierenden Mittels dient insbesondere zur weiterführenden Verringerung des im Allgemeinen schlechten Geschmacks des NSAR-Wirkstoffs bzw. des Lokalanästhetikums. So weist das als NSAR-Wirkstoff eingesetzte Ibuprofen im Allgemeinen insbesondere einen mitunter unangenehm scharfen Geschmack auf, welcher durch die geschmacksmodifizierende bzw. geschmacksmaskierende Mittel kompensiert bzw. maskiert werden kann. Durch die Verwendung des geschmacksmodifizierenden bzw. geschmacksmaskierenden Mittels kann zudem der mitunter unangenehm bittere Geschmack von Lidocain bzw. Lidocainhydrochlorid kompensiert bzw. maskiert werden, und zwar insbesondere auch auf Basis der Verwendung von Cyclodextrin.

Erfindungsgemäß kann das geschmacksmodifizierende bzw. geschmacksmaskierende Mittel auch in Ergänzung zu der zuvor beschriebenen Beschichtung (Coating) des NSAR-Wirkstoffs bzw. dem zuvor angeführten Säuerungsmittel eingesetzt sein, wobei sich die jeweiligen geschmacksmaskierenden Eigenschaften ergänzen können und weiterführend verstärken, so dass insgesamt nachhaltig verbesserte geschmackliche Eigenschaften bzw. organoleptische Eigenschaften in Bezug auf die erfindungsgemäße Darreichungsform vorgegeben werden können. Im Allgemeinen ist es erfindungsgemäß bevorzugt, dass die erste Phase, insbesondere die erste Schicht, und die zweite Phase, insbesondere die zweite Schicht, das in Rede stehende geschmacksmodifizierende bzw. geschmacksmaskierende Mittel aufweisen.

Erfindungsgemäß kann es insbesondere vorgesehen sein, dass die erste Phase, insbesondere die erste Schicht, und/oder die zweite Phase, insbesondere die zweite Schicht, insbesondere unabhängig voneinander, das geschmacksmodifizerende und/oder geschmacksmaskierende Mittel, jeweils in einer (absoluten) Menge im Bereich von 0,1 mg bis 100 mg, insbesondere im Bereich von 0,2 mg bis 80 mg, vorzugsweise im Bereich von 0,5 mg bis 75 mg, bevorzugt im Bereich von 0,6 mg bis 70 mg, aufweisen.

In diesem Zusammenhang kann die Darreichungsform das geschmacksmodifizerende und/oder geschmacksmaskierende Mittel in einer (relativen) Menge im Bereich von 0,01 Gew.-% bis 15 Gew.-%, insbesondere im Bereich von 0,05 Gew.-% bis 10 Gew.-%, vorzugsweise im Bereich von 0,1 Gew.-% bis 7,5 Gew.-%, bevorzugt im Bereich von 0,5 Gew.-% bis 5 Gew.-%, bezogen auf die Darreichungsform (Trockengewicht), aufweisen.

Insbesondere kann die erste Phase, insbesondere die erste Schicht, und/oder die zweite Phase, insbesondere die zweite Schicht, insbesondere unabhängig voneinander, das geschmacksmodifizerende und/oder geschmacksmaskierende Mittel jeweils in einer (relativen) Menge im Bereich von 0,01 Gew.-% bis 25 Gew.-%, insbesondere im Bereich von 0,05 Gew.-% bis 20 Gew.-%, vorzugsweise im Bereich von 0,1 Gew.-% bis 15 Gew.-%, bevorzugt im Bereich von 0,2 Gew.-% bis 12 Gew.-%, bezogen auf die erste Phase und/oder die zweite Phase (Trockengewicht), insbesondere auf die erste Schicht und/oder die zweite Schicht (Trockengewicht), aufweisen.

Gemäß einer speziellen Ausführungsform der vorliegenden Erfindung kann es insbesondere im Hinblick auf eine Abschwächung des scharfen Geschmacks von Ibuprofen, wie er bei oraler Verabreichung bzw. Freisetzung des Wirkstoffs im Mund auftreten kann, vorgesehen sein, dass die erste Phase, insbesondere die erste Schicht, vorzugsweise nur die erste Phase bzw. vorzugsweise nur die erste Schicht, mindestens ein schärfemaskierendes Mittel auf Basis eines Alkali- und/oder Erdalkalilaurylsulfats, insbesondere Natriumlaurylsulfat, aufweist.

In diesem Zusammenhang kann die erste Phase, insbesondere die erste Schicht, das schärfemaskierende Mittel in einer (absoluten) Menge im Bereich von 0,1 mg bis 25 mg, insbesondere im Bereich von 0,5 mg bis 20 mg, vorzugsweise im Bereich von 0,75 mg bis 10 mg, bevorzugt im Bereich von 1 mg bis 10 mg, aufweisen.

Insbesondere kann die Darreichungsform, insbesondere die erste Phase, bevorzugt die erste Schicht, das schärfemaskierende Mittel in einer (relativen) Menge im Bereich von 0,01 Gew.-% bis 5 Gew.-%, insbesondere im Bereich von 0,02 Gew.-% bis 3 Gew.-%, vorzugsweise im Bereich von 0,05 Gew.-% bis 2 Gew.-%, bevorzugt im Bereich von 0,1 Gew.-% bis 1 Gew.-%, bezogen auf die Darreichungsform (Trockengewicht), aufweisen. Zudem kann die erste Phase, bevorzugt die erste Schicht, das schärfemaskierende Mittel in einer (relativen) Menge im Bereich von 0,02 Gew.-% bis 10 Gew.-%, insbesondere im Bereich von 0,04 Gew.-% bis 5 Gew.-%, vorzugsweise im Bereich von 0,06 Gew.-% bis 4 Gew.-%, bevorzugt im Bereich von 0,08 Gew.-% bis 2 Gew.-%, bezogen auf die erste Phase, bevorzugt die erste Schicht, aufweisen.

Erfindungsgemäß kann es weiterhin vorgesehen sein, dass die Darreichungsform, insbesondere die erste Phase und/oder die zweite Phase, vorzugsweise die erste Schicht und/oder die zweite Schicht, insbesondere unabhängig voneinander, mindestens ein Additiv ausweist. In diesem Zusammenhang kann der weitere Inhaltsstoff oder das Additiv ausgewählt sein aus der Gruppe von Verarbeitungshilfsmitteln, insbesondere Fließmitteln und Schmiermitteln, Färbe-, Puffer-, Riech-, Duft-, Streck-, Binde-, Netz- und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und Antiseptika sowie deren Mischungen.

Erfindungsgemäß kommt auch dem Feuchtigkeits- bzw. Wassergehalt bzw. der Restfeuchte der erfindungsgemäßen Darreichungsform bzw. der zugrundeliegenden ersten bzw. zweiten Phase, insbesondere ersten bzw. zweiten Schicht, eine große Bedeutung zu. Durch die Einstellung einer definierten Restfeuchte kann auch die Stabilität bzw. Lagerstabilität der Darreichungsform verbessert werden, und zwar auch im Hinblick auf die mikrobiologische Qualität. Zudem führt eine definierte Restfeuchte zu verbesserten Herstellungseigenschaften, beispielsweise im Hinblick auf die Adhäsivität bzw. Haftfähigkeit der Komponenten bei Herstellung der jeweiligen Phasen bzw. Schichten bzw. der Darreichungsform nach der Erfindung. Zudem führt eine vorgegebene Restfeuchte, wie zuvor definiert, auch zu einem verbesserten Zerfall bzw. Wirkstofffreisetzung bei Applikation der Darreichungsform nach der Erfindung, insbesondere da bei definierter Restfeuchte die Aufnahme bzw. das Eindringen von Flüssigkeit, insbesondere von Speichel, in die Darreichungsform verbessert ist.

In diesem Zusammenhang hat es sich als vorteilhaft erwiesen, wenn die erste Phase, insbesondere die erste Schicht, eine Restfeuchte von höchstens 10 Gew.-%, insbesondere höchstens 5 Gew.-%, vorzugsweise höchstens 3 Gew.-%, bezogen auf die erste Phase (Trockengewicht), insbesondere auf die erste Schicht (Trockengewicht), aufweist und/oder auf die vorgenannten Werte eingestellt ist. Insbesondere kann die erste Phase, insbesondere erste Schicht, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die erste Phase (Trockengewicht), insbesondere auf die erste Schicht (Trockengewicht), aufweist und/oder auf die vorgenannten Werte eingestellt sein.

Weiterhin kann die zweite Phase, insbesondere die zweite Schicht, eine Restfeuchte von höchstens 10 Gew.-%, insbesondere höchstens 5 Gew.-%, vorzugsweise höchstens 3 Gew.-%, bezogen auf die zweite Phase (Trockengewicht), insbesondere auf die zweite Schicht (Trockengewicht), aufweisen und/oder auf die vorgenannten Werte eingestellt sein. Insbesondere kann die zweite Phase, insbesondere die zweite Schicht, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die zweite Phase (Trockengewicht), insbesondere auf die zweite Schicht (Trockengewicht), aufweisen und/oder auf die vorgenannten Werte eingestellt sein.

Erfindungsgemäß ist es insbesondere vorgesehen, dass die erste Phase, insbesondere die erste Schicht, und die zweite Phase, insbesondere die zweite Schicht, zumindest im Wesentlichen dieselbe Restfeuchte aufweisen und/oder zumindest im Wesentlichen auf dieselbe Restfeuchte eingestellt sind.

In diesem Zusammehang ist es erfindungsgemäß bevorzugt, dass die erste Phase, insbesondere die erste Schicht, eine Restfeuchte im Bereich von 0,1 Gew.-% bis 10 Gew.-%, vorzugsweise im Bereich von 1 Gew.-% bis 3 Gew.-%, bezogen auf die erste Phase (Trockengewicht), insbesondere auf die erste Schicht (Trockengewicht), aufweist und/oder auf die vorgenannten Werte eingestellt ist und dass die zweite Phase, insbesondere die zweite Schicht, eine Restfeuchte im Bereich von 0,1 Gew.-% bis 10 Gew.-%, vorzugsweise im Bereich von 1 Gew.-% bis 3 Gew.-%, bezogen auf die zweite Phase (Trockengewicht), insbesondere auf die zweite Schicht (Trockengewicht), aufweist und/oder auf die vorgenannten Werte eingestellt ist.

Folglich verhält es sich erfindungsgemäß insbesondere derart, dass die Darreichungsform, insbesondere die Tablette, eine Restfeuchte von höchstens 10 Gew.-%, insbesondere höchstens 5 Gew.-%, vorzugsweise höchstens 3 Gew.-%, bezogen auf die Darreichungsform (Trockengewicht), aufweist und/oder auf die vorgenannten Werte eingestellt ist. Insbesondere kann die Darreichungsform, insbesondere die Tablette, eine (Geamt-)Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweisen und/oder auf die vorgenannten Werte eingestellt sein.

Im Allgemeinen kann es erfindungsgemäß vorgesehen sein, dass die Darreichungsform, insbesondere die Tablette, ein (Gesamt-)Gewicht im Bereich von 0,25 g bis 7,5 g, insbesondere im Bereich von 0,5 g bis 5 g, vorzugsweise im Bereich von 0,8 g bis 4 g, bevorzugt im Bereich von 1 g bis 3 g, aufweist. Insbesondere kann die erste Phase, insbesondere die erste Schicht, ein Gewicht im Bereich von 0,15 g bis 4 g, insbesondere im Bereich von 0,3 g bis 2,75 g, vorzugsweise im Bereich von 0,45 g bis 2,25 g, bevorzugt im Bereich von 0,6 g bis 1,75 g, aufweisen. Zudem kann die zweite Phase, insbesondere die zweite Schicht, ein Gewicht im Bereich von 0,1 g bis 3,5 g, insbesondere im Bereich von 0,2 g bis 2,25 g, vorzugsweise im Bereich von 0,35 g bis 1,75 g, bevorzugt im Bereich von 0,4 g bis 1,25 g, aufweisen.

Auch dem Gewichtsverhältnis kommt eine weiterführende Bedeutung zu, und zwar auch was die jeweilige Wirkstofffreisetzung aus der ersten Phase bzw. ersten Schicht einerseits und der zweiten Phase bzw. zweiten Schicht andererseits anbelangt: Insbesondere kann das Verhältnis des Gewichts der ersten Phase, insbesondere der ersten Schicht, zum Gewicht der zweiten Phase, insbesondere der zweiten Schicht, im Bereich von 1 : 5 bis 20 : 1, insbesondere im Bereich von 1 : 1 bis 10 : 1, vorzugsweise im Bereich von 1,1 : 1 bis 5 : 1, bevorzugt im Bereich von 1,15 : 1 bis 2 : 1, liegen.

Darüber hinaus kommt auch der körperlichen Ausbildung der erfindungsgemäßen Darreichungsform eine große Bedeutung zu. Durch die spezielle Formgebung kann insbesondere das Mundgefühl verbessert sowie die Zerfalls- bzw. Wirkstofffreisetzungseigenschaften weiterführend vorgegeben werden, beispielsweise im Hinblick auf die Kontaktfläche gegenüber Flüssigkeit, insbesondere Speichel, bei oraler Applikation.

Darüber hinaus kann die Darreichungsform, insbesondere die Tablette, zumindest im Wesentlichen zylinderförmig ausgebildet sein. Dabei können die erste Phase, insbesondere die erste Schicht, und die zweite Phase, insbesondere die zweite Schicht, jeweilige Abschnitte des zumindest im Wesentlichen zylindrischen Grundkörpers der Darreichungsform, insbesondere der Tablette, bilden.

Insbesondere kann es erfindungsgemäß vorgesehen sein, dass die erste Phase, insbesondere die erste Schicht, und die zweite Phase, insbesondere die zweite Schicht, insbesondere unabhängig voneinander, jeweils zumindest im Wesentlichen zylindrisch ausgebildet sind, wobei die erste Phase, insbesondere die erste Schicht, und die zweite Phase, insbesondere die zweite Schicht, über jeweils eine ihrer Grundflächen miteinander in Kontakt stehen, insbesondere miteinander verbunden sind, und/oder einen Verbund ausbilden, vorzugsweise zumindest im Wesentlichen vollflächig, bezogen auf die jeweilige Grundfläche, miteinander in Kontakt stehen, insbesondere miteinander verbunden sind, und/oder einen Verbund ausbilden, insbesondere so dass die Darreichungsform, insbesondere die Tablette, zumindest im Wesentlichen zylinderförmig ausgebildet ist. Insbesondere sind in diesem Zusammenhang die erste Phase, insbesondere die erste Schicht, und die zweite Phase, insbesondere die zweite Schicht, fest miteinander verbunden bzw. bilden die erste Phase, insbesondere die erste Schicht, und die zweite Phase, insbesondere die zweite Schicht, einen festen Verbund aus. Insbesondere werden die jeweiligen Phasen bzw. Schichten somit zueinander bzw. aufeinander zum Haften gebracht.

Die Ausbildung der Darreichungsform bzw. Tablette nach der Erfindung in Form eines Zylinders mit der insbesondere vorgesehenen einander gegenüberliegenden Anordnung der jeweiligen Phasen bzw. Schichten führt zu besonders guten Anwendungseigenschaften, und zwar auch im Hinblick auf den Zerfall bzw. die Wirkstofffreisetzung bei oraler Anwendung. Insbesondere weisen die jeweiligen Phasen bzw. Schichten entsprechend große, bei oraler Applikation mit Flüssigkeit bzw. Speichel in Kontakt stehende Oberflächen auf, so dass auch von daher ein optimaler Zerfall bzw. Wirkstofffreisetzung gegeben ist.

In diesem Zusammenhang kann es erfindungsgemäß insbesondere vorgesehen sein, dass die Grundflächen der Darreichungsform, insbesondere der Tablette, zumindest im Wesentlichen plan und/oder eben ausgebildet sind oder aber gewölbt, insbesondere nach außen gewölbt, ausgebildet sind.

Zudem kann es in diesem Zusammenhang vorgesehen sein, dass die Grundflächen der Darreichungsform, insbesondere der Tablette, zumindest im Wesentlichen parallel zueinander angeordnet sind.

Zudem kommen insbesondere im Hinblick auf das Zerfalls- bzw. Freisetzungsverhalten sowie die Anwendung ("Mundgefühl") der Darreichungsform bzw. Tablette nach der Erfindung den konkreten räumlichen Abmessungen eine weiterführende Bedeutung zu:
So kann es erfindungsgemäß insbesondere vorgesehen sein, dass die Darreichungsform, insbesondere die Tablette, eine (Gesamt-)Dicke (Tablettendicke) bzw. Zylinderhöhe (im Fall einer zumindest im Wesentlichen zylinderförmigen Ausbildung) im Bereich von 1 mm bis 15 mm, insbesondere im Bereich von 2 mm bis 10 mm, vorzugsweise im Bereich von 3 mm bis 8 mm, aufweist.

Insbesondere kann die erste Phase, insbesondere die erste Schicht, eine Dicke (Schichtdicke) bzw. Zylinderhöhe (im Fall einer zumindest im Wesentlichen zylinderförmigen Ausbildung) im Bereich von 0,55 mm bis 8,25 mm, insbesondere im Bereich von 1,1 mm bis 5,5 mm, vorzugsweise im Bereich von 1,65 mm bis 4,4 mm, aufweisen.

Zudem kann die zweite Phase, insbesondere die zweite Schicht, eine Dicke (Schichtdicke) bzw. Zylinderhöhe (im Fall einer zumindest im Wesentlichen zylinderförmigen Ausbildung) im Bereich von 0,45 mm bis 6,75 mm, insbesondere im Bereich von 0,9 mm bis 4,5 mm, vorzugsweise im Bereich von 1,35 mm bis 3,6 mm, aufweisen.

Zudem kann das Verhältnis der Dicke (Schichtdicke) bzw. Zylinderhöhe (im Fall einer zylinderförmigen Ausbildung) der ersten Phase, insbesondere der ersten Schicht, zur Dicke (Schichtdicke) bzw. Zylinderhöhe (im Fall einer zumindest im Wesentlichen zylinderförmigen Ausbildung) der zweiten Phase, insbesondere der zweiten Schicht, im Bereich von 1 : 5 bis 20 : 1, insbesondere im Bereich von 1 : 1 bis 10 : 1, vorzugsweise im Bereich von 1,1 : 1 bis 5 : 1, bevorzugt im Bereich von 1,15 : 1 bis 2 : 1, liegen.

Weiterhin kann es erfindungsgemäß vorgesehen sein, dass die Darreichungsform, insbesondere die Tablette, eine Breite bzw. einen Durchmesser (im Fall einer zumindest im Wesentlichen zylinderförmigen Ausbildung) im Bereich von 3 mm bis 25 mm, insbesondere im Bereich von 5 mm bis 22 mm, vorzugsweise im Bereich von 10 mm bis 20 mm, aufweist.

Insbesondere weisen die Grundflächen der ersten Phase, insbesondere ersten Schicht, und der zweiten ersten Phase, insbesondere zweiten Schicht, zumindest im Wesentlich identische Breiten bzw. Durchmesser auf. Insbesondere sind die erste Phase, insbesondere erste Schicht, und die zweite Phase, insbesondere zweite Schicht, zur Ausbildung der Darreichungsform über ihre zueinander zugewandten Grundflächen vorzugsweise zumindest im Wesentlichen deckungsgleich und/oder zumindest im Wesentlichen vollflächig aufeinander angeordnet und/oder gestapelt.

Die erfindungsgemäße Darreichungsform, insbesondere Tablette, kann insbesondere wie folgt bereitgestellt bzw. hergestellt sein:
Insbesondere kann die Darreichungsform, insbesondere die Tablette, durch Verpressen, vorzugsweise Verpressen der die Darreichungsform, insbesondere die Tablette, ausbildenden Wirk- und Inhaltsstoffe, erhalten sein. Zudem kann die Darreichungsform, insbesondere die Tablette, als Pressling ausgebildet sein und/oder vorliegen.

Erfindungsgemäß wird insbesondere auf ein jeweiliges Verpressen (synonym auch als Komprimieren bezeichnet), vorzugsweise jeweiliges Verpressen der Wirk- bzw. Inhaltsstoffe, der ersten Phase, insbesondere ersten Schicht, einerseits und der zweiten Phase, insbesondere zweiten Schicht, andererseits, abgestellt (Vorverpressen bzw. Vorkomprimieren), wobei die jeweils verpressten (komprimierten) Phasen insbesondere an- bzw. abschließend zum Erhalt der Darreichungsform bzw. der Tablette miteinander (end-)verpresst (hauptverpresst bzw. hauptkomprimiert) sind. Hierdurch kann ein differenziertes und auf die jeweilige Phase bzw. Schicht abgestelltes Verpressen der Wirk- bzw. Inhaltsstoffe vorgenommen werden, wobei auch auf dieser Basis der Zerfall bzw. die Wirkstofffreisetzung gezielt eingestellt bzw. maßgeschneidert werden kann (beispielsweise über den Grad des Verpressens bzw. Komprimierens bzw. Verdichtens), und zwar insbesondere auch im Zusammenwirken mit den weiteren Maßnahmen, wie vorliegend angeführt (beispielsweise was die gezielte Verwendung eines Spreng- bzw. Zerfallsmittel insbesondere für die erste Phase anbelangt). Die spezielle Herstellung durch spezielles Verpressen schlägt sich dabei unmittelbar auf das Produkt in Form der erfindungsgemäßen Darreichungsform bzw. Tablette nieder, so dass die Darreichungsform bzw. Tablette nach der Erfindung auch hierdurch weiterführend charakterisiert ist.

Was das Verpressen anbelangt, so stellt die nachfolgend angegebene Presskraft ([N]) (synonym auch als Kompressionskraft bezeichnet) insbesondere die (Kraft-) Einwirkung auf die erste bzw. zweite Phase bzw. Schicht bzw. auf die Darreichungsform, insbesondere auf die zugrundeliegenden Wirk- und Inhaltsstoffe, dar. Dabei erfolgt die Krafteinwirkung insbesondere über eine Grundfläche (Pressgrundfläche) der ersten bzw. zweiten Phase, insbesondere der ersten bzw. zweiten Schicht, bzw. der Darreichungsform (beispielweise mittels eines in einer Pressvorrichtung, insbesondere Presskammer, insbesondere Matrize), angeordneten (Press-)Stempels oder dergleichen).

Weiterhin bezieht sich der nachfolgend angegebene Pressdruck ([N/mm²]) (synonym auch als Kompressionsdruck bezeichnet) insbesondere auf die Krafteinwirkung in Bezug auf die jeweilige Grundfläche (Pressgrundfläche, d.h. diejenige Grundfläche, über welcher die Krafteinwirkung erfolgt) der ersten bzw. zweiten Phase, insbesondere der ersten bzw. zweiten Schicht, bzw. der Darreichungsform.

Bei den diesbezüglichen Grundflächen bzw. Pressgrundflächen handelt es sich insbesondere um eine jeweilige Zylindergrundfläche (Flachseite) im Fall der bevorzugten körperlichen Ausbildung der Darreichungsform bzw. der jeweiligen Phase bzw. Schicht als zumindest im Wesentlichen Zylinder.

Das Verpressen der Darreichungsform bzw. der jeweiligen Phasen kann dabei in einer entsprechenden Pressvorrichtung, insbesondere Tablettenpresse, vorgenommen sein bzw. werden, insbesondere in einer gemeinsamen Pressvorrichtung, insbesondere Presskammer, bzw. einem diesbezüglichen Ein-Kammer-System.

Gemäß einer bevorzugten Ausführungsform kann es sich erfindungsgemäß insbesondere wie folgt verhalten:
- Insbesondere kann die erste Phase, insbesondere die erste Schicht, durch Verpressen, vorzugsweise Verpressen der die erste Phase, insbesondere erste Schicht, ausbildenden Wirk- und Inhaltsstoffe, erhalten sein und/oder als Pressling ausgebildet sein bzw. vorliegen.
   Insbesondere kann die erste Phase, insbesondere die erste Schicht, durch Verpressen des bevorzugt in Form von Ibuprofen vorliegenden NSAR-Wirkstoffs, insbesondere des granulatförmigen NSAR-Wirkstoffs, vorzugsweise des insbesondere mit einer cellulosebasierten Beschichtung, bevorzugt auf Basis von Hydroxypropylmethylcellulose (HPMC), ausgerüsteten Granulats des NSAR-Wirkstoffs, insbesondere wie zuvor definiert, insbesondere durch gemeinsam mit den weiteren Inhaltsstoffen der ersten Phase, bevorzugt der ersten Schicht, hergestellt sein.
   Insbesondere kann die erste Phase, insbesondere die erste Schicht, den NSAR-Wirkstoff, bevorzugt Ibuprofen, in Form eines verpressten (komprimierten) Granulats, vorzugsweise in Form eines verpressten und insbesondere mit einer cellulosebasierten Beschichtung, bevorzugt auf Basis von Hydroxypropylmethylcellulose (HPMC), ausgerüsteten Granulats, insbesondere wie zuvor definiert, insbesondere in Form eines gemeinsam mit den weiteren Inhaltsstoffen der ersten Phase, bevorzugt der ersten Schicht verpressten Granulats, aufweisen.
   Erfindungsgemäß kann das Verpressen, insbesondere das Verpressen zur Herstellung der ersten Phase, insbesondere der ersten Schicht, mit einer einwirkenden, insbesondere auf die Grundfläche der ersten Phase, insbesondere der ersten Schicht, einwirkenden, Presskraft im Bereich von 0,5 N bis 5 N, insbesondere im Bereich von 0,75 N bis 4 N, vorzugsweise im Bereich von 1 N bis 3 N, erfolgt sein.
   Weiterhin kann das Verpressen, insbesondere das Verpressen zur Herstellung der ersten Phase, insbesondere der ersten Schicht, mit einem Pressdruck im Bereich von 0,001 N/mm² bis 0,25 N/mm², insbesondere im Bereich von 0,003 N/mm² bis 0,05 N/mm², vorzugsweise im Bereich von 0,005 N/mm² bis 0,03 N/mm², bezogen auf die Grundfläche der ersten Phase, insbesondere der ersten Schicht, erfolgt sein.
- Erfindungsgemäß ist es zudem bevorzugt, dass die zweite Phase, insbesondere die zweite Schicht, durch Verpressen, vorzugsweise Verpressen der die zweite Phase, insbesondere zweite Schicht, ausbildenden Wirk- und Inhaltsstoffe, erhalten ist und/oder als Pressling ausgebildet ist bzw. vorliegt.
   Insbesondere kann die zweite Phase, insbesondere die zweite Schicht, durch Verpressen des bevorzugt in Form von Lidocainhydrochlorid vorliegenden Lokalanästhetikums, insbesondere durch gemeinsames Verpressen mit den weiteren Inhaltsstoffen der zweiten Phase, bevorzugt der zweiten Schicht, hergestellt sein.
   Weiterhin kann die zweite Phase, insbesondere die zweite Schicht, das Lokalanästhetikum, bevorzugt Lidocainhydrochlorid, in verpresster Form, insbesondere in gemeinsam mit den weiteren Inhaltsstoffen der zweiten Phase, bevorzugt der zweiten Schicht, verpresster Form aufweisen.
   Insbesondere kann das Verpressen, insbesondere das Verpressen zur Herstellung der zweiten Phase, insbesondere der zweiten Schicht, mit einer einwirkenden, insbesondere auf die Grundfläche der zweiten Phase, insbesondere der zweiten Schicht, einwirkenden, Presskraft im Bereich von 3 N bis 10 N, insbesondere im Bereich von 3,5 N bis 8 N, vorzugsweise im Bereich von 4 N bis 6 N, erfolgt sein.
   Zudem kann das Verpressen, insbesondere das Verpressen zur Herstellung der zweiten Phase, insbesondere der zweiten Schicht, mit einem Pressdruck im Bereich von 0,01 N/mm² bis 0,5 N/mm², insbesondere im Bereich von 0,02 N/mm² bis 0,1 N/mm², vorzugsweise im Bereich von 0,02 N/mm² bis 0,06 N/mm², bezogen auf die Grundfläche der zweiten Phase, insbesondere der zweiten Schicht, erfolgt sein.

Erfindungsgemäß ist es im Hinblick auf die Herstellung bzw. Bereitstellung der Darreichungsform bzw. Tablette nach der Erfindung von weiterführendem Vorteil, wenn die zweite Phase, insbesondere die zweite Schicht, mit einer größeren Presskraft ([N]) und/oder einem größeren Pressdruck ([N/mm²]) als die erste Phase, insbesondere erste Schicht, hergestellt und/oder verpresst ist.

Gleichermaßen ist es erfindungsgemäß von Vorteil, wenn die zum Verpressen der zweiten Phase, insbesondere der zweiten Schicht, vorzugsweise die zum das Verpressen zur Herstellung der zweiten Phase, insbesondere der zweiten Schicht, einwirkende, insbesondere auf die Grundfläche der zweiten Phase, insbesondere der zweiten Schicht, einwirkende, Presskraft ([N]) größer ist als die zum Verpressen der ersten Phase, insbesondere der ersten Schicht, vorzugsweise die zum Verpressen zur Herstellung der ersten Phase, insbesondere der ersten Schicht, einwirkende, insbesondere auf die Grundfläche der ersten Phase, insbesondere der ersten Schicht, einwirkende, Presskraft ([N]).

Entsprechend ist es erfindungsgemäß von Vorteil, wenn der zum Verpressen der zweiten Phase, insbesondere der zweiten Schicht, eingesetzte Pressdruck ([N/mm²]) größer ist als der zum Verpressen der ersten Phase, insbesondere der ersten Schicht, eingesetzte Pressdruck ([N/mm²]).

Diesbezüglich kann es sich insbesondere wie folgt verhalten:
- So kann das Verhältnis der Presskraft ([N]) der zweiten Phase, insbesondere der zweiten Schicht, zur Presskraft ([N]) der ersten Phase, insbesondere der ersten Schicht, im Bereich von 1,05 : 1 bis 20 : 1, insbesondere im Bereich von 1,1 : 1 bis 10 : 1, vorzugsweise im Bereich von 1,2 : 1 bis 6 : 1, bevorzugt im Bereich von 1,5 : 1 bis 4 : 1, liegen.
- Weiterhin kann das Verhältnis des Pressdrucks ([N/mm²]) der zweiten Phase, insbesondere der zweiten Schicht, zum Pressdruck ([N/mm²]) der ersten Phase, insbesondere der ersten Schicht, im Bereich von 1,05 : 1 bis 20 : 1, insbesondere im Bereich von 1,1 : 1 bis 10 : 1, vorzugsweise im Bereich von 1,2 : 1 bis 6 : 1, bevorzugt im Bereich von 1,5 : 1 bis 4 : 1, liegen.

Durch die unterschiedliche Presskraft bzw. den unterschiedlichen Pressdruck für die einzelnen Phasen bzw. Schichten kann - ohne sich auf diese Theorie beschränken oder berufen zu wollen - ein differenzierter bzw. unterschiedlicher Verpressgrad bzw. Verdichtung bzw. Komprimierung eingestellt werden, was wiederum den Zerfall bzw. die Wirkstofffreisetzung bei Kontakt mit Flüssigkeit bzw. Speichel beeinflusst, nämlich insbesondere dahingehend, dass bei höherem Verpressen bzw. Verdichtung ein langsamerer bzw. verzögerter Zerfall bzw. Wirkstofffreisetzung vorliegt, wie es für die zweite Phase der Fall ist. Weiterhin kann durch das vergleichsweise geringe Verpressen bzw. Verdichten ein schnellerer Zerfall bzw. Wirkstofffreisetzung eingestellt sein, wie es für die erste Phase der Fall ist. Auf diese Weise kann somit insbesondere im Zusammenwirken mit den weiteren erfindungsgemäßen Maßnahmen ein differenzierter bzw. unterschiedlicher Zerfall bzw. Wirkstofffreisetzung vorgegeben werden.

Was die Bereitstellung der Darreichungsform bzw. Tablette nach der Erfindung weiterhin anbelangt, so ist es erfindungsgemäß von Vorteil, wenn die erste Phase, insbesondere die erste Schicht, und die zweite Phase, insbesondere die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, und/oder insbesondere wie zuvor definiert, hergestellt sind.

Erfindungsgemäß ist es dabei von Vorteil, wenn zunächst die zweite Phase, insbesondere die zweite Schicht, hergestellt und/oder verpresst ist, insbesondere zuvor definiert, und dass nachfolgend die erste Phase, insbesondere die erste Schicht, hergestellt und/oder verpresst ist, insbesondere wie zuvor definiert. Erfindungsgemäß ist es dabei bevorzugt, dass die erste Phase, insbesondere die erste Schicht, auf der zweiten Phase, insbesondere auf der zweiten Schicht, vorzugsweise auf einer der Grundflächen der zweiten Phase, insbesondere der zweiten Schicht, hergestellt und/oder verpresst ist, insbesondere wie zuvor definiert.

Weiterhin kann die Darreichungsform, insbesondere Tablette, nach der Erfindung durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten sein.

In diesem Zusammenhang kann es sich erfindungsgemäß insbesondere wie folgt verhalten:
- So kann das (End-)Verpressen, insbesondere das (End-) Verpressen zur Herstellung der Darreichungsform, insbesondere der Tablette, mit einer einwirkenden, insbesondere auf die Grundfläche der Darreichungsform einwirkenden, Presskraft im Bereich von 15 N bis 40 N, insbesondere im Bereich von 20 N bis 30 N, vorzugsweise im Bereich von 22 N bis 28 N, erfolgt sein.
- Zudem kann das (End-)Verpressen, insbesondere das (End-) Verpressen zur Herstellung der Darreichungsform, insbesondere der Tablette, mit einem Pressdruck im Bereich von 0,04 N/mm² bis 1 N/mm², insbesondere im Bereich von 0,06 N/mm² bis 0,5 N/mm², vorzugsweise im Bereich von 0,1 N/mm² bis 0,3 N/mm², bezogen auf die Grundfläche der Darreichungsform, insbesondere der Tablette, erfolgt sein.

Gemäß einer speziellen erfindungsgemäßen Ausführungsform kann es sich insbesondere wie folgt verhalten: So kann die erfindungsgemäße Darreichungsform, insbesondere die Tablette, durch Verpressen, vorzugsweise Verpressen der die Darreichungsform, insbesondere die Tablette, ausbildenden Wirk- und Inhaltsstoffe, erhalten bzw. hergestellt sein. Insbesondere kann die Darreichungsform, insbesondere die Tablette, als Pressling ausgebildet sein bzw. vorliegen,
wobei zunächst die zweite Phase, insbesondere die zweite Schicht, erhalten und/oder hergestellt, insbesondere verpresst, ist, wobei die zweite Phase, insbesondere die zweite Schicht, durch Verpressen des in Form von Lidocainhydrochlorid vorliegenden Lokalanästhetikums, insbesondere durch gemeinsames Verpressen mit den weiteren Inhaltsstoffen der zweiten Phase, bevorzugt der zweiten Schicht, erhalten und/oder hergestellt, insbesondere verpresst, ist, wobei das Verpressen zur Herstellung der zweiten Phase, insbesondere der zweiten Schicht, mit einer Presskraft im Bereich von 4 N bis 6 N und/oder mit einem Pressdruck im Bereich von 0,02 N/mm² bis 0,06 N/mm², bezogen auf die Grundfläche der zweiten Phase, insbesondere der zweiten Schicht, erfolgt ist, und
wobei nachfolgend die erste Phase, insbesondere die erste Schicht, durch Verpressen des in Form von Ibuprofen vorliegenden NSAR-Wirkstoffs, vorzugsweise des insbesondere mit einer cellulosebasierten Beschichtung, bevorzugt auf Basis von Hydroxypropylmethylcellulose (HPMC), ausgerüsteten Granulats des NSAR-Wirkstoffs, insbesondere wie zuvor definiert, insbesondere durch gemeinsames Verpressen mit den weiteren Inhaltsstoffen der ersten Phase, bevorzugt der ersten Schicht, erhalten und/oder hergestellt, insbesondere verpresst, ist, wobei das Verpressen zur Herstellung der ersten Phase, insbesondere der ersten Schicht, mit einer Presskraft im Bereich von 1 N bis 3 N und/oder mit einem Pressdruck im Bereich von 0,005 N/mm² bis 0,03 N/mm², bezogen auf die Grundfläche der ersten Phase, insbesondere der ersten Schicht, erfolgt ist, und
wobei die Darreichungsform, insbesondere die Tablette, durch nachfolgend gemeinsames (End-)Verpressen der zuvor erhaltenen und/oder hergestellten, insbesondere verpressten, ersten Phase, insbesondere ersten Schicht, und der zuvor erhaltenen und/oder hergestellten, insbesondere verpressten, zweiten Phase, insbesondere zweiten Schicht, erhalten ist, wobei das (End-)Verpressen (End-)Verpressen zur Herstellung der Darreichungsform, insbesondere der Tablette, mit einer Presskraft im Bereich von 22 N bis 28 N und/oder mit einem Pressdruck im Bereich von 0,1 N/mm² bis 0,3 N/mm², bezogen auf die Grundfläche der Darreichungsform, insbesondere der Tablette, erfolgt ist,
insbesondere wobei die erste Phase, insbesondere die erste Schicht, auf der zweiten Phase, insbesondere zweiten Schicht, erhalten und/oder hergestellt, insbesondere verpresst, ist.

Erfindungsgemäß kann es insbesondere vorgesehen sein, dass der NSAR-Wirkstoff, bevorzugt Ibuprofen, gemeinsam mit den weiteren Inhaltsstoffen, insbesondere gemeinsam mit dem Spreng- und/oder Zerfallsmittel und/oder dem (ersten) insbesondere zerfallsunterstützenden Füll- und/oder Trägerstoff, zumindest im Wesentlichen homogen verteilt und/oder in zumindest im Wesentlichen inniger Mischung in der ersten Phase, insbesondere ersten Schicht, vorliegt bzw. in Form einer innigen bzw. homogenen Mischung mit den weiteren Inhaltsstoffen verpresst ist.

Erfindungsgemäß kann es zudem insbesondere vorgesehen sein, dass das Lokalanästhetikum, bevorzugt Lidocainhydrochlorid, gemeinsam mit den weiteren Inhaltsstoffen, insbesondere gemeinsam dem (zweiten) insbesondere nichtzerfallsunterstützenden Füll- und/oder Trägerstoff, zumindest im Wesentlichen homogen verteilt und/oder in zumindest im Wesentlichen inniger Mischung in der zweiten Phase, insbesondere zweiten Schicht, vorliegt bzw. in Form einer innigen bzw. homogenen Mischung mit den weiteren Inhaltsstoffen verpresst ist.

Erfindungsgemäß kann es sich insbesondere derart verhalten, dass durch gezielte Abstimmung bzw. Auswahl der jeweiligen Inhaltsstoffe der zugrundeliegenden Phasen bzw. Schichten die wechselseitige Verbindung bzw. Haftung der Phasen bzw. Schichten weiterführend verbessert, insbesondere gezielt gesteuert bzw. eingestellt und somit sozusagen maßgeschneidert, werden kann. Hierdurch kann die Verbindungs- bzw. Haftstärke zwischen den Phasen bzw. Schichten weiter eingestellt werden, was auch der physikalischen Stabilität des resultierenden Verbundes zugute kommt.

Weiterhin ist es erfindungsgemäß insbesondere vorgesehen, dass die Darreichungsform, insbesondere die Tablette, (als solche) zumindest im Wesentlichen unbeschichtet ist bzw. dass die Darreichungsform, insbesondere die Tablette, keine (Außen-)Beschichtung, insbesondere keine die Darreichungsform, insbesondere die Tablette, (als solche) umhüllende Beschichtung, aufweist. Hierdurch ist bei oraler Applikation ein unmittelbarer Kontakt mit Flüssigkeit, insbesondere Speichel gewährleistet, was zu einem entsprechend unmittelbaren Beginn der Zerfalls- und Wirkstofffreisetzungsprozesse führt.

Nachfolgend werden weitere spezielle bzw. bevorzugte Ausführungsformen der vorliegenden Erfindung beschrieben:
- Insbesondere kann die erste Phase, insbesondere die erste Schicht, den NSAR-Wirkstoff, insbesondere Ibuprofen, in Form eines verpressten Granulats, insbesondere auf Basis vorzugsweise diskreter Teilchen und/oder Partikel, enthält, wobei das Granulat eine insbesondere wasserlösliche Beschichtung (Coating), vorzugsweise cellulosebasierte Beschichtung, bevorzugt auf Basis von Hydroxypropylmethylcellulose (HPMC), aufweisen. Weiterführend kann die erste Phase, insbesondere die erste Schicht, den NSAR-Wirkstoff, insbesondere Ibuprofen, in Form von mit den weiteren Inhaltsstoffen der ersten Phase, insbesondere ersten Schicht, verpressten Granulaten des NSAR-Wirkstoff, insbesondere Ibuprofen, enthält, wobei das Granulat eine insbesondere wasserlösliche Beschichtung (Coating), vorzugsweise cellulosebasierte Beschichtung, bevorzugt auf Basis von Hydroxypropylmethylcellulose (HPMC), aufweisen.
- Erfindungsgemäß kann es sich zudem derart verhalten, dass die erste Phase, insbesondere die erste Schicht, neben dem NSAR-Wirkstoff, insbesondere Ibuprofen, vorzugsweise granulatförmigem Ibuprofen, (i) mindestens ein Spreng- und/oder Zerfallsmittel, insbesondere wie zuvor definiert, (ii) mindestens einen zerfallsunterstützenden Füll- und/oder Trägerstoff, insbesondere wie in zuvor definiert, (iii) mindestens ein Säuerungsmittel, insbesondere wie zuvor definiert, (iv) mindestens ein geschmacksmodifizerendes und/oder geschmacksmaskierendes Mittel, insbesondere wie zuvor definiert, sowie gegebenenfalls (v) mindestens ein Additiv, insbesondere mindestens ein Fließmittel und/oder mindestens ein Schmiermittel, insbesondere wie zuvor definiert, aufweist und
   dass die zweite Phase, insbesondere die zweite Schicht, neben dem Lokalanästhetikum, insbesondere Lidocainhydrochlorid, (i) mindestens einen nichtzerfallsunterstützenden Füll- und/oder Trägerstoff, insbesondere wie zuvor definiert, (ii) mindestens ein Säuerungsmittel, insbesondere wie zuvor definiert, (iii) mindestens ein geschmacksmodifizerendes und/oder geschmacksmaskierendes Mittel, insbesondere wie zuvor definiert, sowie gegebenenfalls (v) mindestens ein Additiv, insbesondere mindestens ein Fließmittel und/oder mindestens ein Schmiermittel, insbesondere wie zuvor definiert, aufweist.
   In diesem Zusammenhang ist es zudem bevorzugt, dass die zweite Phase, insbesondere die zweite Schicht, zumindest im Wesentlichen kein wie zuvor definiertes Spreng- und/oder Zerfallsmittel, insbesondere zumindest im Wesentlichen kein Spreng- und/oder Zerfallsmittel, aufweist bzw. zumindest im Wesentlichen frei von einem wie zuvor definierten Spreng- und/oder Zerfallsmittel, insbesondere zumindest im Wesentlichen frei von einem Spreng- bzw. Zerfallsmittel, ist.

Auf Basis der vorgenannten Maßnahmen können besonders definierte Zerfalls- und Wirkstofffreisetzungseigenschaften vorgegeben werden, und zwar auch im Hinblick auf die Ausbildung der ersten Phase bzw. der ersten Schicht mit schnellem bzw. unmittelbarem Zerfall bzw. Wirkstofffreisetzung und der zweiten Phase bzw. zweiten Schicht mit langsamem bzw. retardiertem Zerfall bzw. Wirkstofffreisetzung.

Gemäß einer weiteren speziellen Ausführungsform der vorliegenden Erfindung kann es sich insbesondere derart verhalten, dass die erste Phase, insbesondere die erste Schicht, neben dem NSAR-Wirkstoff, insbesondere Ibuprofen, vorzugsweise granulatförmigem Ibuprofen,
(i) mindestens ein Spreng- und/oder Zerfallsmittel in Form von Croscarmellose-Natrium und/oder Polyvinylpolypyrrolidon (Crospovidon), bevorzugt Polyvinylpolypyrrolidon (Crospovidon), in einer (relativen) Menge im Bereich von 3 Gew.-% bis 90 Gew.-%, bevorzugt im Bereich von 15 Gew.-% bis 25 Gew.-%, bezogen auf die erste Phase (Trockengewicht), insbesondere auf die erste Schicht (Trockengewicht),
(ii) mindestens einen zerfallsunterstützenden Füll- und/oder Trägerstoff auf Basis von Zuckeralkoholen, bevorzugt Mannitol, in einer (relativen) Menge im Bereich von 5 Gew.-% bis 90 Gew.-%, bevorzugt im Bereich von 30 Gew.-% bis 45 Gew.-%, bezogen auf die erste Phase (Trockengewicht), insbesondere auf die erste Schicht (Trockengewicht),
(iii) mindestens ein Säuerungsmittel in Form einer organischen Säure oder deren Salze und Ester, bevorzugt Citronensäure, in einer (relativen) Menge im Bereich von 0,01 Gew.-% bis 10 Gew.-%, bevorzugt im Bereich von 0,5 Gew.-% bis 2,5 Gew.-%, bezogen auf die erste Phase (Trockengewicht), insbesondere auf die erste Schicht (Trockengewicht),
(iv) mindestens ein geschmacksmodifizerendes und/oder geschmacksmaskierendes Mittel, insbesondere in Form von Erdalkali- und/oder Alkalilaurylsulfat, bevorzugt Natriumlaurylsulfat, in einer (relativen) Menge im Bereich von 0,02 Gew.-% bis 10 Gew.-%, bevorzugt im Bereich von 0,08 Gew.-% bis 2 Gew.-%, bezogen auf die erste Phase (Trockengewicht), insbesondere auf die erste Schicht (Trockengewicht),
(v) gegebenenfalls mindestens ein Additiv, insbesondere mindestens ein Fließmittel und/oder mindestens ein Schmiermittel, insbesondere wie in Anspruch 28 definiert, aufweist und

dass die zweite Phase, insbesondere die zweite Schicht, neben dem Lokalanästhetikum, insbesondere Lidocainhydrochlorid,
   (i) mindestens einen nichtzerfallsunterstützenden Füll- und/oder Trägerstoff auf Basis von Zuckeralkoholen, bevorzugt Sorbitol und/oder Isomalt, in einer (relativen) Menge im Bereich von 10 Gew.-% bis 98 Gew.-%, bevorzugt im Bereich von 30 Gew.-% bis 85 Gew.-%, bezogen auf die zweite Phase (Trockengewicht), insbesondere auf die zweite Schicht (Trockengewicht),
   (ii) mindestens ein Säuerungsmittel in Form einer organischen Säure oder deren Salze und Ester, bevorzugt Citronensäure, in einer (relativen) Menge im Bereich von 0,01 Gew.-% bis 10 Gew.-%, bevorzugt im Bereich von 0,5 Gew.-% bis 2,5 Gew.-%, bezogen auf die zweite Phase (Trockengewicht), insbesondere auf die zweite Schicht (Trockengewicht),
   (iii) mindestens ein geschmacksmodifizerendes und/oder geschmacksmaskierendes Mittel in Form von Cyclodextrin und/oder Natriumlaurylsulfat, insbesondere Cyclodextrin, in einer (relativen) Menge im Bereich von 0,01 Gew.-% bis 25 Gew.-%, bevorzugt im Bereich von 0,2 Gew.-% bis 10 Gew.-%, bezogen auf die zweite Phase (Trockengewicht), insbesondere auf die zweite Schicht (Trockengewicht),
   (v) gegebenenfalls mindestens ein Additiv, insbesondere mindestens ein Fließmittel und/oder mindestens ein Schmiermittel, insbesondere wie zuvor definiert,
   aufweist,
wobei die erste Phase, insbesondere die erste Schicht, den NSAR-Wirkstoff in einer (relativen) Menge im Bereich von 5 Gew.-% bis 90 Gew.-%, bevorzugt im Bereich von 20 Gew.-% bis 30 Gew.-%, berechnet als Ibuprofen und bezogen auf die erste Phase (Trockengewicht), insbesondere auf die erste Schicht (Trockengewicht), aufweist und
wobei die zweite Phase, insbesondere die zweite Schicht, das Lokalanästhetikum in einer (relativen) Menge im Bereich von 0,1 Gew.-% bis 20 Gew.-%, bevorzugt im Bereich von 0,5 Gew.-% bis 1,5 Gew.-%, berechnet als Lidocain und bezogen auf die zweite Phase (Trockengewicht), insbesondere auf die zweite Schicht (Trockengewicht), aufweist.

In diesem Zusammenhang kann es insbesondere vorgesehen sein, dass die zweite Phase, insbesondere die zweite Schicht, zumindest im Wesentlichen kein wie zuvor definiertes Spreng- und/oder Zerfallsmittel, insbesondere zumindest im Wesentlichen kein Spreng- und/oder Zerfallsmittel, aufweist bzw. zumindest im Wesentlichen frei von einem wie zuvor definierten Spreng- und/oder Zerfallsmittel, insbesondere zumindest im Wesentlichen frei von einem Spreng- und/oder Zerfallsmittel, ist.

Erfindungsgemäß können unter Verwendung der erfindungsgemäßen Darreichungsformen eine Vielzahl verschiedener entzündlicher Erkrankungen des Mund/Rachen-Raums, welche insbesondere schmerzbegleitet sind, behandelt werden:
Im Rahmen der vorliegenden Erfindung können die insbesondere schmerzbegleiteten entzündlichen Erkrankungen des Mund/Rachen-Raums bakteriell, mykotisch und/oder viral hervorgerufene Erkrankungen sein. Zudem können die insbesondere schmerzbegleiteten entzündlichen Erkrankungen des Mund/Rachen-Raums mit Hals- und/oder Rachenschmerzen, insbesondere Halsschmerzen, einhergehende Erkrankungen sein. Weiterhin können die insbesondere schmerzbegleiteten entzündlichen Erkrankungen des Mund/Rachen-Raums mit Schmerzen an Mund- und/oder Rachen- und/oder Halsschleimhaut und/oder mit Zahnfleischschmerzen einhergehende Erkrankungen sein.

Erfindungsgemäß können die insbesondere schmerzbegleiteten entzündlichen Erkrankungen des Mund/Rachen-Raums weiterhin mit Heiserkeit und/oder Räusperzwang und/oder Schluckbeschwerden einhergehende Erkrankungen sein.

Darüber hinaus können die insbesondere schmerzbegleiteten entzündlichen Erkrankungen des Mund/Rachen-Raums ausgewählt sein aus der Gruppe von Erkältungskrankheiten, grippalen Infekten, Grippe (Influenza), Coronavirus-Erkrankungen, wie COVID-19 (Coronavirus SARS-CoV-2), und deren Kombinationen.

Insbesondere können die insbesondere schmerzbegleiteten entzündlichen Erkrankungen des Mund/Rachen-Raums ausgewählt sein aus der Gruppe von Halsentzündungen, insbesondere Angina; Entzündungen des Kehlkopfs, insbesondere Laryngitis; Entzündungen der Rachenschleimhaut, insbesondere Pharyngitis; Entzündungen der (Rachen-) Mandeln, insbesondere Tonsillitis; Entzündungen im Bereich der Mundhöhle, insbesondere Stomatitis, Gingivitis, Mundschleimhautläsionen, wie Aphthen, Mundschleimhautentzündungen; und deren Kombinationen.

Gleichermaßen können die insbesondere schmerzbegleiteten entzündlichen Erkrankungen des Mund/Rachen-Raums Halsschmerzen von einer insbesondere übergeordneten Erkrankung, ausgewählt aus der Gruppe von Scharlach, EBV-Infektionen (Pfeiffersches Drüsenfieber), Diphtherie und deren Kombinationen, hervorgerufen bzw. hierdurch bedingt sein.

Erfindungsgemäß können die insbesondere schmerzbegleiteten entzündlichen Erkrankungen des Mund/Rachen-Raums auch Verletzungen und/oder (Schleimhaut-)Läsionen im Mund/Rachen-Raum, insbesondere Verletzungen der Mundschleimhaut und/oder des Zahnfleischs, sein.

Die vorliegende Erfindung betrifft auch die erfindungsgemäße Darreichungsform zur Verwendung bei der prophylaktischen und/oder therapeutischen Behandlung von Schmerzen und/oder Entzündungen insbesondere im Mund/Rachen-Raum, vorzugsweise von im Zusammenhang mit operativen und/oder diagnostischen Eingriffen im Mund/Rachen-Raum stehenden bzw. hiervon hervorgerufenen Schmerzen und/oder Entzündungen.

Insbesondere kann die Darreichungsform nach der Erfindung zur Verwendung bei der prophylaktischen und/oder therapeutischen Behandlung von postoperativen Schmerzen bzw. Entzündungen insbesondere im Mund/Rachen-Raum oder von nach diagnostischen Eingriffen vorliegenden Schmerzen und/oder Entzündungen insbesondere im Mund/Rachen-Raum eingesetzt werden, beispielsweise im Zusammenhang mit Mundschleimhaut- bzw. Zahnfleischbehandlungen, endotrachealen Intubationen oder dergleichen.

Insbesondere kann die erfindungsgemäße Darreichungsform zur Verwendung bei der Prävention und/oder prophylaktischen Behandlung von Schmerzen und/oder Entzündungen insbesondere im Mund/Rachen-Raum, insbesondere zur lokalen Anästhesie im Mund/Rachen-Raum und/oder als (Lokal-)Anästhetikum verwendet werden, beispielsweise im Zusammenhang mit operativen oder diagnostischen Eingriffen.

In diesem Zusammenhang kann die erfindungsgemäße Darreichungsform somit im Rahmen einer Postmedikation sowie einer Prämedikation eingesetzt werden. Insbesondere kann die erfindungsgemäße Darreichungsform zur Behandlung von akuten oder chronischen Schmerzen und/oder Entzündungen im Mund/Rachen-Raum eingesetzt bzw. verwendet werden. Darüber hinaus kann die erfindungsgemäße Darreichungsform präventiv bzw. zur prophylaktischen Behandlung im Hinblick auf Schmerzen und Entzündungen insbesondere im Mund/Rachen-Raum verwendet werden.

Im Zusammenhang mit der Applikation bzw. Verabreichung der erfindungsgemäßen Darreichungsform ist es erfindungsgemäß bevorzugt, wenn die Darreichungsform mit einer Einzeldosis an NSAR-Wirkstoff, bevorzugt Ibuprofen, im Bereich von 50 mg bis 800 mg, insbesondere im Bereich von 100 mg bis 400 mg, vorzugsweise im Bereich von 150 mg bis 300 mg, bevorzugt im Bereich von 190 mg bis 210 mg, verabreicht wird. Insbesondere kann die Darreichungsform nach der Erfindung zur Verabreichung einer Einzeldosis an NSAR-Wirkstoff, bevorzugt Ibuprofen, im Bereich von 50 mg bis 800 mg, insbesondere im Bereich von 100 mg bis 400 mg, vorzugsweise im Bereich von 150 mg bis 300 mg, bevorzugt im Bereich von 190 mg bis 210 mg, hergerichtet sein.

Gleichermaßen ist es erfindungsgemäß bevorzugt, wenn die Darreichungsform mit einer Einzeldosis an Lokalanästhetikum, bevorzugt Lidocainhydrochlorid, im Bereich von 2 mg bis 50 mg, insbesondere im Bereich von 4 mg bis 20 mg, vorzugsweise im Bereich von 5 mg bis 10 mg, bevorzugt im Bereich von 6 mg bis 9 mg, verabreicht wird. Diesbezüglich kann es erfindungsgemäß insbesondere vorgesehen sein, dass die Darreichungsform zur Verabreichung einer Einzeldosis an Lokalanästhetikum, bevorzugt Lidocainhydrochlorid, im Bereich von 2 mg bis 50 mg, insbesondere im Bereich von 4 mg bis 20 mg, vorzugsweise im Bereich von 5 mg bis 10 mg, bevorzugt im Bereich von 6 mg bis 9, hergerichtet ist.

Insbesondere in Bezug auf die zuvor genannten Einzeldosen kann es erfindungsgemäß vorgesehen sein, dass die Darreichungsform ein- bis sechsmal pro Tag, insbesondere zwei- bis dreimal pro Tag, verabreicht bzw. eingenommen wird bzw. zur diesbezüglichen Verabreichung hergerichtet ist, vorzugsweise über den Tag verteilt.

Insbesondere kann es erfindungsgemäß vorgesehen sein, den NSAR-Wirkstoff, bevorzugt Ibuprofen, mit einer Tagesdosis im Bereich von 50 mg/diem bis 1.200 mg/diem, insbesondere im Bereich von 75 mg/diem bis 1.000 mg/diem, vorzugsweise im Bereich von 120 mg/diem bis 800 mg/diem, bevorzugt im Bereich von 150 mg/diem bis 400 mg/diem, zu verabreichen. Zudem kann das Lokalanästhetikum, bevorzugt Lidocainhydrochlorid, mit einer Tagesdosis im Bereich von 1 mg/diem bis 150 mg/diem, insbesondere im Bereich von 3 mg/diem bis 50 mg/diem, vorzugsweise im Bereich von 4 mg/diem bis 20 mg/diem, bevorzugt im Bereich von 4,75 mg/diem bis 18 mg/diem, verabreicht werden.

Die vorliegende Erfindung betrifft gemäß dem vorliegenden Aspekt auch folgende spezielle Ausführungsformen der erfindungsgemäßen Darreichungsform:
So betrifft die vorliegende Erfindung gemäß dem vorliegenden Aspekt auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure), wobei die erste Phase, bevorzugt die erste Schicht, den NSAR-Wirkstoff in Form eines vorzugsweise verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

Weiterhin betrifft die vorliegende Erfindung gemäß dem vorliegenden Aspekt auch die Darreichungsform nach der Erfindung, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
      wobei die Darreichungsform, insbesondere die Tablette, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweist und/oder auf die vorgenannten Werte eingestellt ist.

Gemäß dem vorliegenden Aspekt betrifft die vorliegende Erfindung weiterhin auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei das Verhältnis der Zerfallszeit der zweiten Phase, insbesondere der zweiten Schicht, zur Zerfallszeit der ersten Phase, insbesondere der ersten Schicht im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind, und/oder
wobei das Verhältnis der Wirkstofffreisetzungsgeschwindigkeit der ersten Phase, insbesondere der ersten Schicht, zur Wirkstofffreisetzungsgeschwindigkeit der zweiten Phase, insbesondere der zweiten Schicht im Bereich von 50 : 1 bis 10.000 : 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

Zudem betrifft die vorliegende Erfindung gemäß dem vorliegenden Aspekt weiterhin auch die Darreichungsform nach der Erfindung, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform, wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die erste Phase, insbesondere die erste Schicht, und die zweite Phase, insbesondere die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, hergestellt sind und wobei die Darreichungsform, insbesondere die Tablette, durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten ist.

Zudem betrifft die vorliegende Erfindung gemäß dem vorliegenden Aspekt auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die erste Phase, insbesondere die erste Schicht, und die zweite Phase, insbesondere die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, hergestellt sind,
wobei die zweite Phase, insbesondere die zweite Schicht, mit einer größeren Presskraft ([N]) und/oder einem größeren Pressdruck ([N/mm²]) als die erste Phase, insbesondere erste Schicht, hergestellt und/oder verpresst ist, und
wobei die Darreichungsform, insbesondere die Tablette, durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten ist.

In diesem Zusammenhang betrifft die vorliegende Erfindung gemäß dem vorliegenden Aspekt auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure), wobei die erste Phase, bevorzugt die erste Schicht, den NSAR-Wirkstoff in Form eines vorzugsweise verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die Darreichungsform, insbesondere die Tablette, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweist und/oder auf die vorgenannten Werte eingestellt ist; und
wobei das Verhältnis der Zerfallszeit der zweiten Phase, insbesondere der zweiten Schicht, zur Zerfallszeit der ersten Phase, insbesondere der ersten Schicht, im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind, und/oder
wobei das Verhältnis der Wirkstofffreisetzungsgeschwindigkeit der ersten Phase, insbesondere der ersten Schicht, zur Wirkstofffreisetzungsgeschwindigkeit der zweiten Phase, insbesondere der zweiten Schicht, im Bereich von 50 : 1 bis 10.000 : 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

Weiterhin betrifft die vorliegende Erfindung gemäß dem vorliegenden Aspekt auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen sowie dessen pharmazeutisch verträglichen Salzen und Estern, bevorzugt Ibuprofen, wobei die erste Phase, bevorzugt die erste Schicht, den NSAR-Wirkstoff in Form eines verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain sowie dessen pharmazeutisch verträglichen Salzen und Estern, bevorzugt Lidocainhydrochlorid,
wobei die erste Phase, insbesondere die erste Schicht, und die zweite Phase, insbesondere die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, hergestellt sind, wobei die zweite Phase, insbesondere die zweite Schicht, mit einer größeren Presskraft ([N]) und/oder einem größeren Pressdruck ([N/mm²]) als die erste Phase, insbesondere erste Schicht, hergestellt und/oder verpresst ist, und wobei die Darreichungsform, insbesondere die Tablette, durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten ist;
wobei die Darreichungsform, insbesondere die Tablette, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweist und/oder auf die vorgenannten Werte eingestellt ist; und
wobei das Verhältnis der Zerfallszeit der zweiten Phase, insbesondere der zweiten Schicht, zur Zerfallszeit der ersten Phase, insbesondere der ersten Schicht, im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind, und/oder
wobei das Verhältnis der Wirkstofffreisetzungsgeschwindigkeit der ersten Phase, insbesondere der ersten Schicht, zur Wirkstofffreisetzungsgeschwindigkeit der zweiten Phase, insbesondere der zweiten Schicht, im Bereich von 50 : 1 bis 10.000 : 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

Gemäß dem vorliegenden Aspekt betrifft die vorliegende Erfindung auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
      wobei die erste Schicht, den NSAR-Wirkstoff in Form eines vorzugsweise verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

Weiterhin betrifft die Erfindung gemäß dem vorliegenden Aspekt auch die Darreichungsform nach der Erfindung, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
      wobei die Darreichungsform, insbesondere die Tablette, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweist und/oder auf die vorgenannten Werte eingestellt ist.

Insbesondere betrifft die Erfindung gemäß dem vorliegenden Aspekt auch die Darreichungsform nach der Erfindung, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei das Verhältnis der Zerfallszeit der zweiten Schicht zur Zerfallszeit der ersten Schicht im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind, und/oder
wobei das Verhältnis der Wirkstofffreisetzungsgeschwindigkeit der ersten Schicht zur Wirkstofffreisetzungsgeschwindigkeit der zweiten Schicht im Bereich von 50 : 1 bis 10.000 : 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

Die vorliegende Erfindung betrifft gemäß dem vorliegenden Aspekt weiterhin auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die erste Schicht und die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, hergestellt sind und wobei die Darreichungsform, insbesondere die Tablette, durch nachfolgend gemeinsames (End-)Verpressen der zuvor hergestellten und/oder verpressten ersten Schicht und der zuvor hergestellten und/oder verpressten zweiten Schicht erhalten ist.

Die Erfindung betrifft gemäß dem vorliegenden Aspekt weiterführend auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die erste Schicht und die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, hergestellt sind, wobei die zweite Phase, insbesondere die zweite Schicht, mit einer größeren Presskraft ([N]) und/oder einem größeren Pressdruck ([N/mm²]) als die erste Phase, insbesondere erste Schicht, hergestellt und/oder verpresst ist, und wobei die Darreichungsform, insbesondere die Tablette, durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten ist.

Zudem ist gemäß dem vorliegenden Aspekt erfindungsgemäß auch die erfindungsgemäße Darreichungsform, insbesondere pharmazeutische Darreichungsform, beschrieben, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
      wobei die erste Schicht, den NSAR-Wirkstoff in Form eines vorzugsweise verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die Darreichungsform, insbesondere die Tablette, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweist und/oder auf die vorgenannten Werte eingestellt ist; und
wobei das Verhältnis der Zerfallszeit der zweiten Schicht zur Zerfallszeit der ersten Schicht im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind, und/oder
wobei das Verhältnis der Wirkstofffreisetzungsgeschwindigkeit der ersten Schicht zur Wirkstofffreisetzungsgeschwindigkeit der zweiten Schicht im Bereich von 50 : 1 bis 10.000 : 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

Die vorliegende Erfindung betrifft gemäß dem vorliegenden Aspekt weiterführend auch die Darreichungsform nach der Erfindung, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
   - wobei eine erste Schicht als orodispersible Schicht (Schmelztabletten-Schicht) mit schneller und/oder unmittelbarer (sofortiger) Freisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)-propionsäure) sowie dessen pharmazeutisch verträglichen Salzen und Estern, bevorzugt Ibuprofen (2-(4-Isobutylphenyl)propionsäure),
      wobei die erste Schicht den NSAR-Wirkstoff in Form eines verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
   - wobei eine zweite Schicht als oral-transmukosale Schicht (Lutschtabletten-Schicht) mit langsamer und/oder retardierter (kontrollierter) Freisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid,
wobei die erste Schicht und die zweite Schicht, insbesondere unabhängig voneinander und/oder getrennt voneinander und/oder aufeinander abfolgend, jeweils einzeln durch Verpressen, vorzugsweise durch Verpressen der die jeweilige Phase, insbesondere Schicht, ausbildenden Wirk- und Inhaltsstoffe, hergestellt sind, wobei die zweite Schicht mit einer größeren Presskraft ([N]) und/oder einem größeren Pressdruck ([N/mm²]) als die erste Schicht hergestellt und/oder verpresst ist, und wobei die Darreichungsform, insbesondere die Tablette, durch nachfolgend gemeinsames (End-)Verpressen der zuvor hergestellten und/oder verpressten ersten Schicht und der zuvor hergestellten und/oder verpressten zweiten Schicht erhalten ist;
wobei die Darreichungsform, insbesondere die Tablette, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Darreichungsform (Trockengewicht), aufweist und/oder auf die vorgenannten Werte eingestellt ist; und
wobei das Verhältnis der Zerfallszeit der zweiten Schicht zur Zerfallszeit der ersten Schicht im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind, und/oder
wobei das Verhältnis der Wirkstofffreisetzungsgeschwindigkeit der ersten Schicht zur Wirkstofffreisetzungsgeschwindigkeit der zweiten Schicht im Bereich von 50 : 1 bis 10.000 : 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

Die vorliegende Erfindung betrifft gleichermaßen die Darreichungsform nach der Erfindung, wie sie gemäß dem nachfolgend noch beschriebenen Herstellungsverfahren erhältlich ist.

Gemäß dem vorliegenden Aspekt betrifft die vorliegende Erfindung insbesondere auch die erfindungsgemäße Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, insbesondere eine wie zuvor definierte Darreichungsform, wobei die Darreichungsform nach dem nachfolgend noch beschriebenen Herstellungsverfahren erhältlich ist.

Für weitergehende Einzelheiten zu dem vorstehend beschriebenen ersten Erfindungsaspekt kann zudem Bezug genommen werden auf die Ausführungen zu den weiteren Erfindungsaspekten, wobei diese Ausführungen gleichermaßen für den vorliegenden ersten Erfindungsaspekt entsprechend gelten.

Weiterhin ist vorliegend beschrieben die Verwendung einer Darreichungsform, insbesondere pharmazeutische Darreichungsform, vorzugsweise wie zuvor definiert, insbesondere für die orale Applikation, zur prophylaktischen und/oder therapeutischen Behandlung von insbesondere schmerzbegleiteten entzündlichen Erkrankungen des Mund/Rachen-Raums,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen sowie dessen pharmazeutisch verträglichen Salzen und Estern, bevorzugt Ibuprofen,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von sowie dessen pharmazeutisch verträglichen Salzen und Estern, bevorzugt Lidocainhydrochlorid.
Vorliegend beschrieben ist auch die Verwendung einer Darreichungsform, insbesondere pharmazeutischen Darreichungsform, vorzugsweise wie zuvor definiert, insbesondere für die orale Applikation, zur Herstellung eines Arzneimittels oder Medikaments zur prophylaktischen und/oder therapeutischen Behandlung von insbesondere schmerzbegleiteten entzündlichen Erkrankungen des Mund/Rachen-Raums,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen sowie dessen pharmazeutisch verträglichen Salzen und Estern, bevorzugt Ibuprofen,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain sowie dessen pharmazeutisch verträglichen Salzen und Estern, bevorzugt Lidocainhydrochlorid.

In diesem Zusammenhang betrifft die vorliegende Erfindung auch eine Darreichungsform, insbesondere pharmazeutische Darreichungsform, wie zuvor definiert, insbesondere für die orale Applikation, zur (Verwendung bei der) prophylaktischen und/oder therapeutischen Behandlung von insbesondere schmerzbegleiteten entzündlichen Erkrankungen des Mund/Rachen-Raums,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen sowie dessen pharmazeutisch verträglichen Salzen und Estern, bevorzugt Ibuprofen,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain sowie dessen pharmazeutisch verträglichen Salzen und Estern, bevorzugt Lidocainhydrochlorid.

Für weitergehende Einzelheiten zu den vorstehend beschriebenen Gegenständen kann zudem Bezug genommen werden auf die Ausführungen zu den weiteren Aspekten, wobei diese Ausführungen gleichermaßen für die vorliegend beschriebenen Gegenstände entsprechend gelten.

Zudem ist vorliegend beschrieben das Verfahren zur prophylaktischen und/oder therapeutischen Behandlung von insbesondere schmerzbegleiteten entzündlichen Erkrankungen des Mund/Rachen-Raums,
wobei bei dem Verfahren einem an den insbesondere schmerzbegleiteten entzündlichen Erkrankungen des Mund/Rachen-Raums erkrankten Patienten eine pharmazeutisch wirksame bzw. therapeutisch wirksame Menge einer Darreichungsform, insbesondere pharmazeutischen Darreichungsform, vorzugsweise wie zuvor definiert, insbesondere für die orale Applikation, verabreicht wird,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
   - wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen sowie dessen pharmazeutisch verträglichen Salzen und Estern, bevorzugt Ibuprofen,
   - wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain sowie dessen pharmazeutisch verträglichen Salzen und Estern, bevorzugt Lidocainhydrochlorid.

Für weitergehende Einzelheiten zu diesem Verfahren kann auf die Ausführungen zu den weiteren Aspekten verwiesen werden, welche im Hinblick auf das vorliegend beschriebene Verfahren entsprechend gelten.

Gegenstand der vorliegenden Erfindung - gemäß einem **weiteren** Aspekt der vorliegenden Erfindung - ist zudem das erfindungsgemäße Verfahren zur Herstellung einer Darreichungsform, insbesondere pharmazeutischen Darreichungsform, vorzugsweise der erfindungsgemäßen Darreichungsform, wie zuvor definiert, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums, wobei die Darreichungsform als mehrphasige, insbesondere mindestens zweiphasige, vorzugsweise zweiphasige, Tablette, besonders bevorzugt als Zweischicht-Tablette, ausgebildet wird, wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
- wobei eine erste Phase, insbesondere erste Schicht, einen NSAR-Wirkstoff (nichtsteroidales Antirheumatikum) aufweist, wobei der NSAR-Wirkstoff ausgewählt wird aus der Gruppe von Ibuprofen sowie dessen pharmazeutisch verträglichen Salzen und Estern, bevorzugt Ibuprofen,
- wobei eine zweite Phase, insbesondere zweite Schicht, mindestens ein Lokalanästhetikum aufweist, wobei das Lokalanästhetikum ausgewählt wird aus der Gruppe von Lidocain sowie dessen pharmazeutisch verträglichen Salzen und Estern, bevorzugt Lidocainhydrochlorid,

wobei die Herstellung der Darreichungsform mit den Maßgaben erfolgt,
dass die erste Phase, insbesondere die erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet wird, und dass die zweite Phase, insbesondere die zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet wird; insbesondere wobei die Herstellung der Darreichungsform zudem mit der weiteren Maßgabe erfolgt, dass mindestens eine(s) der nachfolgenden Merkmale und/oder Maßnahmen (i), (ii), (iii) durchgeführt wird, insbesondere mindestens Merkmal und/oder Maßnahme (i), vorzugsweise Merkmale und/oder Maßnahmen (i) und (ii), bevorzugt Merkmale und/oder Maßnahmen (i), (ii) und (iii):
   (i) Die erste Phase, insbesondere die erste Schicht, wird mit mindestens einem Spreng- und/oder Zerfallsmittel ausgerüstet und die zweite Phase, insbesondere zweite Schicht, weist kein Spreng- und/oder Zerfallsmittel auf oder aber
      die erste Phase, insbesondere die erste Schicht, und auch die zweite Phase, insbesondere die zweite Schicht, werden jeweils mit mindestens einem Spreng- und/oder Zerfallsmittel ausgerüstet, jedoch mit der Maßgabe, dass in der zweiten Phase, insbesondere zweiten Schicht, eine geringere Menge an Spreng- und/oder Zerfallsmittel als in der ersten Phase, insbesondere ersten Schicht, eingesetzt wird, insbesondere wie zuvor definiert;
   (ii) die erste Phase, insbesondere die erste Schicht, und die zweite Phase, insbesondere die zweite Schicht werden, insbesondere unabhängig voneinander und/oder getrennt voneinander und/oder aufeinander abfolgend, jeweils einzeln durch Verpressen hergestellt, vorzugsweise durch Verpressen der die jeweilige Phase, insbesondere Schicht, ausbildenden Wirk- und Inhaltsstoffe, wobei die zweite Phase, insbesondere die zweite Schicht, mit einer größeren Presskraft ([N]) und/oder mit einem größeren Pressdruck ([N/mm²]) als die erste Phase, insbesondere erste Schicht, hergestellt und/oder verpresst wird, insbesondere wie zuvor definiert;
   (iii) der NSAR-Wirkstoff, bevorzugt Ibuprofen, wird in Form eines Granulats eingesetzt, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist.

Hinsichtlich zuvor angeführten Merkmale bzw. Maßnahmen (i), (ii) bzw. (iii) wird insgesamt gewährleistet, dass mit dem erfindungsgemäßen Verfahren hergestellte Darreichungsform nach der Erfindung in Bezug auf die jeweiligen Phasen bzw. Schichten differenzierte bzw. voneinander verschiedene Zerfalls- und Wirkstofffreisetzungseigenschaften aufweist, wie zuvor angeführt, wobei durch die jeweiligen Maßnahmen nicht zuletzt auch in ihrer Kombination die entsprechenden Eigenschaften weiterführend eingestellt bzw. maßgeschneidert werden können.

Im Hinblick auf Merkmal bzw. Maßnahme (i) kann, insbesondere derart vorgegangen werden, dass die erste Phase, insbesondere erste Schicht, das Spreng- und/oder Zerfallsmittel, insbesondere Polyvinylpolypyrrolidon (Crospovidon), in einer (relativen) Menge im Bereich von 3 Gew.-% bis 90 Gew.-%, insbesondere im Bereich von 5 Gew.-% bis 70 Gew.-%, vorzugsweise im Bereich von 10 Gew.-% bis 50 Gew.-%, bevorzugt im Bereich von 15 Gew.-% bis 25 Gew.-%, bezogen auf die erste Phase (Trockengewicht), insbesondere auf die erste Schicht (Trockengewicht), aufweist.

Zudem kann es für Merkmal bzw. Maßnahme (i) vorgesehen sein, dass für den Fall, wonach die erste Phase, insbesondere die erste Schicht, und auch die zweite Phase, insbesondere die zweite Schicht, jeweils mit dem Spreng- und/oder Zerfallsmittel ausgerüstet werden, die Menge an Spreng- und/oder Zerfallsmittel in der zweiten Phase, insbesondere zweiten Schicht, höchstens 60 %, insbesondere höchstens 40 %, vorzugsweise höchstens 20 %, bevorzugt höchstens 10 %, der absoluten Menge an Spreng- und/oder Zerfallsmittel in der ersten Phase, insbesondere ersten Schicht, beträgt bzw. auf die vorgenannten Werte eingestellt wird.

Zudem kann es für Merkmal und/oder Maßnahme (ii) vorgesehen sein, dass derart verfahren wird, dass das Verhältnis der Presskraft ([N]) der zweiten Phase, insbesondere der zweiten Schicht, zur Presskraft ([N]) der ersten Phase, insbesondere der ersten Schicht, im Bereich von 1,05 : 1 bis 20 : 1, insbesondere im Bereich von 1,1 : 1 bis 10: 1, vorzugsweise im Bereich von 1,2 : 1 bis 6 : 1, bevorzugt im Bereich von 1,5: 1 bis 4 : 1, liegt und/oder auf die vorgenannten Werte eingestellt wird.

In diesem Zusammenhang kann es auch vorgesehen sein, dass das Verhältnis des Pressdrucks ([N/mm²]) der zweiten Phase, insbesondere der zweiten Schicht, zum Pressdruck ([N/mm²]) der ersten Phase, insbesondere der ersten Schicht, im Bereich von 1,05 : 1 bis 20 : 1, insbesondere im Bereich von 1,1 : 1 bis 10 : 1, vorzugsweise im Bereich von 1,2 : 1 bis 6 : 1, bevorzugt im Bereich von 1,5 : 1 bis 4 : 1, liegt und/oder auf die vorgenannten Werte eingestellt wird.

Erfindungsgemäß kann zudem in Bezug auf Merkmal bzw. Maßnahme (ii) derart vorgegangen werden, dass zunächst die zweite Phase, insbesondere die zweite Schicht, hergestellt bzw. verpresst wird und dass nachfolgend die erste Phase, insbesondere erste Schicht, hergestellt bzw. verpresst wird. Diesbezüglich kann insbesondere derart verfahren werden, dass die erste Phase, insbesondere die erste Schicht, auf der (zuvor hergestellten bzw. verpressten) zweiten Phase, insbesondere zweiten Schicht, vorzugsweise auf einer der Grundflächen der zweiten Phase, insbesondere der zweiten Schicht, hergestellt bzw. verpresst wird.

Somit kann zuerst die zweite Phase, insbesondere die zweite Schicht, durch Verpressen, insbesondere durch Verpressen der die zweite Phase, insbesondere die zweite Schicht, ausbildenden Wirk- und/oder Inhaltsstoffe, hergestellt werden und nachfolgend bzw. hierauf abfolgend die erste Phase, insbesondere die erste Schicht, durch Verpressen, insbesondere durch Verpressen der die ersten Phase, insbesondere erste Schicht, ausbildenden Wirk- bzw. Inhaltsstoffe, hergestellt werden. So kann beispielsweise in einer hierfür eingesetzten Pressvorrichtung, insbesondere Presskammer, zunächst die Herstellung der zweiten Phase bzw. zweiten Schicht mit der größeren Presskraft bzw. mit dem größeren Pressdruck erfolgen. Anschließend kann, insbesondere in derselben Pressvorrichtung, insbesondere Presskammer, auf der so hergestellten zweiten Phase, insbesondere zweiten Schicht, die erste Phase, insbesondere erste Schicht, durch Verpressen hergestellt werden, und zwar insbesondere mit kleinerer Presskraft bzw. kleinerem Pressdruck im Vergleich zu der zuvor hergestellten zweiten Phase bzw. zweiten Schicht.

Weiterführend kann es im Hinblick auf Merkmal bzw. Maßnahme (ii) zudem vorgesehen sein, dass die Darreichungsform, insbesondere die Tablette, durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten wird. Durch das (End-)Verpressen der zuvor hergestellten Phasen bzw. Schichten wird ein besonders fester und somit mechanisch stabiler Verbund zwischen den jeweiligen Phasen bzw. Schichten gewährleistet.

In Bezug auf Merkmal bzw. Maßnahme (iii) hat es sich zudem als vorteilhaft erwiesen, wenn das Granulat mit einer mittleren Teilchen- und/oder Partikelgröße D₅₀ im Bereich von 5 µm bis 500 µm, insbesondere im Bereich von 10 µm bis 200 µm, vorzugsweise im Bereich von 50 µm bis 100 µm, bevorzugt im Bereich von 60 µm bis 85 µm, eingesetzt wird, insbesondere bestimmt mittels Laserdiffraktometrie, insbesondere gemäß Ph. Eur., 10. Ausgabe, Methode 2.9.31.

Auf Basis der Verwendung eines Granulats mit definierter Teilchen- bzw. Partikelgröße, wie zuvor definiert, wird auch der Verpressvorgang sowie die Eigenschaften des resultierenden Presslings optimiert. Insbesondere führt die Verwendung entsprechender Granulate zu weiter verbesserten Eigenschaften hinsichtlich des Zerfalls bzw. der Wirkstofffreisetzung, und zwar auch im Hinblick auf die bei Applikation vorliegende (systemische) Aufnahme des Wirkstoffs bzw. der zugrundeliegenden Granulate. Auch wird die Stabilität, insbesondere Lagerstabilität der Darreichungsform verbessert.

Für weitergehende Einzelheiten zu diesem Erfindungsaspekt kann Bezug genommen werden auf die Ausführungen zu den weiteren Erfindungsaspekten, wobei diese Ausführungen gleichermaßen für den vorliegenden Erfindungsaspekt entsprechend gelten.

Die vorliegende Erfindung sowohl gemäß dem ersten Aspekt der vorliegenden Erfindung als auch gemäß sämtlichen übrigen Aspekten der vorliegenden Erfindung ist insgesamt mit einer Vielzahl von Vorteilen und Besonderheiten verbunden, welche das erfindungsgemäße Anwendungs- bzw. Therapiekonzept einzigartig und besonders hocheffizient machen.

Im Folgenden wird die vorliegende Erfindung anhand von bevorzugten Ausführungsbeispielen bzw. Ausführungsformen darstellenden Zeichnungen bzw. Figurendarstellungen näher erläutert. Im Zusammenhang mit der Erläuterung dieser bevorzugten Ausführungsbeispiele bzw. Ausführungsformen der vorliegenden Erfindung, welche jedoch in Bezug auf die vorliegende Erfindung keinesfalls beschränkend sind, werden auch weitergehende Vorteile, Eigenschaften, Aspekte und Merkmale der vorliegenden Erfindung aufgezeigt.

In den Figurendarstellungen zeigt:

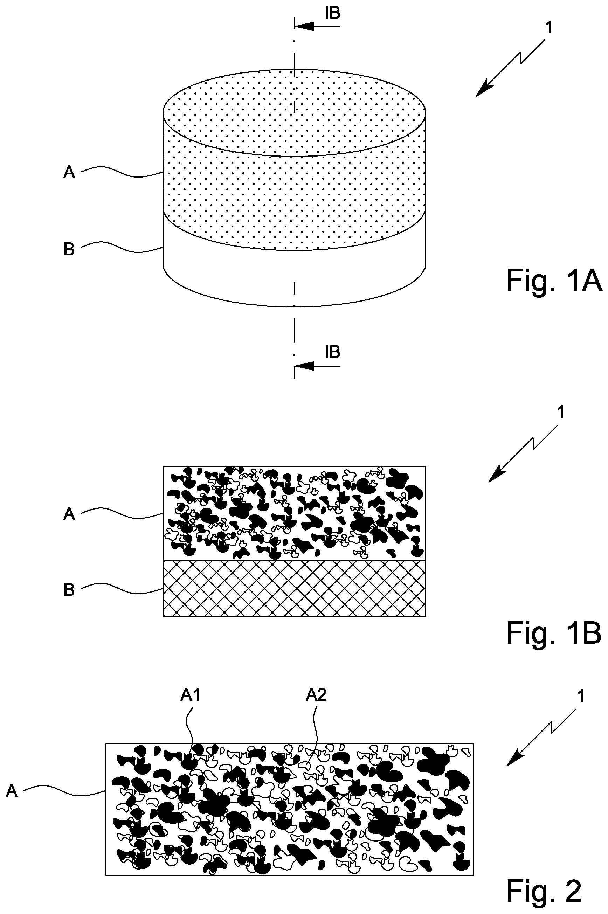

- Fig. 1A: eine schematische Darstellung mit einer perspektivischen Ansicht auf die erfindungsgemäße Darreichungsform 1 in Form einer Zweischicht-Tablette, wobei die Darreichungsform eine erste Phase (Phase A) (welche den NSAR-Wirkstoff enthält), insbesondere erste Schicht (Schicht A), sowie eine zweite Phase (Phase B) (welche das Lokalanästhetikum enthält), insbesondere zweite Schicht (Schicht B), aufweist;
- Fig. 1B: eine schematische Querschnittsdarstellung der erfindungsgemäßen Darreichungsform 1 mit der ersten Phase (Phase A), insbesondere ersten Schicht (Schicht A), und der zweiten Phase (Phase B), insbesondere zweiten Schicht (Schicht B);
- Fig. 2: eine schematische Darstellung in Form eines Querschnitts der ersten Phase (Phase A), insbesondere ersten Schicht (Schicht A), der erfindungsgemäßen Darreichungsform 1, wobei die Schicht einen insbesondere granulatförmigen und gegebenenfalls beschichteten NSAR-Wirkstoff, bevorzugt Ibuprofen, sowie weiterführend ein Spreng- bzw. Zerfallsmittel A2 aufweist;

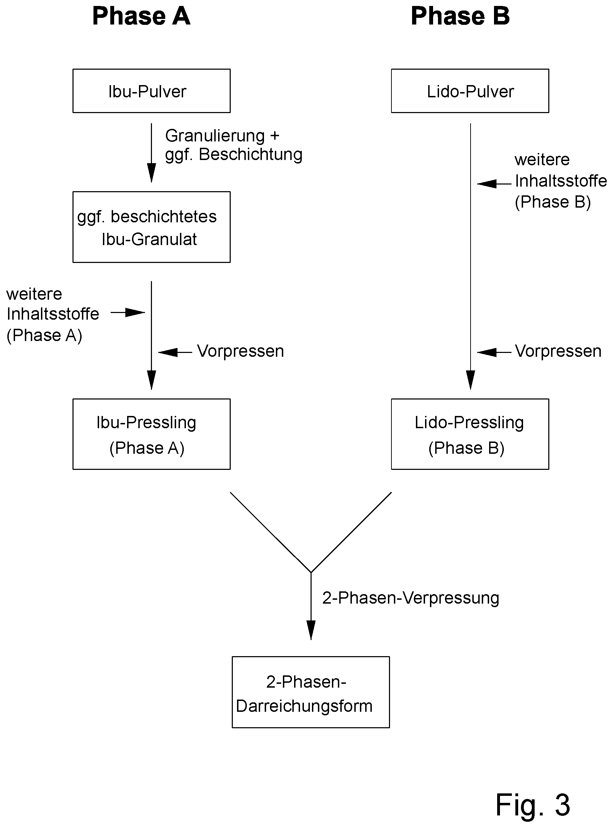

- Fig. 3: ein Ablaufdiagramm mit einer beispielhaften Durchführung des erfindungsgemäßen Verfahrens zur Herstellung der erfindungsgemäßen Darreichungsform insbesondere in Form einer Zweischicht-Tablette.

Insgesamt verdeutlichen die Figurendarstellungen gemäß Fig. 1A, Fig. 1B und Fig. 2 die erfindungsgemäße Konzeption mit der Bereitstellung einer speziellen Darreichungsform 1, insbesondere pharmazeutischen Darreichungsform, insbesondere für die orale Applikation, vorzugsweise zur Verwendung bei der prophylaktischen bzw. therapeutischen Behandlung von insbesondere schmerzbegleiteten entzündlichen Erkrankungen des Mund/Rachen-Raums, wobei die Darreichungsform 1 als zweiphasige Tablette bzw. Zweischicht-Tablette ausgebildet ist,
wobei die zweiphasige Tablette bzw. Zweischicht-Tablette mindestens zwei voneinander verschiedene Phasen A, B, insbesondere mindestens zwei voneinander verschiedene Schichten A, B, mit bei oraler Applikation voneinander verschiedenen Zerfallszeiten bzw. Wirkstofffreisetzungseigenschaften aufweist oder hieraus besteht,
- wobei eine Phase A, insbesondere Schicht A, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die Phase A, insbesondere die Schicht A, mindestens einen NSAR-Wirkstoff A1 aufweist, wobei der NSAR-Wirkstoff A1 ausgewählt ist aus der Gruppe von Ibuprofen sowie dessen pharmazeutisch verträglichen Salzen und Estern, bevorzugt Ibuprofen,
- wobei eine Phase B, insbesondere Schicht B, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die Phase B, insbesondere die Schicht B, mindestens ein Lokalanästhetikum aufweist, wobei das Lokalanästhetikum ausgewählt ist aus der Gruppe von Lidocain sowie dessen pharmazeutisch verträglichen Salzen und Estern, bevorzugt Lidocainhydrochlorid.

Zudem verdeutlicht die Figurendarstellung gemäß Fig. 2, die erfindungsgemäß bevorzugte Ausführungsform, wonach die erste Phase bzw. erste Schicht (Phase A) mindestens ein Spreng- bzw. Zerfallsmittel A2 aufweisen kann.

Fig. 3 veranschaulicht das erfindungsgemäße Herstellungsverfahren gemäß einer Ausführungsform der vorliegenden Erfindung:
So können die erste Phase (Phase A) und die zweite Phase (Phase B) jeweils durch (Vor-)Verpressen der die jeweiligen Phasen ausbildenden Wirk- und Inhaltsstoffe hergestellt werden.

Für die Phase A bzw. Schicht A kann beispielsweise derart vorgegangen werden, dass zunächst der NSAR-Wirkstoff in Form von Ibuprofen-Pulver (Ibu-Pulver) granuliert und das erhaltene Ibuprofen-Granulat gegebenenfalls nachfolgend mit einer Beschichtung ausgerüstet wird, so dass ein gegebenenfalls beschichtetes Ibuprofen-Granulat (Ibu-Granulat) resultiert. Das Granulat kann anschließend mit den weiteren Inhaltsstoffen, wie dem Spreng- bzw. Zerfallsmittel zusammengeführt und insbesondere innig bzw. homogen vermischt werden. Die Wirk- und Inhaltsstoffe können anschließend zum Erhalt eines Ibuprofen-Presslings (Ibu-Pressling, Phase A) (vor-)gepresst werden.

Für die Phase B bzw. Schicht B kann zudem derart vorgegangen werden, dass das Lokalanästhetikum, insbesondere Lidocainhydrochlorid, in Form eines Pulvers (Lido-Pulver) eingesetzt wird, welches mit den weiteren die Phase B ausbildenden Inhaltsstoffen innig bzw. homogen vermischt so und anschließend (vor-)verpresst werden, so dass ein Lidocainhydrochlorid-Pressling (Lido-Pressling, Phase B) erhalten wird.

Insbesondere kann zunächst die zweite Phase bzw. Schicht (Phase B) und nachfolgend hierzu die erste Phase bzw. Schicht (Phase A) hergestellt werden, wobei die erste Phase bzw. Schicht (Phase A) auf der zuvor hergestellten zweiten Phase bzw. Schicht (Phase B) verpresst bzw. hergestellt werden kann.

Weiterhin können die jeweiligen Presslinge (Phase A und Phase B) zum Erhalt der erfindungsgemäßen Darreichungsform aufeinander verpresst werden, insbesondere mittels einer Zweiphasen- bzw. Zweischicht-Verpressung (2-Phasen-Verpressung), so dass auf dieser Basis die erfindungsgemäße Darreichungsform, insbesondere Zweiphasen-Tablette (2-Phasen-Darreichungsform) erhalten wird.

Erfindungsgemäß kann es in diesem Zusammenhang und gleichermaßen unter Bezugnahme auf Fig. 3 insbesondere vorgesehen sein, dass die Phase A und die Phase B, insbesondere unabhängig voneinander bzw. getrennt voneinander bzw. aufeinander abfolgend, zunächst jeweils einzeln durch (Vor-) Verpressen der jeweiligen Wirk- und Inhaltsstoffe hergestellt werden. Insbesondere kann dabei derart vorgegangen werden, dass zuerst die Phase B hergestellt bzw. (vor-) verpresst wird, und zwar auf Basis der die Phase B ausbildenden Wirk- und Inhaltsstoffe, und dass nachfolgend die Phase A hergestellt bzw. (vor-)verpresst wird, und zwar auf Basis der die Phase A ausbildenden Wirk- und Inhaltsstoffe. Insbesondere kann dabei in einer gemeinsamen bzw. in derselben Pressvorrichtung, insbesondere Presskammer, in welcher zuvor die Phase B hergestellt worden ist, die Phase A auf der Phase B hergestellt bzw. (vor-)verpresst werden, gefolgt von einer (End-)Verpressung (2-Schichten-Verpressung) der zuvor hergestellten Phase B und der darauf hergestellten Phase A zum Erhalt der erfindungsgemäßen Darreichungsform in Form einer Zweiphasen-Darreichungsform bzw. Zweischicht-Darreichungsform (Zweischicht-Tablette). Im Allgemeinen wird die Phase B dabei mit einer geringeren Presskraft als die Phase A verpresst.

Für weitergehende Einzelheiten zu den in den Figurendarstellungen dargestellten Ausführungsformen und Ausführungsbeispielen kann zur Vermeidung unnötiger Wiederholungen auch auf die obigen Ausführungen sowie auf die nachfolgenden ergänzenden Ausführungen zu den Ausführungsbeispielen verwiesen werden, welche in Bezug auf die Figurendarstellungen entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellung von erfindungsgemäßen Darreichungsformen in Form von Zweischicht-Tabletten

### TEIL A

Vorliegend werden erfindungsgemäße Darreichungsformen in Form von Zweischicht-Tabletten auf Basis der nachfolgend in Tabelle 1 angegebenen Grundrezeptur hergestellt.

Dabei wird derart vorgegangen, dass zunächst die Lokalanästhetikum-Phase (nachfolgend auch als zweite Phase bzw. Schicht = Phase B bezeichnet) hergestellt wird. Hierzu wird ein Lidocainhydrochlorid-Pulver eingesetzt und mit den weiteren Inhaltsstoffen der Phase B, wie in Tabelle 1 angegeben, innig bzw. homogen vermischt. Die Mischung wird in eine diesbezügliche Tabletten-Presskammer (Matrize) eingebracht, wobei nachfolgend unter Einwirkung einer Presskraft (Kompressionskraft) von etwa 6 Newton (N) (Vorpressung zum Erhalt von Phase B) die zweite Phase bzw. Schicht in Form eines Presslings erhalten wird.

Anschließend erfolgt die Herstellung der NSAR-Wirkstoff-Phase und somit der ersten Phase bzw. der ersten Schicht (= Phase A). Hierzu wird das Ibuprofen in Form eines Granulats (mittlerer Teilchendurchmesser D₅₀ = 70µm) verwendet, wobei das Granulat eine HPMC-Beschichtung (Beschichtung auf Basis von Hydroxypropylmethylcellulose) aufweist. Das Granulat wird mit den weiteren Inhaltsstoffen, welche für die Ausbildung von der ersten Phase bzw. Phase A eingesetzt werden, innig bzw. homogen vermischt. Die resultierende Mischung wird anschließend in dieselbe Presskammer auf den dort bereits befindlichen Pressling der Phase B eingeführt und anschließend mit einer Presskraft (Kompressionskraft) von etwa 2 N zur Ausbildung von Phase A in Form eines Presslings verpresst bzw. komprimiert (Vorpressen zum Erhalt von Phase A).

Anschließend erfolgt eine (End-)Verpressung der aufeinander angeordneten Presslinge der Phasen A und B zur Ausbildung der erfindungsgemäßen Darreichungsformen in Form der Zweischicht-Tablette. Hierzu wird eine Presskraft (Kompressionskraft) von etwa 25 N einwirken gelassen.

Anschließend erfolgt ein Auswurf der erhaltenen Darreichungsformen in Form der Zweischicht-Tablette aus der Presskammer bzw. Matrize.

Die so erhaltenen erfindungsgemäßen Darreichungsformen weisen eine ebenfalls zylinderförmige Formgebung auf, wobei die jeweils zylinderförmig ausgebildeten Phasen A und B an ihren einander gegenüberliegenden Grundflächen miteinander verbunden sind. Die erste Phase bzw. Schicht (Phase A) weist dabei eine Dicke bzw. Zylinderhöhe von etwa 3,8 mm auf, und die zweite Phase bzw. Schicht (Phase B) weist eine Höhe von etwa 2,5 mm auf. Folglich weisen die resultierenden Zweischicht-Tabletten eine Gesamtdicke bzw. Gesamtzylinderhöhe von etwa 6,3 mm auf. Die Zweischicht-Tabletten weisen dabei einen Durchmesser von etwa 19 mm auf. Die insgesamt resultierenden Zweischicht-Tabletten weisen einen stabilen Verbund der jeweiligen Phasen auf und zeichnen sich zudem durch eine hohe Abriebfestigkeit und Staubfreiheit sowie durch eine hohe Stabilität, insbesondere Lagerstabilität, aus.

Alle erfindungsgemäß hergestellten Darreichungsformen in Form von Zweischicht-Tabletten werden dabei auf eine Restfeuchte, bezogen auf die Darreichungsform, von etwa 1,5 Gew.-% bis 2,5 Gew.-% eingestellt. Dies gewährleistet eine besonders gute Stabilität, insbesondere Lagerstabilität, der resultierenden Darreichungsformen und darüber hinaus eine gute Verarbeitbarkeit der Wirk- und Inhaltsstoffe der jeweiligen Phasen A und B beim Verarbeiten, insbesondere Verpressen, sowie einen besonders stabilen Verbund der beiden Phasen A und B untereinander sowie der Wirk- und Inhaltsstoffe innerhalb der einzelnen Phasen.

Wie nachfolgend noch im Detail geschildert, weisen die vorliegend in Teil A beschriebenen Zweischicht-Tabletten definierte und in Bezug auf die jeweiligen Phasen verschiedene bzw. gezielt eingestellte Zerfalls- und Wirkstofffreisetzungseigenschaften auf.

In der nachfolgenden Tabelle 1 ist eine Grundrezeptur für die erfindungsgemäßen Darreichungsformen wiedergegeben, wobei alle nachfolgenden Mengen- und Gewichtsangaben auf das Trockengewicht der Darreichungsformen bezogen sind. Innerhalb der nachfolgend angegebenen Bereichsgrenzen lassen sich ausgezeichnete Darreichungsformen nach der vorliegenden Erfindung erhalten. Die relativen Mengen werden derart ausgewählt, dass stets 100 % (Gew.-%) resultieren.

**Tabelle 1**

| | | | **Zusammensetzung** | **Menge in hergestellter Tablette** |
|---|---|---|---|---|
| | **Wirk- bzw. Inhaltsstoffe** | | **[Gew.-%]** | **[mg]** |
| **erste Phase bzw. Schicht (Phase A) (Ibuprofen-Phase; orodispersible Phase)** | | | | |
| Wirkstoff | HPMC-beschichtetes Ibuprofen-Granulat (D₅₀ = 70 µm) | | 25 - 35 | 190 - 210 * |
| weitere Inhaltsstoffe | Füll- und/oder Trägerstoffe (zerfallsunterstützend) | | 30 - 45 | |
| | Spreng- und/oder Zerfallsmittel | | 15-25 | |
| | Geschmacksmodifizierer/-maskierer, Geschmacks-/Aromastoffe, Süßstoffe, Süßungsmittel | | 1,2 - 40 | |
| | Säuerungsmittel (Citronensäure) | | 0,5 - 2,5 | |
| | Fließmittel und/oder Schmiermittel | | 1 - 20 | |
| **gesamt Phase A** | | | **ad 100,00** | **ad 800,00** |

| **zweite Phase bzw. Schicht (Phase B) (Lidocainhydrochlorid-Phase; orodispersible Phase)** | | | | |
|---|---|---|---|---|
| Wirkstoff | Lidocainhydrochlorid (Lidocain-HCl) | | 0,5 - 1,5 | 6 - 9 ** |
| weitere Inhaltsstoffe | Füll- und/oder Trägerstoffe (nichtzerfallsunterstützend) | | 30 - 85 | |
| | Geschmacksmodifizierer/-maskierer, Geschmacks-/Aromastoffe, Süßstoffe, Süßungsmittel | | 1,2 - 40 | |
| | Säuerungsmittel (Citronensäure) | | 0,5 - 2,5 | |
| | Fließmittel und/oder Schmiermittel) | | 1 - 20 | |
| **gesamt Phase B** | | | **ad 100,00** | **ad 700,00** |
| **gesamt Darreichungsform / Tablette** | | | | **1.500,00** |

| | | | | |
|---|---|---|---|---|
| * berechnet als Ibuprofen ** berechnet als Lidocain | | | | |

### TEIL B

In entsprechender Weise zu den Ausführungen in Teil A wird eine erfindungsgemäße Darreichungsform in Form von Zweischicht-Tabletten auf Basis der konkreten Rezeptur gemäß einer bevorzugten Ausführungsform der Erfindung, wie sie in Tabelle 2 angeführt ist, hergestellt. Auch diesbezüglich resultieren stabile Tabletten mit hoher Abriebfestigkeit und Staubfreiheit sowie ausgezeichneten Stabilitäten, insbesondere Lagerstabilitäten.

Wie nachfolgend noch im Detail geschildert, weisen die vorliegend in Teil B beschriebenen Zweischicht-Tabletten definierte und in Bezug auf die jeweiligen Phasen verschiedene bzw. gezielt eingestellte Zerfalls- und Wirkstofffreisetzungseigenschaften auf.

In der nachfolgenden Tabelle 2 ist eine konkrete Rezeptur gemäß einer bevorzugten Ausführungsform der Erfindung für die erfindungsgemäßen Darreichungsformen wiedergegeben, wobei alle nachfolgenden Mengen- und Gewichtsangaben auf das Trockengewicht der Darreichungsformen bezogen sind.

**Tabelle 2**

| | | | **Zusammensetzung** | **Menge in hergestellter Tablette** |
|---|---|---|---|---|
| | **Wirk- bzw. Inhaltsstoffe** | | **[Gew.-%]** | **[mg]** |
| **erste Phase bzw. Schicht (Phase A) (Ibuprofen-Phase; orodispersible Phase)** | | | | |
| Wirkstoff | HPMC-beschichtetes Ibuprofen-Granulat (D₅₀ = 70 µm) | | 30 | ca. 200 * |
| weitere Inhaltsstoffe | Füll- und/oder Trägerstoffe (zerfallsunterstützend) | | 44 | |
| | Spreng- und/oder Zerfallsmittel | | 20 | |
| | Geschmacksmodifizierer/-maskierer, Geschmacks-/Aromastoffe, Süßstoffe, Süßungsmittel | | 3 | |
| | Säuerungsmittel (Citronensäure) | | 1 | |
| | Fließmittel und/oder Schmiermittel | | 2 | |
| **gesamt Phase A** | | | **ad 100,00** | **ad 800,00** |

| **zweite Phase bzw. Schicht (Phase B) (Lidocainhydrochlorid-Phase; orodispersible Phase)** | | | | |
|---|---|---|---|---|
| Wirkstoff | Lidocainhydrochlorid (Lidocain-HCl) | | 1,14 | ca. 8 ** |
| weitere Inhaltsstoffe | Füll- und/oder Trägerstoffe (nichtzerfallsunterstützend) | | 83,06 | |
| | Geschmacksmodifizierer/-maskierer, Geschmacks-/Aromastoffe, Süßstoffe, Süßungsmittel | | 12,55 | |
| | Säuerungsmittel (Citronensäure) | | 1,25 | |
| | Fließmittel und/oder Schmiermittel) | | 2 | |
| **gesamt Phase B** | | | **ad 100,00** | **ad 700,00** |
| **gesamt Darreichungsform / Tablette** | | | | **1.500,00** |

| | | | | |
|---|---|---|---|---|
| * berechnet als Ibuprofen ** berechnet als Lidocain | | | | |

### 2. Untersuchungen bezüglich der Zerfalls- und Wirkstofffreisetzunaseigenschaften der erfindungsgemäßen Darreichungsformen in Form von Zweischicht-Tabletten

An den wie zuvor beschrieben hergestellten erfindungsgemäßen Darreichungsformen in Form von Zweischicht-Tabletten werden die folgenden Untersuchungen bezüglich der Zerfallseigenschaften durchgeführt.

Zunächst werden die Zerfallszeiten der jeweiligen Phasen der erfindungsgemäßen Zweischicht-Tabletten gemäß Ph. Eur., 10. Ausgabe, Methode 2.9.1, bestimmt. Bei sämtlichen wie zuvor beschrieben hergestellten Zweischicht-Tabletten weist die erste Phase bzw. Schicht (Phase A = orodispersible Phase bzw. Schicht) mit schnellem und/oder unmittelbarem (sofortigem) Zerfall und/oder Wirkstofffreisetzung jeweils Zerfallszeiten im Bereich von 10 s bis 30 s auf; die zweite Phase bzw. Schicht (Phase B = oral-transmukosale Phase bzw. Schicht) weist bei sämtlichen wie zuvor beschrieben hergestellten Zweischicht-Tabletten jeweils Zerfallszeiten im Bereich von 8 min bis 12 min auf.

Des Weiteren werden an den wie zuvor beschrieben hergestellten erfindungsgemäßen Darreichungsformen in Form von Zweischicht-Tabletten auch die folgenden Untersuchungen bezüglich der Wirkstofffreisetzungseigenschaften durchgeführt:
Zu diesem Zweck werden die Wirkstofffreisetzungsgeschwindigkeiten der jeweiligen Phasen der erfindungsgemäßen Zweischicht-Tabletten gemäß Ph. Eur., 10. Ausgabe, Methode 2.9.3.

Bei sämtlichen wie zuvor beschrieben hergestellten Zweischicht-Tabletten liegt das Verhältnis der Wirkstofffreisetzungsgeschwindigkeit der ersten Phase bzw. Schicht (Phase A = orodispersible Phase bzw. Schicht) zur Wirkstofffreisetzungsgeschwindigkeit der zweiten Phase bzw. Schicht (Phase B = oral-transmukosale Phase bzw. Schicht) im Bereich von 500 : 1 bis 2.000 : 1.

### 3. Stabilitätsuntersuchungen

An den wie zuvor beschrieben hergestellten erfindungsgemäßen Darreichungsformen in Form von Zweischicht-Tabletten (vgl. vorherige Ziff. 1, Teile A und B) werden die folgenden Stabilitätsuntersuchungen durchgeführt.

Die Stabilitätsuntersuchungen werden gemäß ICH Topic Q1A (R2), *"*Stability Testing of new Drug Substances and Products", European Medicines Agency, August 2003, unter beschleunigten Bedingungen durchgeführt. Die untersuchten Darreichungsformen in Form von Zweischicht-Tabletten, wie zuvor beschrieben, weisen dabei ausgezeichnete Stabilitätseigenschaften, insbesondere hohe Lagerstabilitäten, insbesondere in Konformität mit der vorgenannten Vorschrift, auf.

Demgegenüber weisen entsprechende Vergleichsdarreichungsformen, bei welchen jeweils eine der nachfolgenden Abwandlungen vorgenommen werden, nämlich (a) feinteiliges Ibuprofen-Pulver anstelle von Ibuprofen-Granulat eingesetzt ist oder aber (b) unbeschichtetes Ibuprofen-Granulat anstelle von beschichtetem Ibuprofen-Granulat eingesetzt ist oder aber (c) in Phasen A und B jeweils keine Citronensäure vorliegt (d. h. vollständiges Weglassen der Citronensäure), im Vergleich verminderte Stabilitäten bzw. Langzeitstabilitäten auf, auch wenn diese insgesamt noch im zufriedenstellenden Bereich liegen.

Auf Basis der vorliegenden Erfindung werden somit insgesamt im Hinblick auf die Behandlung von insbesondere schmerzbegleiteten entzündlichen Erkrankungen des Mund-/Rachenraums leistungsfähige Darreichungsformen insbesondere in Form von Zweischicht-Tabletten bereitgestellt, welche insgesamt hervorragende Anwendungs- bzw. Wirkeigenschaften aufweisen, und zwar infolge der Ausbildung der zugrundeliegenden Phasen mit speziellen Zerfalls- und Wirkstofffreisetzungseigenschaften, welche dem jeweiligen pharmakodynamischen bzw. pharmakokinetischen Eigenschaften des NSAR-Wirkstoffs einerseits und des Lokalanästhetikums andererseits in Abstimmung zueinander entsprechend Rechnung tragen.

Zusammenfassend wird erfindungsgemäß den Schmerz- und Entzündungszuständen des Mund-/Rachenraums multifaktoriell auf mehreren Ebenen begegnet und entgegengewirkt, wie zuvor ausgeführt, nämlich durch eine unmittelbare lokalsymptomatische Behandlung des Schmerzzustands durch das Lokalanästhetikum, welche durch den retardieren bzw. kontrollierten Zerfall über einen verlängerten Zeitraum bewirkt wird, daneben durch eine unmittelbare lokal-analgetische Behandlung durch das Ibuprofen am Ort der Freisetzung (Mund-/Rachenraum) und schließlich durch eine überwiegend kausal-systemische Behandlung durch das systemisch resorbierte Ibuprofen.

### Bezugszeichenliste

- 1: Darreichungsform, insbesondere pharmazeutische Darreichungsform
- A: erste Phase, insbesondere erste Schicht (orodispersible Phase bzw. Schicht)
- A1: NSAR-Wirkstoff, bevorzugt Ibuprofen
- A2: Spreng- bzw. Zerfallsmittel
- B: zweite Phase, insbesondere zweite Schicht (oral-transmukosale Phase bzw. Schicht), mit Lokalanästhetikum, bevorzugt Lidocainhydrochlorid

## Patentansprüche

1. Darreichungsform, insbesondere pharmazeutische Darreichungsform, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
- wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
- wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

2. Darreichungsform nach Anspruch 1,
wobei die Darreichungsform als Zweischicht-Tablette ausgebildet ist,
wobei die Zweischicht-Tablette zwei voneinander verschiedene Schichten mit bei oraler Applikation voneinander verschiedenen Freisetzungsgeschwindigkeiten aufweist oder hieraus besteht,
- wobei eine erste Schicht als orodispersible Schicht (Schmelztabletten-Schicht) mit schnellem und/oder unmittelbarem (sofortigem) Zerfall und/oder Wirkstofffreisetzung ausgebildet ist, wobei die erste Schicht mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
- wobei eine zweite Schicht als oral-transmukosale Schicht (Lutschtabletten-Schicht) mit langsamem und/oder retardiertem (kontrolliertem) Zerfall und/oder Wirkstofffreisetzung ausgebildet ist, wobei die zweite Schicht mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

3. Darreichungsform nach Anspruch 1 oder Anspruch 2,
wobei der NSAR-Wirkstoff Ibuprofen ist und wobei das Lokalanästhetikum Lidocainhydrochlorid ist; und/oder
wobei die erste Phase, insbesondere die erste Schicht, Ibuprofen als NSAR-Wirkstoff aufweist und wobei die zweite Phase, insbesondere die zweite Schicht, Lidocainhydrochlorid als Lokalanästhetikum aufweist; und/oder
wobei die erste Phase, insbesondere die erste Schicht, außer Ibuprofen keinen weiteren NSAR-Wirkstoff aufweist und wobei die zweite Phase, insbesondere die zweite Schicht, außer Lidocainhydrochlorid kein weiteres Lokalanästhetikum aufweist; und/oder
wobei der NSAR-Wirkstoff Ibuprofenlysinat ist.

4. Darreichungsform nach einem der vorangehenden Ansprüche,
wobei die Darreichungsform, insbesondere die erste Phase, bevorzugt die erste Schicht, den NSAR-Wirkstoff in einer (absoluten) Menge im Bereich von 50 mg bis 800 mg, insbesondere im Bereich von 100 mg bis 400 mg, vorzugsweise im Bereich von 150 mg bis 300 mg, bevorzugt im Bereich von 190 mg bis 210 mg, berechnet als Ibuprofen, aufweist; und/oder
wobei die Darreichungsform, insbesondere die zweite Phase, bevorzugt die zweite Schicht, das Lokalanästhetikum in einer (absoluten) Menge im Bereich von 2 mg bis 50 mg, insbesondere im Bereich von 4 mg bis 20 mg, vorzugsweise im Bereich von 5 mg bis 10 mg, bevorzugt im Bereich von 6 mg bis 9 mg, berechnet als Lidocain, aufweist.

5. Darreichungsform nach einem der vorangehenden Ansprüche,
wobei der NSAR-Wirkstoff zumindest im Wesentlichen nur in der ersten Phase, insbesondere ersten Schicht, vorliegt; und/oder
wobei die zweite Phase, insbesondere die zweite Schicht, zumindest im Wesentlichen keinen NSAR-Wirkstoff aufweist und/oder zumindest im Wesentlichen frei von dem NSAR-Wirkstoff ist; und/oder
wobei das Lokalanästhetikum zumindest im Wesentlichen nur in der zweiten Phase, insbesondere zweiten Schicht, vorliegt; und/oder
wobei die erste Phase, insbesondere die erste Schicht, zumindest im Wesentlichen kein Lokalanästhetikum aufweist und/oder zumindest im Wesentlichen frei von dem Lokalanästhetikum ist.

6. Darreichungsform nach einem der vorangehenden Ansprüche,
wobei die erste Phase, bevorzugt die erste Schicht, den NSAR-Wirkstoff in Form eines Granulats, insbesondere auf Basis vorzugsweise diskreter Teilchen und/oder Partikel, aufweist und/oder wobei der NSAR-Wirkstoff, bevorzugt Ibuprofen, granulatförmig ist und/oder in Form eines Granulats, insbesondere auf Basis vorzugsweise diskreter Teilchen und/oder Partikel, eingesetzt ist und/oder vorliegt;
insbesondere wobei das Granulat eine mittlere Teilchen- und/oder Partikelgröße D₅₀ im Bereich von 5 µm bis 500 µm, insbesondere im Bereich von 10 µm bis 200 µm, vorzugsweise im Bereich von 50 µm bis 100 µm, bevorzugt im Bereich von 60 µm bis 85 µm, aufweist, insbesondere bestimmt mittels Laserdiffraktometrie, insbesondere gemäß Ph. Eur., 10. Ausgabe, Methode 2.9.31; und/oder
insbesondere wobei das Granulat, insbesondere die einzelnen Teilchen und/oder Partikel des Granulats, eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist und/oder wobei das Granulat in beschichteter Form eingesetzt ist und/oder vorliegt, insbesondere wobei die Beschichtung eine wasserlösliche Beschichtung, vorzugsweise cellulosebasierte Beschichtung, bevorzugt auf Basis von Hydroxypropylmethylcellulose (HPMC), ist;
insbesondere wobei das Granulat, insbesondere die einzelnen Teilchen und/oder Partikel des Granulats, zumindest im Wesentlichen vollständig beschichtet und/oder zumindest im Wesentlichen vollständig von der Beschichtung umhüllt sind.

7. Darreichungsform nach einem der vorangehenden Ansprüche,
wobei die erste Phase, insbesondere die erste Schicht, eine Zerfallszeit, insbesondere bestimmt gemäß Ph. Eur., 10. Ausgabe, Methode 2.9.1, im Bereich von 1 s bis 120 s, insbesondere im Bereich von 5 s bis 60 s, vorzugsweise im Bereich von 10 s bis 30 s, aufweist; und/oder
wobei die zweite Phase, insbesondere die zweite Schicht, eine Zerfallszeit, insbesondere bestimmt gemäß Ph. Eur., 10. Ausgabe, Methode 2.9.1, im Bereich von 3 min bis 30 min, insbesondere im Bereich von 5 min bis 20 min, vorzugsweise im Bereich von 8 min bis 15 min, aufweist.

8. Darreichungsform nach einem der vorangehenden Ansprüche,
wobei das Verhältnis der Zerfallszeit der zweiten Phase, insbesondere der zweiten Schicht, zur Zerfallszeit der ersten Phase, insbesondere der ersten Schicht, im Bereich von 1,5 : 1 bis 1.500 : 1, insbesondere im Bereich von 5 : 1 bis 500 : 1, vorzugsweise im Bereich von 10 : 1 bis 100 : 1, liegt, insbesondere wobei die Zerfallszeiten gemäß Ph. Eur., 10. Ausgabe, Methode 2.9.1 bestimmt sind; und/oder
wobei das Verhältnis der Wirkstofffreisetzungsgeschwindigkeit der ersten Phase, insbesondere der ersten Schicht, zur Wirkstofffreisetzungsgeschwindigkeit der zweiten Phase, insbesondere der zweiten Schicht, im Bereich von 50 : 1 bis 10.000: 1, insbesondere im Bereich von 100 : 1 bis 5.000 : 1, vorzugsweise im Bereich von 500 : 1 bis 2.000 : 1, liegt, insbesondere wobei die Wirkstofffreisetzungsgeschwindigkeiten gemäß Ph. Eur., 10. Ausgabe, Methode 2.9.3 bestimmt sind.

9. Darreichungsform nach einem der vorangehenden Ansprüche,
wobei die erste Phase, insbesondere die erste Schicht, vorzugsweise nur und/oder ausschließlich die erste Phase, insbesondere die erste Schicht, mindestens ein Spreng- und/oder Zerfallsmittel aufweist und/oder hiermit ausgerüstet ist;
insbesondere wobei das Spreng- und/oder Zerfallsmittel der ersten Phase, insbesondere der ersten Schicht, ausgewählt ist aus der Gruppe von Polyvinylpolypyrrolidon (Crospovidon); Cellulosen, insbesondere mikrokristallinen Cellulosen, Methylcellulosen, Croscarmellose-Salz, vorzugsweise Croscarmellose-Alkali und -Erdalkali, bevorzugt Croscarmellose-Natrium; Stärken, insbesondere Maisstärke, Kartoffelstärke, vorverkleisterten Stärken; Alginsäure und deren Salzen, insbesondere Alkali- und Erdalkalisalzen, vorzugsweise Calciumalginat und Natriumalginat; Carboxyalkylstärken und deren Salze, insbesondere Alkali- und Erdalkalicarboxyalkylstärken, vorzugsweise Natriumcarboxymethylstärke; Alkali- und Erdalkalihydrogencarbonat, vorzugsweise Natriumhydrogencarbonat, insbesondere in Kombination mit mindestens einer vorzugsweise organischen Säure, vorzugsweise Natriumhydrogencarbonat in Kombination mit Citronensäure und/oder Weinsäure; oberflächenaktiven Substanzen insbesondere zur Hydrophilisierung; und deren Mischungen oder Kombinationen, besonders bevorzugt Polyvinylpolypyrrolidon (Crospovidon) und/oder Croscarmellose-Natrium; und/oder
insbesondere wobei das Spreng- und/oder Zerfallsmittel der ersten Phase, insbesondere der ersten Schicht, Croscarmellose-Alkali oder -Erdalkali, insbesondere Croscarmellose-Natrium, und/oder Polyvinylpolypyrrolidon (Crospovidon), bevorzugt Polyvinylpolypyrrolidon (Crospovidon), ist; und/oder
insbesondere wobei die erste Phase, insbesondere die erste Schicht, das Spreng- und/oder Zerfallsmittel, insbesondere Polyvinylpolypyrrolidon (Crospovidon), in einer (absoluten) Menge im Bereich von 20 mg bis 750 mg, insbesondere im Bereich von 50 mg bis 350 mg, vorzugsweise im Bereich von 100 mg bis 250 mg, bevorzugt im Bereich von 140 mg bis 180 mg, aufweist; und/oder
insbesondere wobei die Darreichungsform, insbesondere die erste Phase, bevorzugt die erste Schicht, das Spreng- und/oder Zerfallsmittel, insbesondere Polyvinylpolypyrrolidon (Crospovidon), in einer (relativen) Menge im Bereich von 2 Gew.-% bis 45 Gew.-%, insbesondere im Bereich von 3 Gew.-% bis 35 Gew.-%, vorzugsweise im Bereich von 4 Gew.-% bis 25 Gew.-%, bevorzugt im Bereich von 5 Gew.-% bis 15 Gew.-%, bezogen auf die Darreichungsform (Trockengewicht), aufweist; und/oder
insbesondere wobei die erste Phase, insbesondere die erste Schicht, das Spreng- und/oder Zerfallsmittel, insbesondere Polyvinylpolypyrrolidon (Crospovidon), in einer (relativen) Menge im Bereich von 3 Gew.-% bis 90 Gew.-%, insbesondere im Bereich von 5 Gew.-% bis 70 Gew.-%, vorzugsweise im Bereich von 10 Gew.-% bis 50 Gew.-%, bevorzugt im Bereich von 15 Gew.-% bis 25 Gew.-%, bezogen auf die erste Phase (Trockengewicht), insbesondere auf die erste Schicht (Trockengewicht), aufweist; und/oder
insbesondere wobei das Spreng- und/oder Zerfallsmittel, insbesondere Polyvinylpolypyrrolidon (Crospovidon), zumindest im Wesentlichen nur in der ersten Phase, insbesondere ersten Schicht, vorliegt.

10. Darreichungsform nach einem der vorangehenden Ansprüche,
wobei die erste Phase, insbesondere die erste Schicht, neben dem NSAR-Wirkstoff, insbesondere Ibuprofen, vorzugsweise granulatförmigem Ibuprofen, (i) mindestens ein Spreng- und/oder Zerfallsmittel, insbesondere wie in Anspruch 9 definiert, (ii) mindestens einen zerfallsunterstützenden Füll- und/oder Trägerstoff, (iii) mindestens ein Säuerungsmittel, (iv) mindestens ein geschmacksmodifizerendes und/oder geschmacksmaskierendes Mittel sowie gegebenenfalls (v) mindestens ein Additiv, insbesondere mindestens ein Fließmittel und/oder mindestens ein Schmiermittel, aufweist und
wobei die zweite Phase, insbesondere die zweite Schicht, neben dem Lokalanästhetikum, insbesondere Lidocainhydrochlorid, (i) mindestens einen nichtzerfallsunterstützenden Füll- und/oder Trägerstoff, (ii) mindestens ein Säuerungsmittel, (iii) mindestens ein geschmacksmodifizerendes und/oder geschmacksmaskierendes Mittel sowie gegebenenfalls (v) mindestens ein Additiv, insbesondere mindestens ein Fließmittel und/oder mindestens ein Schmiermittel, aufweist,
insbesondere wobei die zweite Phase, insbesondere die zweite Schicht, zumindest im Wesentlichen kein wie in Anspruch 9 definiertes Spreng- und/oder Zerfallsmittel, insbesondere zumindest im Wesentlichen kein Spreng- und/oder Zerfallsmittel, aufweist und/oder zumindest im Wesentlichen frei von einem wie in Anspruch 9 definierten Spreng- und/oder Zerfallsmittel, insbesondere zumindest im Wesentlichen frei von einem Spreng- und/oder Zerfallsmittel, ist.

11. Darreichungsform nach einem der vorangehenden Ansprüche,
wobei die erste Phase, insbesondere die erste Schicht, neben dem NSAR-Wirkstoff, insbesondere Ibuprofen, vorzugsweise granulatförmigem Ibuprofen,
(i) mindestens ein Spreng- und/oder Zerfallsmittel in Form von Croscarmellose-Natrium und/oder Polyvinylpolypyrrolidon (Crospovidon), bevorzugt Polyvinylpolypyrrolidon (Crospovidon), in einer (relativen) Menge im Bereich von 3 Gew.-% bis 90 Gew.-%, bevorzugt im Bereich von 15 Gew.-% bis 25 Gew.-%, bezogen auf die erste Phase (Trockengewicht), insbesondere auf die erste Schicht (Trockengewicht),
(ii) mindestens einen zerfallsunterstützenden Füll- und/oder Trägerstoff auf Basis von Zuckeralkoholen, bevorzugt Mannitol, in einer (relativen) Menge im Bereich von 5 Gew.-% bis 90 Gew.-%, bevorzugt im Bereich von 30 Gew.-% bis 45 Gew.-%, bezogen auf die erste Phase (Trockengewicht), insbesondere auf die erste Schicht (Trockengewicht),
(iii) mindestens ein Säuerungsmittel in Form einer organischen Säure oder deren Salze und Ester, bevorzugt Citronensäure, in einer (relativen) Menge im Bereich von 0,01 Gew.-% bis 10 Gew.-%, bevorzugt im Bereich von 0,5 Gew.-% bis 2,5 Gew.-%, bezogen auf die erste Phase (Trockengewicht), insbesondere auf die erste Schicht (Trockengewicht),
(iv) mindestens ein geschmacksmodifizerendes und/oder geschmacksmaskierendes Mittel, insbesondere in Form von Erdalkali- und/oder Alkalilaurylsulfat, bevorzugt Natriumlaurylsulfat, in einer (relativen) Menge im Bereich von 0,02 Gew.-% bis 10 Gew.-%, bevorzugt im Bereich von 0,08 Gew.-% bis 2 Gew.-%, bezogen auf die erste Phase (Trockengewicht), insbesondere auf die erste Schicht (Trockengewicht),
(v) gegebenenfalls mindestens ein Additiv, insbesondere mindestens ein Fließmittel und/oder mindestens ein Schmiermittel,
aufweist und
wobei die zweite Phase, insbesondere die zweite Schicht, neben dem Lokalanästhetikum, insbesondere Lidocainhydrochlorid,
(i) mindestens einen nichtzerfallsunterstützenden Füll- und/oder Trägerstoff auf Basis von Zuckeralkoholen, bevorzugt Sorbitol und/oder Isomalt, in einer (relativen) Menge im Bereich von 10 Gew.-% bis 98 Gew.-%, bevorzugt im Bereich von 30 Gew.-% bis 85 Gew.-%, bezogen auf die zweite Phase (Trockengewicht), insbesondere auf die zweite Schicht (Trockengewicht),
(ii) mindestens ein Säuerungsmittel in Form einer organischen Säure oder deren Salze und Ester, bevorzugt Citronensäure, in einer (relativen) Menge im Bereich von 0,01 Gew.-% bis 10 Gew.-%, bevorzugt im Bereich von 0,5 Gew.-% bis 2,5 Gew.-%, bezogen auf die zweite Phase (Trockengewicht), insbesondere auf die zweite Schicht (Trockengewicht),
(iii) mindestens ein geschmacksmodifizerendes und/oder geschmacksmaskierendes Mittel, insbesondere in Form von Cyclodextrin, in einer (relativen) Menge im Bereich von 0,01 Gew.-% bis 25 Gew.-%, bevorzugt im Bereich von 0,2 Gew.-% bis 10 Gew.- %, bezogen auf die zweite Phase (Trockengewicht), insbesondere auf die zweite Schicht (Trockengewicht),
(v) gegebenenfalls mindestens ein Additiv, insbesondere mindestens ein Fließmittel und/oder mindestens ein Schmiermittel,
aufweist,
wobei die erste Phase, insbesondere die erste Schicht, den NSAR-Wirkstoff in einer (relativen) Menge im Bereich von 5 Gew.-% bis 90 Gew.-%, bevorzugt im Bereich von 20 Gew.-% bis 30 Gew.-%, berechnet als Ibuprofen und bezogen auf die erste Phase (Trockengewicht), insbesondere auf die erste Schicht (Trockengewicht), aufweist und
wobei die zweite Phase, insbesondere die zweite Schicht, das Lokalanästhetikum in einer (relativen) Menge im Bereich von 0,1 Gew.-% bis 20 Gew.-%, bevorzugt im Bereich von 0,5 Gew.-% bis 1,5 Gew.-%, berechnet als Lidocain und bezogen auf die zweite Phase (Trockengewicht), insbesondere auf die zweite Schicht (Trockengewicht), aufweist;
insbesondere wobei die zweite Phase, insbesondere die zweite Schicht, zumindest im Wesentlichen kein wie in Anspruch 9 definiertes Spreng- und/oder Zerfallsmittel, insbesondere zumindest im Wesentlichen kein Spreng- und/oder Zerfallsmittel, aufweist und/oder zumindest im Wesentlichen frei von einem wie in Anspruch 9 definierten Spreng- und/oder Zerfallsmittel, insbesondere zumindest im Wesentlichen frei von einem Spreng- und/oder Zerfallsmittel, ist.

12. Darreichungsform nach einem der vorangehenden Ansprüche,
wobei die Darreichungsform als mehrphasige, insbesondere mindestens zweiphasige, vorzugsweise zweiphasige, Tablette, besonders bevorzugt als Zweischicht-Tablette, ausgebildet ist,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
- wobei eine erste Phase, insbesondere erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet ist, wobei die erste Phase, insbesondere die erste Schicht, mindestens einen NSAR-Wirkstoff (nichtsteroidales Antirheumatikum) aufweist, wobei der NSAR-Wirkstoff ausgewählt ist aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure), wobei die erste Phase, bevorzugt die erste Schicht, den NSAR-Wirkstoff in Form eines vorzugsweise verpressten Granulats enthält, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist,
- wobei eine zweite Phase, insbesondere zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet ist, wobei die zweite Phase, insbesondere die zweite Schicht, mindestens ein Lokalanästhetikum aufweist, wobei das Lokalanästhetikum ausgewählt ist aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid.

13. Darreichungsform, insbesondere pharmazeutische Darreichungsform, nach einem der Ansprüche 1 bis 12, insbesondere für die orale Applikation, zur Verwendung bei der prophylaktischen und/oder therapeutischen Behandlung von insbesondere schmerzbegleiteten entzündlichen Erkrankungen des Mund/Rachen-Raums.

14. Verfahren zur Herstellung einer Darreichungsform, insbesondere pharmazeutischen Darreichungsform, nach einem der Ansprüche 1 bis 13, insbesondere für die orale Applikation, vorzugsweise zur Verwendung bei der prophylaktischen und/oder therapeutischen Behandlung von insbesondere schmerzbegleiteten entzündlichen Erkrankungen des Mund/Rachen-Raums,
wobei die Darreichungsform als mehrphasige, insbesondere mindestens zweiphasige, vorzugsweise zweiphasige, Tablette, besonders bevorzugt als Zweischicht-Tablette, ausgebildet wird,
wobei die mehrphasige Tablette, bevorzugt Zweischicht-Tablette, mindestens zwei voneinander verschiedene Phasen, insbesondere mindestens zwei voneinander verschiedene Schichten, mit bei oraler Applikation voneinander verschiedenen Zerfallszeiten und/oder Wirkstofffreisetzungsgeschwindigkeiten aufweist oder hieraus besteht,
- wobei eine erste Phase, insbesondere erste Schicht, einen NSAR-Wirkstoff (nichtsteroidales Antirheumatikum) aufweist, wobei der NSAR-Wirkstoff ausgewählt wird aus der Gruppe von Ibuprofen (2-(4-Isobutylphenyl)propionsäure) sowie dessen pharmazeutisch verträglichen Salzen und Estern, bevorzugt Ibuprofen (2-(4-Isobutylphenyl)propionsäure),
- wobei eine zweite Phase, insbesondere zweite Schicht, mindestens ein Lokalanästhetikum aufweist, wobei das Lokalanästhetikum ausgewählt wird aus der Gruppe von Lidocain (2-Diethylamino-N-(2,6-dimethylphenyl)acetamid) sowie dessen pharmazeutisch verträglichen Salzen und Estern, bevorzugt Lidocainhydrochlorid, wobei die Herstellung der Darreichungsform mit den Maßgaben erfolgt,
dass die erste Phase, insbesondere die erste Schicht, als orodispersible Phase (Schmelztabletten-Phase), insbesondere orodispersible Schicht (Schmelztabletten-Schicht), mit schnellem und/oder unmittelbarem (sofortigem) Zerfall und/oder Wirkstofffreisetzung ausgebildet wird, und
dass die zweite Phase, insbesondere die zweite Schicht, als oral-transmukosale Phase (Lutschtabletten-Phase), insbesondere oral-transmukosale Schicht (Lutschtabletten-Schicht), mit langsamem und/oder retardiertem (kontrolliertem) Zerfall und/oder Wirkstofffreisetzung ausgebildet wird;
insbesondere wobei die Herstellung der Darreichungsform zudem mit der weiteren Maßgabe erfolgt, dass mindestens eine(s) der nachfolgenden Merkmale und/oder Maßnahmen (i), (ii), (iii) durchgeführt wird, insbesondere mindestens Merkmal und/oder Maßnahme (i), vorzugsweise mindestens Merkmale und/oder Maßnahmen (i) und (ii), bevorzugt Merkmale und/oder Maßnahmen (i), (ii) und (iii):
(i) Die erste Phase, insbesondere die erste Schicht, wird mit mindestens einem Spreng- und/oder Zerfallsmittel ausgerüstet und die zweite Phase, insbesondere zweite Schicht, weist kein Spreng- und/oder Zerfallsmittel auf oder aber
die erste Phase, insbesondere die erste Schicht, und auch die zweite Phase, insbesondere die zweite Schicht, werden jeweils mit mindestens einem Spreng- und/oder Zerfallsmittel ausgerüstet, jedoch mit der Maßgabe, dass in der zweiten Phase, insbesondere zweiten Schicht, eine geringere Menge an Spreng- und/oder Zerfallsmittel als in der ersten Phase, insbesondere ersten Schicht, eingesetzt wird, insbesondere wie zuvor definiert;
(ii) die erste Phase, insbesondere die erste Schicht, und die zweite Phase, insbesondere die zweite Schicht werden, insbesondere unabhängig voneinander und/oder getrennt voneinander und/oder aufeinander abfolgend, jeweils einzeln durch Verpressen hergestellt, vorzugsweise durch Verpressen der die jeweilige Phase, insbesondere Schicht, ausbildenden Wirk- und Inhaltsstoffe, wobei die zweite Phase, insbesondere die zweite Schicht, mit einer größeren Presskraft ([N]) und/oder mit einem größeren Pressdruck ([N/mm²]) als die erste Phase, insbesondere erste Schicht, hergestellt und/oder verpresst wird, insbesondere wie zuvor definiert;
(iii) der NSAR-Wirkstoff, bevorzugt Ibuprofen, wird in Form eines Granulats eingesetzt, insbesondere wobei das Granulat eine Beschichtung (Coating), insbesondere wasserlösliche Beschichtung, vorzugsweise cellulosebasierte Beschichtung, bevorzugt auf Basis von Hydroxypropylmethylcellulose (HPMC), aufweist.

15. Verfahren nach Anspruch 14,
wobei die erste Phase, insbesondere die erste Schicht, das Spreng- und/oder Zerfallsmittel, insbesondere Polyvinylpolypyrrolidon (Crospovidon), in einer (relativen) Menge im Bereich von 3 Gew.-% bis 90 Gew.-%, insbesondere im Bereich von 5 Gew.-% bis 70 Gew.-%, vorzugsweise im Bereich von 10 Gew.-% bis 50 Gew.-%, bevorzugt im Bereich von 15 Gew.-% bis 25 Gew.-%, bezogen auf die erste Phase (Trockengewicht), insbesondere auf die erste Schicht (Trockengewicht), aufweist; und/oder
wobei für den Fall, dass die erste Phase, insbesondere die erste Schicht, und auch die zweite Phase, insbesondere die zweite Schicht, jeweils mit dem Spreng- und/oder Zerfallsmittel ausgerüstet werden, die Menge an Spreng- und/oder Zerfallsmittel in der zweiten Phase, insbesondere zweiten Schicht, höchstens 60 %, insbesondere höchstens 40 %, vorzugsweise höchstens 20 %, bevorzugt höchstens 10 %, der absoluten Menge an Spreng- und/oder Zerfallsmittel in der ersten Phase, insbesondere ersten Schicht, beträgt und/oder auf die vorgenannten Werte eingestellt wird; und/oder
wobei das Verhältnis der Presskraft ([N]) der zweiten Phase, insbesondere der zweiten Schicht, zur Presskraft ([N]) der ersten Phase, insbesondere der ersten Schicht, im Bereich von 1,05 : 1 bis 20 : 1, insbesondere im Bereich von 1,1 : 1 bis 10 : 1, vorzugsweise im Bereich von 1,2 : 1 bis 6 : 1, bevorzugt im Bereich von 1,5 : 1 bis 4 : 1, liegt und/oder auf die vorgenannten Werte eingestellt wird; und/oder
wobei das Verhältnis des Pressdrucks ([N/mm²]) der zweiten Phase, insbesondere der zweiten Schicht, zum Pressdruck ([N/mm²]) der ersten Phase, insbesondere der ersten Schicht, im Bereich von 1,05 : 1 bis 20 : 1, insbesondere im Bereich von 1,1 : 1 bis 10 : 1, vorzugsweise im Bereich von 1,2 : 1 bis 6 : 1, bevorzugt im Bereich von 1,5 : 1 bis 4 : 1, liegt und/oder auf die vorgenannten Werte eingestellt wird; und/oder
wobei zunächst die zweite Phase, insbesondere die zweite Schicht, hergestellt und/oder verpresst wird und wobei nachfolgend die erste Phase, insbesondere die erste Schicht, hergestellt und/oder verpresst wird, insbesondere wobei die erste Phase, insbesondere die erste Schicht, auf der zweiten Phase, insbesondere auf der zweiten Schicht, vorzugsweise auf einer der Grundflächen der zweiten Phase, insbesondere der zweiten Schicht, hergestellt und/oder verpresst wird; und/oder
wobei die Darreichungsform, insbesondere die Tablette, durch nachfolgend gemeinsames (End-)Verpressen der zuvor hergestellten und/oder verpressten ersten Phase, insbesondere ersten Schicht, und der zuvor hergestellten und/oder verpressten zweiten Phase, insbesondere zweiten Schicht, erhalten wird; und/oder
wobei das Granulat eine mittlere Teilchen- und/oder Partikelgröße D₅₀ im Bereich von 5 µm bis 500 µm, insbesondere im Bereich von 10 µm bis 200 µm, vorzugsweise im Bereich von 50 µm bis 100 µm, bevorzugt im Bereich von 60 µm bis 85 µm, aufweist, insbesondere bestimmt mittels Laserdiffraktometrie, insbesondere gemäß Ph. Eur., 10. Ausgabe, Methode 2.9.31.

## Claims

1. Dosage form, in particular pharmaceutical dosage form, especially for oral administration, preferably for use in the prophylactic and/or therapeutic treatment of, in particular, inflammatory diseases of the mouth/throat region accompanied by pain,
wherein the dosage form is designed as a multiphase, in particular at least two-phase, preferably two-phase tablet, particularly preferably as a two-layer tablet,
wherein the multiphase tablet, preferably a two-layer tablet, comprises or consists of at least two phases that are different from each other, in particular at least two layers that are different from each other, with different disintegration times and/or active ingredient release rates upon oral administration,
- wherein a first phase, in particular a first layer, is designed as an orodispersible phase (melting tablet phase), in particular an orodispersible layer (melting tablet layer), with rapid and/or immediate (instant) disintegration and/or release of active ingredient, wherein the first phase, in particular the first layer, comprises at least one NSAID active ingredient (non-steroidal anti-inflammatory drug), wherein the NSAID active ingredient is selected from the group of ibuprofen (2-(4-isobutylphenyl)propionic acid) and its pharmaceutically acceptable salts and esters, preferably ibuprofen (2-(4-isobutylphenyl)propionic acid),
- wherein a second phase, in particular a second layer, is formed as an oral transmucosal phase (lozenge phase), in particular an oral transmucosal layer (lozenge layer), with slow and/or delayed (controlled) disintegration and/or active ingredient release, wherein the second phase, in particular the second layer, comprises at least one local anesthetic, wherein the local anesthetic is selected from the group consisting of lidocaine (2-diethylamino-N-(2,6-dimethylphenyl)acetamide) and its pharmaceutically acceptable salts and esters, preferably lidocaine hydrochloride.

2. Dosage form according to claim 1,
wherein the dosage form is designed as a two-layer tablet,
wherein the two-layer tablet comprises or consists of two different layers with different release rates upon oral administration,
- wherein a first layer is designed as an orodispersible layer (melting tablet layer) with rapid and/or immediate (instant) disintegration and/or release of active ingredient, wherein the first layer comprises at least one NSAID active ingredient (non-steroidal anti-inflammatory drug), wherein the NSAID active ingredient is selected from the group consisting of ibuprofen (2-(4-isobutylphenyl)propionic acid) and its pharmaceutically acceptable salts and esters, preferably ibuprofen (2-(4-isobutylphenyl)propionic acid),
- wherein a second layer is formed as an oral transmucosal layer (lozenge layer) with slow and/or delayed (controlled) disintegration and/or active ingredient release, wherein the second layer comprises at least one local anesthetic, wherein the local anesthetic is selected from the group consisting of lidocaine (2-diethylamino-N-(2,6-dimethylphenyl)acetamide) and its pharmaceutically acceptable salts and esters, preferably lidocaine hydrochloride.

3. Dosage form according to claim 1 or claim 2,
wherein the NSAID active ingredient is ibuprofen and wherein the local anesthetic is lidocaine hydrochloride; and/or
wherein the first phase, in particular the first layer, comprises ibuprofen as the NSAID active ingredient and wherein the second phase, in particular the second layer, comprises lidocaine hydrochloride as the local anesthetic; and/or
wherein the first phase, in particular the first layer, comprises no other NSAID active ingredient apart from ibuprofen and wherein the second phase, in particular the second layer, comprises no other local anesthetic apart from lidocaine hydrochloride; and/or
wherein the NSAID active ingredient is ibuprofen lysinate.

4. Dosage form according to one of the preceding claims,
wherein the dosage form, in particular the first phase, preferably the first layer, contains the NSAID active ingredient in an (absolute) amount in the range of 50 mg to 800 mg, in particular in the range of 100 mg to 400 mg, preferably in the range of 150 mg to 300 mg, preferably in the range of 190 mg to 210 mg, calculated as ibuprofen; and/or
wherein the dosage form, in particular the second phase, preferably the second layer, comprises the local anesthetic in an (absolute) amount in the range of 2 mg to 50 mg, in particular in the range of 4 mg to 20 mg, preferably in the range of 5 mg to 10 mg, preferably in the range of 6 mg to 9 mg, calculated as lidocaine.

5. Dosage form according to one of the preceding claims,
wherein the NSAID active ingredient is present at least substantially only in the first phase, in particular the first layer; and/or
wherein the second phase, in particular the second layer, contains at least substantially no NSAID active ingredient and/or is at least substantially free of the NSAID active ingredient; and/or
wherein the local anesthetic is present at least substantially only in the second phase, in particular the second layer; and/or
wherein the first phase, in particular the first layer, at least substantially does not contain any local anesthetic and/or is at least substantially free of the local anesthetic.

6. Dosage form according to one of the preceding claims,
wherein the first phase, preferably the first layer, comprises the NSAID active ingredient in the form of granules, in particular based on preferably discrete particles and/or granules, and/or wherein the NSAID active ingredient, preferably ibuprofen, is in granular form and/or is used and/or is present in the form of granules, in particular based on preferably discrete particles and/or granules;
in particular wherein the granules have an average particle size D₅₀ in the range from 5 µm to 500 µm, in particular in the range from 10 µm to 200 µm, preferably in the range from 50 µm to 100 µm, preferred in the range from 60 µm to 85 µm, in particular determined by laser diffractometry, in particular according to Ph. Eur., 10th edition, method 2.9.31; and/or
in particular wherein the granulate, in particular the individual particles and/or particles of the granulate, comprises a coating, in particular a water-soluble coating, preferably a cellulose-based coating, preferably based on hydroxypropyl methylcellulose (HPMC), and/or wherein the granules are used and/or are present in coated form, in particular wherein the coating is a water-soluble coating, preferably a cellulose-based coating, preferably based on hydroxypropyl methylcellulose (HPMC);
in particular wherein the granules, in particular the individual particles and/or particles of the granules, are at least substantially completely coated and/or at least substantially completely enveloped by the coating.

7. Dosage form according to one of the preceding claims,
wherein the first phase, in particular the first layer, has a disintegration time, in particular determined according to Ph. Eur., 10th edition, method 2.9.1, in the range of 1 s to 120 s, in particular in the range of 5 s to 60 s, preferably in the range of 10 s to 30 s; and/or
wherein the second phase, in particular the second layer, has a disintegration time, in particular determined according to Ph. Eur., 10th edition, method 2.9.1, in the range of 3 min to 30 min, in particular in the range of 5 min to 20 min, preferably in the range of 8 min to 15 min.

8. Dosage form according to one of the preceding claims,
wherein the ratio of the disintegration time of the second phase, in particular the second layer, to the disintegration time of the first phase, in particular the first layer, is in the range of 1.5 : 1 to 1,500 : 1, in particular in the range from 5 : 1 to 500 : 1, preferably in the range from 10 : 1 to 100 : 1, in particular wherein the disintegration times are determined according to Ph. Eur., 10th edition, Method 2.9.1; and/or
wherein the ratio of the drug release rate of the first phase, in particular the first layer, to the drug release rate of the second phase, in particular the second layer, is in the range of 50 : 1 to 10,000 : 1, in particular in the range of 100 : 1 to 5,000 : 1, preferably in the range of 500 : 1 to 2,000 : 1, in particular wherein the active ingredient release rates are determined according to Ph. Eur., 10th edition, method 2.9.3.

9. Dosage form according to one of the preceding claims,
wherein the first phase, in particular the first layer, preferably only and/or exclusively the first phase, in particular the first layer, comprises and/or is equipped with at least one disintegrant and/or disintegrating agent;
in particular wherein the disintegrant and/or dispersing agent of the first phase, in particular the first layer, is selected from the group consisting of polyvinylpolypyrrolidone (crospovidone); celluloses, in particular microcrystalline celluloses, methylcelluloses, croscarmellose salt, preferably croscarmellose alkali and alkaline earth, preferably croscarmellose sodium; starches, in particular corn starch, potato starch, pregelatinized starches; Alginic acid and its salts, in particular alkali and alkaline earth salts, preferably calcium alginate and sodium alginate; carboxyalkyl starches and their salts, in particular alkali and alkaline earth carboxyalkyl starches, preferably sodium carboxymethyl starch; alkali and alkaline earth hydrogen carbonate, preferably sodium hydrogen carbonate, in particular in combination with at least one preferably organic acid, preferably sodium hydrogen carbonate in combination with citric acid and/or tartaric acid; surface-active substances, in particular for hydrophilization; and mixtures or combinations thereof, particularly preferred polyvinylpolypyrrolidone (crospovidone) and/or croscarmellose sodium; and/or
in particular, wherein the disintegrant and/or disintegrating agent of the first phase, in particular the first layer, is croscarmellose alkali or alkaline earth, in particular croscarmellose sodium, and/or polyvinylpolypyrrolidone (crospovidone), preferably polyvinylpolypyrrolidone (crospovidone); and/or
in particular wherein the first phase, in particular the first layer, contains the disintegrant and/or disintegrating agent, in particular polyvinylpolypyrrolidone (crospovidone), in an (absolute) amount in the range from 20 mg to 750 mg, in particular in the range from 50 mg to 350 mg, preferably in the range from 100 mg to 250 mg, preferably in the range from 140 mg to 180 mg; and/or
in particular, the dosage form, in particular the first phase, preferably the first layer, contains the disintegrant and/or disintegrating agent, in particular polyvinylpolypyrrolidone (crospovidone), in a (relative) amount in the range of 2 wt.% to 45 wt.%, in particular in the range of 3 wt.% to 35 wt.%, preferably in the range of 4 wt.% to 25 wt.%, preferably in the range of 5 wt.% to 15 wt.%, based on the dosage form (dry weight); and/or
in particular wherein the first phase, in particular the first layer, contains the disintegrant and/or disintegrating agent, in particular polyvinylpolypyrrolidone (crospovidone), in a (relative) amount in the range of 3 wt.% to 90 wt.%, in particular in the range of 5 wt.% to 70 wt.%, preferably in the range of 10 wt.% to 50 wt.%, preferably in the range of 15 wt.% to 25 wt.%, based on the first phase (dry weight), in particular on the first layer (dry weight); and/or
in particular, wherein the disintegrant and/or disintegrating agent, in particular polyvinylpolypyrrolidone (crospovidone), is present at least substantially only in the first phase, in particular the first layer.

10. Dosage form according to one of the preceding claims,
wherein the first phase, in particular the first layer, in addition to the NSAID active ingredient, in particular ibuprofen, preferably granulated ibuprofen, (i) at least one disintegrant and/or disintegrating agent, in particular as defined in claim 9, (ii) at least one disintegration-promoting filler and/or carrier, (iii) at least one acidifying agent, (iv) at least one flavor-modifying and/or flavor-masking agent, and, optionally, (v) at least one additive, in particular at least one flow agent and/or at least one lubricant, and
wherein the second phase, in particular the second layer, comprises, in addition to the local anesthetic, in particular lidocaine hydrochloride, (i) at least one non-disintegration-promoting filler and/or carrier, (ii) at least one acidifying agent, (iii) at least one taste-modifying and/or taste-masking agent, and, optionally, (v) at least one additive, in particular at least one flow agent and/or at least one lubricant,
in particular, wherein the second phase, in particular the second layer, at least essentially no disintegrant and/or disintegrating agent as defined in claim 9, in particular at least essentially no disintegrant and/or disintegrating agent, and/or is at least essentially free of a disintegrant and/or disintegrating agent as defined in claim 9, in particular at least essentially free of a disintegrant and/or disintegrating agent.

11. Dosage form according to one of the preceding claims,
wherein the first phase, in particular the first layer, comprises, in addition to the NSAID active ingredient, in particular ibuprofen, preferably granulated ibuprofen,
(i) at least one disintegrant and/or dispersing agent in the form of croscarmellose sodium and/or polyvinylpolypyrrolidone (crospovidone), preferably polyvinylpoly-pyrrolidone (crospovidone), in a (relative) amount in the range of 3 wt.% to 90 wt.%, preferably in the range of 15 wt.% to 25 wt.%, based on the first phase (dry weight), in particular on the first layer (dry weight),
(ii) at least one disintegration-promoting filler and/or carrier based on sugar alcohols, preferably mannitol, in a (relative) amount in the range of 5 wt.% to 90 wt.%, preferably in the range of 30 wt.% to 45 wt.%, based on the first phase (dry weight), in particular on the first layer (dry weight),
(iii) at least one acidulant in the form of an organic acid or its salts and esters, preferably citric acid, in a (relative) amount in the range of 0.01 wt.% to 10 wt.%, preferably in the range of 0.5 wt.% to 2.5 wt.%, based on the first phase (dry weight), in particular on the first layer (dry weight),
(iv) at least one flavor-modifying and/or flavor-masking agent, in particular in the form of alkaline earth and/or alkali lauryl sulfate, preferably sodium lauryl sulfate, in a (relative) amount in the range of 0.02 wt.% to 10 wt.%, preferably in the range of 0.08 wt.% to 2 wt.%, based on the first phase (dry weight), in particular on the first layer (dry weight),
(v) optionally, at least one additive, in particular at least one flow agent and/or at least one lubricant,
and
wherein the second phase, in particular the second layer, comprises, in addition to the local anesthetic, in particular lidocaine hydrochloride,
(i) at least one non-disintegration-promoting filler and/or carrier based on sugar alcohols, preferably sorbitol and/or isomalt, in a (relative) amount in the range of 10 wt.% to 98 wt.%, preferably in the range of 30 wt.% to 85 wt.%, based on the second phase (dry weight), in particular on the second layer (dry weight),
(ii) at least one acidulant in the form of an organic acid or its salts and esters, preferably citric acid, in a (relative) amount in the range of 0.01 wt.% to 10 wt.%, preferably in the range of 0.5 wt.% to 2.5 wt.%, based on the second phase (dry weight), in particular on the second layer (dry weight),
(iii) at least one flavor-modifying and/or flavor-masking agent, in particular in the form of cyclodextrin, in a (relative) amount in the range of 0.01 wt.% to 25 wt.%, preferably in the range of 0.2 wt.% to 10 wt.%, based on the second phase (dry weight), in particular on the second layer (dry weight),
(v) optionally, at least one additive, in particular at least one flow agent and/or at least one lubricant,
wherein the first phase, in particular the first layer, comprises the NSAID active ingredient in a (relative) amount in the range of 5 wt.% to 90 wt.%, preferably in the range of 20 wt.% to 30 wt.%, calculated as ibuprofen and based on the first phase (dry weight), in particular on the first layer (dry weight), and
wherein the second phase, in particular the second layer, contains the local anesthetic in a (relative) amount in the range of 0.1 wt.% to 20 wt.%%, preferably in the range of 0.5 wt.% to 1.5 wt.%, calculated as lidocaine and based on the second phase (dry weight), in particular on the second layer (dry weight);
in particular, wherein the second phase, in particular the second layer, at least essentially no disintegrant and/or disintegrating agent as defined in claim 9, in particular at least essentially no disintegrant and/or disintegrating agent, and/or is at least essentially free of a disintegrant and/or disintegrating agent as defined in claim 9, in particular at least essentially free of a disintegrant and/or disintegrating agent.

12. Dosage form according to one of the preceding claims,
wherein the dosage form is designed as a multiphase, in particular at least two-phase, preferably two-phase tablet, particularly preferably as a two-layer tablet,
wherein the multiphase tablet, preferably a two-layer tablet, comprises or consists of at least two different phases, in particular at least two different layers, with different disintegration times and/or active ingredient release rates upon oral administration,
- wherein a first phase, in particular a first layer, is designed as an orodispersible phase (melting tablet phase), in particular an orodispersible layer (melting tablet layer), with rapid and/or immediate (instant) disintegration and/or active ingredient release, wherein the first phase, in particular the first layer, comprises at least one NSAID active ingredient (non-steroidal anti-inflammatory drug), wherein the NSAID active ingredient is selected from the group of ibuprofen (2-(4-isobutylphenyl)propionic acid) and its pharmaceutically acceptable salts and esters, preferably ibuprofen (2-(4-isobutylphenyl)propionic acid),
wherein the first phase, preferably the first layer, contains the NSAID active ingredient in the form of preferably compressed granules, in particular wherein the granules have a coating, in particular a water-soluble coating, preferably a cellulose-based coating, preferably based on hydroxypropyl methylcellulose (HPMC),
- wherein a second phase, in particular a second layer, is formed as an oral transmucosal phase (lozenge phase), in particular an oral transmucosal layer (lozenge layer), with slow and/or delayed (controlled) disintegration and/or release of the active ingredient, wherein the second phase, in particular the second layer, comprises at least one local anesthetic, wherein the local anesthetic is selected from the group consisting of lidocaine (2-diethylamino-N-(2,6-dimethylphenyl)acetamide) and its pharmaceutically acceptable salts and esters, preferably lidocaine hydrochloride.

13. Dosage form, in particular pharmaceutical dosage form, according to one of claims 1 to 12, in particular for oral application, for use in the prophylactic and/or therapeutic treatment of, in particular, inflammatory diseases of the mouth/throat area accompanied by pain.

14. Method for producing a dosage form, in particular a pharmaceutical dosage form, according to one of claims 1 to 13, in particular for oral administration, preferably for use in the prophylactic and/or therapeutic treatment of, in particular, inflammatory diseases of the mouth/throat region accompanied by pain,
wherein the dosage form is designed as a multiphase, in particular at least two-phase, preferably two-phase tablet, particularly preferably as a two-layer tablet,
wherein the multiphase tablet, preferably a two-layer tablet, comprises or consists of at least two phases that are different from each other, in particular at least two layers that are different from each other, with different disintegration times and/or active ingredient release rates upon oral administration,
- wherein a first phase, in particular a first layer, comprises an NSAID (non-steroidal anti-inflammatory drug) active ingredient, wherein the NSAID active ingredient is selected from the group consisting of ibuprofen (2-(4-isobutylphenyl)propionic acid) and its pharmaceutically acceptable salts and esters, preferably ibuprofen (2-(4-isobutylphenyl)propionic acid),
- wherein a second phase, in particular a second layer, comprises at least one local anesthetic, wherein the local anesthetic is selected from the group consisting of lidocaine (2-diethylamino-N-(2,6-dimethylphenyl)acetamide) and its pharmaceutically acceptable salts and esters, preferably lidocaine hydrochloride,
wherein the dosage form is produced with the proviso
that the first phase, in particular the first layer, is formed as an orodispersible phase (melting tablet phase), in particular an orodispersible layer (melting tablet layer), with rapid and/or immediate (instant) disintegration and/or release of the active ingredient, and
that the second phase, in particular the second layer, is designed as an oral transmucosal phase (lozenge phase), in particular an oral transmucosal layer (lozenge layer), with slow and/or delayed (controlled) disintegration and/or release of the active ingredient;
in particular wherein, the preparation of the dosage form is also carried out with the further proviso that at least one of the following features and/or measures (i), (ii), (iii) is carried out, in particular at least feature and/or measure (i), preferably at least features and/or measures (i) and (ii), preferably features and/or measures (i), (ii) and (iii):
(i) The first phase, in particular the first layer, is equipped with at least one disintegrant and/or disintegrating agent, and the second phase, in particular the second layer, does not contain any disintegrant and/or disintegrating agent, or
the first phase, in particular the first layer, and also the second phase, in particular the second layer, are each equipped with at least one disintegrant and/or disintegrating agent, but with the proviso that a smaller amount of disintegrant and/or disintegrating agent is used in the second phase, in particular the second layer, than in the first phase, in particular the first layer, in particular as defined above;
(ii) the first phase, in particular the first layer, and the second phase, in particular the second layer, are in particular independently of one another and/or separately from one another and/or sequentially, each individually produced by pressing, preferably by pressing the active ingredients and constituents forming the respective phase, in particular layer, wherein the second phase, in particular the second layer, is produced with a greater pressing force ([N]) and/or with a greater pressing pressure ([N/mm²]) than the first phase, in particular the first layer, and/or pressed, in particular as defined above;
(iii) the NSAR active ingredient, preferably ibuprofen, is used in the form of a granulate, in particular wherein the granulate has a coating, in particular a water-soluble coating, preferably a cellulose-based coating, preferably based on hydroxypropyl methylcellulose (HPMC).

15. Method according to claim 14,
wherein the first phase, in particular the first layer, contains the disintegrant and/or disintegrating agent, in particular polyvinylpolypyrrolidone (crospovidone), in a (relative) amount in the range of 3 wt.% to 90 wt.%, in particular in the range of 5 wt.% to 70 wt.%, preferably in the range of 10 wt.% to 50 wt.%, preferably in the range of 15 wt.% to 25 wt.%, based on the first phase (dry weight), in particular on the first layer (dry weight); and/or
wherein, in the event that the first phase, in particular the first layer, and also the second phase, in particular the second layer, are each equipped with the disintegrant and/or disintegrating agent, the amount of disintegrant and/or disintegrating agent in the second phase, in particular the second layer, is at most 60%, in particular a maximum of 40%, preferably a maximum of 20%, preferably a maximum of 10%, of the absolute amount of disintegrant and/or disintegrating agent in the first phase, in particular the first layer, and/or is set to the aforementioned values; and/or
wherein the ratio of the pressing force ([N]) of the second phase, in particular the second layer, to the pressing force ([N]) of the first phase, in particular the first layer, is in the range of 1.05 : 1 to 20 : 1, in particular in the range of 1.1 : 1 to 10 : 1, preferably in the range of 1.2 : 1 to 6 : 1, preferably in the range of 1.5 : 1 to 4 : 1, and/or is set to the aforementioned values; and/or
wherein the ratio of the pressing pressure ([N/mm²]) of the second phase, in particular the second layer, to the pressing pressure ([N/mm²]) of the first phase, in particular the first layer, is in the range of 1.05 : 1 to 20 : 1, in particular in the range from 1.1 : 1 to 10 : 1, preferably in the range from 1.2 : 1 to 6 : 1, preferably in the range from 1.5 : 1 to 4 : 1, and/or is set to the aforementioned values; and/or
wherein the second phase, in particular the second layer, is first produced and/or compressed and wherein the first phase, in particular the first layer, is subsequently produced and/or compressed, in particular wherein the first phase, in particular the first layer, is produced and/or compressed on the second phase, in particular on the second layer, preferably on one of the base surfaces of the second phase, in particular the second layer; and/or
wherein the dosage form, in particular the tablet, is obtained by subsequently joined (final) compressing of the previously produced and/or compressed first phase, in particular first layer, and of the previously produced and/or compressed second phase, in particular second layer; and/or
wherein the granulate has a mean particle size D₅₀ in the range from 5 µm to 500 µm, in particular in the range from 10 µm to 200 µm, preferably in the range from 50 µm to 100 µm, preferably in the range from 60 µm to 85 µm, in particular determined by laser diffractometry, in particular according to Ph. Eur., 10th edition, method 2.9.31.

## Revendications

1. Forme galénique, en particulier forme galénique pharmaceutique, en particulier pour l'administration orale, de préférence pour une utilisation dans le traitement prophylactique et/ou thérapeutique, en particulier des maladies inflammatoires de la cavité bucco-pharyngée, en particulier accompagnées de douleurs,
la forme galénique étant conçue comme un comprimé multiphase, en particulier au moins biphase, de préférence biphase, de manière particulièrement préférée comme comprimé à deux couches,
le comprimé multiphase, de préférence le comprimé à deux couches, comportant ou étant constitué d'au moins deux phases différentes l'une de l'autre, en particulier d'au moins deux couches différentes l'une de l'autre, avec des temps de désagrégation et/ou des vitesses de libération du principe actif différents l'un de l'autre lors de l'administration orale,
- une première phase, en particulier une première couche, étant conçue comme une phase orodispersible (phase de comprimé à dissolution rapide), en particulier une couche orodispersible (couche de comprimé à dissolution rapide), avec une désintégration et/ou une libération du principe actif rapide et/ou immédiate (instantanée), la première phase, en particulier la première couche, contenant au moins un principe actif AINS (anti-inflammatoire non stéroïdien), le principe actif AINS étant choisi dans le groupe de l'ibuprofène (acide 2-(4-isobutylphényl)propionique) ainsi que ses sels et esters pharmaceutiquement acceptables, de préférence l'ibuprofène (acide 2-(4-isobutylphényl)propionique),
- une deuxième phase, en particulier une deuxième couche, étant formée en tant que phase transmuqueuse orale (phase de comprimé à sucer), en particulier une couche transmucosale orale (couche de comprimé à sucer), avec une désintégration et/ou une libération de principe actif lente et/ou retardée (contrôlée), la deuxième phase, en particulier la deuxième couche, contenant au moins un anesthésique local, l'anesthésique local étant choisi parmi le groupe de la lidocaïne (2-diéthylamino-N-(2,6-diméthylphényl)acétamide) ainsi que ses sels et esters pharmaceutiquement acceptables, de préférence le chlorhydrate de lidocaïne.

2. Forme galénique selon la revendication 1,
la forme galénique étant conçue comme un comprimé à deux couches,
le comprimé à deux couches comportant ou étant constitué de deux couches différentes l'une de l'autre, ayant des vitesses de libération différentes l'une de l'autre lors d'une administration par voie orale,
- une première couche étant conçue comme une couche orodispersible (couche de comprimé à dissolution rapide) avec une désintégration et/ou une libération du principe actif rapide et/ou immédiate (instantanée), la première couche contenant au moins un principe actif AINS (anti-inflammatoire non stéroïdien), le principe actif AINS étant choisi dans le groupe de l'ibuprofène (acide 2-(4-isobutylphényl)propionique) ainsi que ses sels et esters pharmaceutiquement acceptables, de préférence l'ibuprofène (acide 2-(4-isobutylphényl)propionique),
- une deuxième couche étant conçue comme une couche transmucosale orale (couche de comprimé à sucer) à désintégration et/ou libération de principe actif lente et/ou retardée (contrôlée), la deuxième couche comportant au moins un anesthésique local, l'anesthésique local étant choisi parmi le groupe comprenant la lidocaïne (2-diéthylamino-N-(2,6-diméthylphényl)acétamide) ainsi que de ses sels et esters pharmaceutiquement acceptables, de préférence le chlorhydrate de lidocaïne.

3. Forme galénique selon la revendication 1 ou la revendication 2,
dans laquelle le principe actif AINS est l'ibuprofène et l'anesthésique local est le chlorhydrate de lidocaïne; et/ou
la première phase, en particulier la première couche, contenant de l'ibuprofène comme principe actif AINS et la deuxième phase, en particulier la deuxième couche, contenant du chlorhydrate de lidocaïne comme anesthésique local; et/ou
dans laquelle la première phase, en particulier la première couche, ne contient pas d'autre principe actif AINS que l'ibuprofène et dans laquelle la deuxième phase, en particulier la deuxième couche, ne contient pas d'autre anesthésique local que le chlorhydrate de lidocaïne; et/ou
la substance active AINS étant le lysinate d'ibuprofène.

4. Forme galénique selon l'une des revendications précédentes,
la forme galénique, en particulier la première phase, de préférence la première couche, contenant le principe actif AINS en une quantité (absolue) comprise entre 50 mg et 800 mg, en particulier comprise entre 100 mg et 400 mg, de préférence comprise entre 150 mg et 300 mg, de préférence comprise entre 190 mg et 210 mg, calculée en ibuprofène; et/ou
la forme galénique, en particulier la deuxième phase, de préférence la deuxième couche, contenant l'anesthésique local en une quantité (absolue) comprise entre 2 mg et 50 mg, en particulier entre 4 mg et 20 mg, de préférence entre 5 mg et 10 mg, de préférence entre 6 mg et 9 mg, calculée en lidocaïne.

5. Forme galénique selon l'une des revendications précédentes,
dans laquelle le principe actif AINS n'est présent au moins essentiellement que dans la première phase, en particulier dans la première couche; et/ou
la deuxième phase, en particulier la deuxième couche, ne contenant au moins pour l'essentiel aucun principe actif AINS et/ou étant au moins pour l'essentiel exempte du principe actif AINS; et/ou
l'anesthésique local n'étant présent au moins essentiellement que dans la deuxième phase, en particulier dans la deuxième couche; et/ou
la première phase, en particulier la première couche, ne contenant au moins pour l'essentiel aucun anesthésique local et/ou étant au moins pour l'essentiel exempte de l'anesthésique local.

6. Forme galénique selon l'une des revendications précédentes,
la première phase, de préférence la première couche, contenant le principe actif AINS sous forme de granulés, en particulier à base de particules et/ou de particules de préférence discrètes, et/ou le principe actif AINS, de préférence l'ibuprofène, se présente sous forme de granulés et/ou est utilisé et/ou se présente sous forme de granulés, en particulier à base de particules et/ou de grains de préférence discrets;
en particulier les granulés ayant une taille moyenne de particules D₅₀ comprise entre 5 µm et 500 µm, en particulier comprise entre 10 µm et 200 µm, de préférence comprise entre 50 µm et 100 µm, de préférence comprise entre 60 µm et 85 µm, déterminée en particulier par diffractométrie laser, en particulier selon la Ph. Eur., 10e édition, méthode 2.9.31; et/ou
en particulier, les granulés, en particulier les particules individuelles et/ou les particules des granulés, présentent un enrobage (coating), en particulier un enrobage hydrosoluble, de préférence un enrobage à base de cellulose, de préférence à base d'hydroxypropylméthylcellulose (HPMC), et/ou les granulés étant utilisés et/ou se présentant sous forme enrobée, en particulier l'enrobage étant un enrobage hydrosoluble, de préférence à base de cellulose, de préférence à base d'hydroxypropylméthylcellulose (HPMC);
en particulier, les granulés, en particulier les différentes particules et/ou particules des granulés, sont au moins essentiellement entièrement enrobés et/ou au moins essentiellement entièrement recouverts par l'enrobage.

7. Forme galénique selon l'une des revendications précédentes,
la première phase, en particulier la première couche, ayant un temps de désagrégation, en particulier déterminé selon la Ph. Eur., 10e édition, méthode 2.9.1, compris entre 1 s et 120 s, en particulier compris entre 5 s et 60 s, de préférence compris entre 10 s et 30 s; et/ou
la deuxième phase, en particulier la deuxième couche, présentant un temps de désintégration, déterminé en particulier selon la Ph. Eur., 10e édition, méthode 2.9.1, compris entre 3 min et 30 min, en particulier compris entre 5 min et 20 min, de préférence compris entre 8 min et 15 min.

8. Forme galénique selon l'une des revendications précédentes,
le rapport entre le temps de désintégration de la deuxième phase, en particulier de la deuxième couche, et le temps de désintégration de la première phase, en particulier de la première couche, étant compris entre 1,5 : 1 à 1 500 : 1, en particulier dans la plage de 5 : 1 à 500 : 1, de préférence dans la plage de 10 : 1 à 100 : 1, les temps de désintégration étant notamment déterminés selon la Ph. Eur., 10e édition, méthode 2.9.1; et/ou
le rapport entre la vitesse de libération du principe actif de la première phase, en particulier de la première couche, et la vitesse de libération du principe actif de la deuxième phase, en particulier de la deuxième couche, étant compris entre 50 : 1 et 10 000 : 1, en particulier entre 100 : 1 et 5 000 : 1, de préférence entre 500 : 1 à 2 000 : 1, en particulier les vitesses de libération du principe actif étant déterminées selon la Ph. Eur., 10e édition, méthode 2.9.3.

9. Forme galénique selon l'une des revendications précédentes,
dans laquelle la première phase, en particulier la première couche, de préférence uniquement et/ou exclusivement la première phase, en particulier la première couche, comprend au moins un agent désintégrant et/ou désagrégeant et/ou en est pourvue;
en particulier, l'agent désintégrant et/ou désagrégeant de la première phase, en particulier de la première couche, étant choisi dans le groupe constitué par la polyvinylpolypyrrolidone (crospovidone); celluloses, en particulier celluloses microcristallines, méthylcelluloses, sel de croscarmellose, de préférence croscarmellose alcaline et alcalino-terreux, de préférence croscarmellose sodique; amidons, en particulier amidon de maïs, amidon de pomme de terre, amidons pré-gélatinisés; l'acide alginique et ses sels, en particulier les sels alcalins et alcalino-terreux, de préférence l'alginate de calcium et l'alginate de sodium; les amidons carboxyalkyles et leurs sels, en particulier les amidons carboxyalkyles alcalins et alcalino-terreux, de préférence l'amidon carboxyméthylé sodique; hydrogénocarbonate alcalin et alcalino-terreux, de préférence hydrogénocarbonate de sodium, en particulier en combinaison avec au moins un acide de préférence organique, de préférence hydrogénocarbonate de sodium en combinaison avec de l'acide citrique et/ou de l'acide tartrique; substances tensioactives, en particulier pour l'hydrophilisation; et leurs mélanges ou combinaisons, de préférence la polyvinylpolypyrrolidone (crospovidone) et/ou la croscarmellose sodique; et/ou
en particulier, l'agent désintégrant et/ou désagrégeant de la première phase, en particulier de la première couche, étant la croscarmellose alcaline ou alcalino-terreux, en particulier la croscarmellose sodique, et/ou la polyvinylpolypyrrolidone (crospovidone), de préférence la polyvinylpolypyrrolidone (crospovidone); et/ou
en particulier, la première phase, en particulier la première couche, l'agent désintégrant et/ou désagrégeant, en particulier la polyvinylpolypyrrolidone (crospovidone), étant présente en une quantité (absolue) comprise entre 20 mg et 750 mg, en particulier comprise entre 50 mg et 350 mg, de préférence comprise entre 100 mg et 250 mg, de préférence comprise entre 140 mg et 180 mg; et/ou
en particulier, la forme galénique, en particulier la première phase, de préférence la première couche, contenant l'agent désintégrant et/ou désagrégeant, en particulier la polyvinylpolypyrrolidone (crospovidone), en une quantité (relative) comprise entre 2 % en poids et 45 % en poids, en particulier comprise entre 3 % en poids et 35 % en poids, de préférence comprise entre 4 % et 25 % en poids, de préférence comprise entre 5 % et 15 % en poids, par rapport à la forme galénique (poids sec); et/ou
en particulier, la première phase, en particulier la première couche, contenant l'agent désintégrant et/ou désagrégeant, en particulier la polyvinylpolypyrrolidone (crospovidone), en une quantité (relative) comprise entre 3 % et 90 % en poids, en particulier dans la plage de 5 % en poids à 70 % en poids, de préférence dans la plage de 10 % en poids à 50 % en poids, de préférence dans la plage de 15 % en poids à 25 % en poids, par rapport à la première phase (poids sec), en particulier par rapport à la première couche (poids sec); et/ou
en particulier, l'agent désintégrant et/ou désagrégeant, en particulier la polyvinylpolypyrrolidone (crospovidone), n'étant présent au moins essentiellement que dans la première phase, en particulier dans la première couche.

10. Forme galénique selon l'une des revendications précédentes,
la première phase, en particulier la première couche, contenant, outre le principe actif AINS, en particulier l'ibuprofène, de préférence sous forme de granulés, (i) au moins un agent désintégrant et/ou désagrégant, en particulier tel que défini dans la revendication 9, (ii) au moins un agent de remplissage et/ou un excipient favorisant la désagrégation, (iii) au moins un agent acidifiant, (iv) au moins un agent modifiant le goût et/ou masquant le goût, ainsi que, le cas échéant, (v) au moins un additif, en particulier au moins un agent fluidifiant et/ou au moins un lubrifiant, et
la deuxième phase, en particulier la deuxième couche, contenant, outre l'anesthésique local, en particulier le chlorhydrate de lidocaïne, (i) au moins un agent de remplissage et/ou un excipient ne favorisant pas la désintégration, (ii) au moins un agent acidifiant, (iii) au moins un agent modifiant le goût et/ou masquant le goût, ainsi que, le cas échéant, (v) au moins un additif, en particulier au moins un agent fluidifiant et/ou au moins un lubrifiant,
en particulier la deuxième phase, en particulier la deuxième couche, ne contient au moins pour l'essentiel aucun agent désintégrant et/ou désagrégeant tel que défini dans la revendication 9, en particulier au moins pour l'essentiel aucun agent désintégrant et/ou désagrégeant, et/ou est au moins pour l'essentiel exempte d'un agent désintégrant et/ou désagrégeant tel que défini dans la revendication 9, en particulier au moins pour l'essentiel exempte d'un agent désintégrant et/ou désagrégeant.

11. Forme galénique selon l'une des revendications précédentes,
dans laquelle la première phase, en particulier la première couche, contient, outre le principe actif AINS, en particulier l'ibuprofène, de préférence sous forme de granulés,
(i) au moins un agent désintégrant et/ou désagrégeant sous forme de croscarmellose sodique et/ou de polyvinylpolypyrrolidone (crospovidone), de préférence de polyvinylpoly-pyrrolidone (crospovidone), en une quantité (relative) comprise entre 3 % en poids et 90 % en poids, de préférence comprise entre 15 % et 25 % en poids, par rapport à la première phase (poids sec), en particulier par rapport à la première couche (poids sec),
(ii) au moins un agent de remplissage et/ou un excipient favorisant la désagrégation à base d'alcools de sucre, de préférence de mannitol, en une quantité (relative) comprise entre 5 % et 90 % en poids, de préférence entre 30 % et 45 % en poids, par rapport à la première phase (poids sec), en particulier par rapport à la première couche (poids sec),
(iii) au moins un agent acidifiant sous forme d'un acide organique ou de ses sels et esters, de préférence l'acide citrique, en une quantité (relative) comprise entre 0,01 % en poids et 10 % en poids, de préférence entre 0,5 % en poids et 2,5 % en poids, par rapport à la première phase (poids sec), en particulier par rapport à la première couche (poids sec),
(iv) au moins un agent modificateur et/ou masquant le goût, en particulier sous la forme d'un sulfate d'alcalino-terreux et/ou alcalin lauryl, de préférence du laurylsulfate de sodium, en une quantité (relative) comprise entre 0,02 % en poids et 10 % en poids, de préférence comprise entre 0,08 % en poids et 2 % en poids, par rapport à la première phase (poids sec), en particulier à la première couche (poids sec),
(v) le cas échéant, au moins un additif, en particulier au moins un agent fluidifiant et/ou au moins un lubrifiant,
et
la deuxième phase, en particulier la deuxième couche, contenant, outre l'anesthésique local, en particulier le chlorhydrate de lidocaïne,
(i) au moins un agent de remplissage et/ou un excipient non favorisant la désintégration à base d'alcools de sucre, de préférence de sorbitol et/ou d'isomalt, en une quantité (relative) comprise entre 10 % en poids et 98 % en poids%, de préférence comprise entre 30 % en poids et 85 % en poids, par rapport à la deuxième phase (poids sec), en particulier par rapport à la deuxième couche (poids sec),
(ii) au moins un agent acidifiant sous forme d'un acide organique ou de ses sels et esters, de préférence l'acide citrique, en une quantité (relative) comprise entre 0,01 % en poids et 10 % en poids, de préférence entre 0,5 % en poids et 2,5 % en poids, par rapport à la deuxième phase (poids sec), en particulier par rapport à la deuxième couche (poids sec),
(iii) au moins un agent modificateur et/ou masquant le goût, en particulier sous forme de cyclodextrine, en une quantité (relative) comprise entre 0,01 % en poids et 25 % en poids, de préférence entre 0,2 % en poids et 10 % en poids, par rapport à la deuxième phase (poids sec), en particulier par rapport à la deuxième couche (poids sec),
(v) le cas échéant, au moins un additif, en particulier au moins un agent fluidifiant et/ou au moins un lubrifiant,
la première phase, en particulier la première couche, contenant le principe actif AINS en une quantité (relative) comprise entre 5 % en poids et 90 % en poids, de préférence comprise entre 20 % en poids et 30 % en poids, calculée en ibuprofène et rapportée à la première phase (poids sec), en particulier à la première couche (poids sec), et
la deuxième phase, en particulier la deuxième couche, contenant l'anesthésique local en une quantité (relative) comprise entre 0,1 % en poids et 20 % en poids%, de préférence dans la plage de 0,5 % en poids à 1,5 % en poids, calculée en tant que lidocaïne et par rapport à la deuxième phase (poids sec), en particulier par rapport à la deuxième couche (poids sec);
en particulier, la deuxième phase, en particulier la deuxième couche, ne contient au moins essentiellement aucun l'agent désintégrant et/ou désagrégeant, tel que défini dans la revendication 9, en particulier au moins essentiellement aucun l'agent désintégrant et/ou désagrégeant,, et/ou est au moins essentiellement exempte d'un l'agent désintégrant et/ou désagrégeant, tel que défini dans la revendication 9, en particulier au moins essentiellement exempte d'un l'agent désintégrant et/ou désagrégeant,.

12. Forme galénique selon l'une des revendications précédentes,
la forme galénique étant conçue comme un comprimé multiphase, en particulier au moins biphase, de préférence biphase, de manière particulièrement préférée comme un comprimé à deux couches,
le comprimé multiphase, de préférence à deux couches, comportant au moins deux phases différentes, en particulier au moins deux couches différentes, ayant des temps de désagrégation et/ou des vitesses de libération du principe actif différents lors de l'administration par voie orale, ou étant constitué de celles-ci,
- une première phase, en particulier une première couche, étant conçue comme une phase orodispersible (phase de comprimé à dissolution rapide), en particulier une couche orodispersible (couche de comprimé à dissolution rapide), avec une désintégration et/ou une libération du principe actif rapide et/ou immédiate (instantanée), la première phase, en particulier la première couche, contenant au moins un principe actif AINS (anti-inflammatoire non stéroïdien), le principe actif AINS étant choisi dans le groupe de l'ibuprofène (acide 2-(4-isobutylphényl)propionique) ainsi que ses sels et esters pharmaceutiquement acceptables, de préférence l'ibuprofène (acide 2-(4-isobutylphényl)propionique),
la première phase, de préférence la première couche, contenant le principe actif AINS sous forme de granulés de préférence compressés, les granulés comportant en particulier un enrobage (coating), en particulier un enrobage hydrosoluble, de préférence un enrobage à base de cellulose, de préférence à base d'hydroxypropylméthylcellulose (HPMC),
- une deuxième phase, en particulier une deuxième couche, étant formée en tant que phase transmucosale orale (phase de comprimé à sucer), en particulier une couche transmucosale orale (couche de comprimé à sucer), avec une désintégration et/ou une libération de principe actif lente et/ou retardée (contrôlée), la deuxième phase, en particulier la deuxième couche, comportant au moins un anesthésique local, l'anesthésique local étant choisi dans le groupe constitué par la lidocaïne (2-diéthylamino-N-(2,6-diméthylphényl)acétamide) ainsi que ses sels et esters pharmaceutiquement acceptables, de préférence le chlorhydrate de lidocaïne.

13. Forme galénique, en particulier forme galénique pharmaceutique, selon l'une des revendications 1 à 12, en particulier pour l'application orale, destinée à être utilisée dans le traitement prophylactique et/ou thérapeutique de maladies inflammatoires de la cavité bucco-pharyngée, en particulier accompagnées de douleurs.

14. Procédé de fabrication d'une forme galénique, en particulier d'une forme galénique pharmaceutique, selon l'une des revendications 1 à 13, en particulier pour l'administration par voie orale, de préférence pour une utilisation dans le traitement prophylactique et/ou thérapeutique des maladies inflammatoires de la cavité bucco-pharyngée, en particulier accompagnées de douleurs.
la forme galénique étant conçue comme un comprimé multiphase, en particulier au moins biphase, de préférence biphase, de manière particulièrement préférée comme comprimé à deux couches,
le comprimé multiphase, de préférence le comprimé à deux couches, présentant ou étant constitué d'au moins deux phases différentes l'une de l'autre, en particulier d'au moins deux couches différentes l'une de l'autre, avec des temps de désintégration et/ou des vitesses de libération du principe actif différents l'un de l'autre lors de l'administration orale,
- une première phase, en particulier une première couche, présentant un principe actif AINS (anti-inflammatoire non stéroïdien), le principe actif AINS étant choisi dans le groupe de l'ibuprofène (acide 2-(4-isobutylphényl)propionique) ainsi que ses sels et esters pharmaceutiquement acceptables, de préférence l'ibuprofène (acide 2-(4-isobutylphényl)propionique),
- une deuxième phase, en particulier une deuxième couche, contenant au moins un anesthésique local, l'anesthésique local étant choisi parmi le groupe comprenant la lidocaïne (2-diéthylamino-N-(2,6-diméthylphényl)acétamide) ainsi que ses sels et esters pharmaceutiquement acceptables, de préférence le chlorhydrate de lidocaïne,
la fabrication de la forme galénique s'effectuant selon les conditions suivantes
la première phase, en particulier la première couche, est conçue comme une phase orodispersible (phase de comprimé à dissolution rapide), en particulier une couche orodispersible (couche de comprimé à dissolution rapide), avec une désintégration et/ou une libération du principe actif rapide et/ou immédiate (instantanée), et
que la deuxième phase, en particulier la deuxième couche, est conçue comme une phase transmucosale orale (phase de comprimé à sucer), en particulier une couche transmucosale orale (couche de comprimé à sucer), avec une désintégration et/ou une libération du principe actif lentes et/ou retardées (contrôlées);
la fabrication de la forme galénique étant en outre réalisée avec la condition supplémentaire qu'au moins l'une des caractéristiques et/ou mesures suivantes (i), (ii), (iii) est mise en œuvre, en particulier au moins la caractéristique et/ou la mesure (i), de préférence au moins les caractéristiques et/ou mesures (i) et (ii), de préférence les caractéristiques et/ou mesures (i), (ii) et (iii):
(i) la première phase, en particulier la première couche, est équipée d'au moins un agent désintégrant et/ou désagrégeant et la deuxième phase, en particulier la deuxième couche, ne comporte pas d'agent désintégrant et/ou désagrégeant, ou bien
la première phase, en particulier la première couche, et également la deuxième phase, en particulier la deuxième couche, sont chacune équipées d'au moins un agent désintégrant et/ou désagrégeant, à condition toutefois que dans la deuxième phase, en particulier la deuxième couche, une quantité agent désintégrant et/ou désagrégeant inférieure à celle utilisée dans la première phase, en particulier la première couche, soit utilisée, en particulier comme défini précédemment;
(ii) la première phase, en particulier la première couche, et la deuxième phase, en particulier la deuxième couche, sont en particulier indépendamment l'une de l'autre et/ou séparément l'une de l'autre et/ou successivement, sont produites individuellement par compression, de préférence par compression des substances actives et des ingrédients formant la phase respective, en particulier la couche, la deuxième phase, en particulier la deuxième couche, étant produite et/ou comprimée avec une force de compression ([N]) et/ou une pression de compression ([N/mm²]) que la première phase, en particulier la première couche, et/ou pressée, en particulier comme défini précédemment;
(iii) le principe actif AINS, de préférence l'ibuprofène, est utilisé sous forme de granulés, en particulier les granulés comportant un enrobage (coating), en particulier un enrobage hydrosoluble, de préférence un enrobage à base de cellulose, de préférence à base d'hydroxypropylméthylcellulose (HPMC).

15. Procédé selon la revendication 14,
dans laquelle la première phase, en particulier la première couche, contient l'agent désintégrant et/ou désagrégant, en particulier la polyvinylpolypyrrolidone (crospovidone), en une quantité (relative) comprise entre 3 % en poids et 90 % en poids%, en particulier comprise entre 5 % en poids et 70 % en poids, de préférence comprise entre 10 % en poids et 50 % en poids, de préférence comprise entre 15 % en poids et 25 % en poids, par rapport à la première phase (poids sec), en particulier par rapport à la première couche (poids sec); et/ou
dans le cas où la première phase, en particulier la première couche, et également la deuxième phase, en particulier la deuxième couche, sont chacune équipées de agent désintégrant et/ou désagrégeant, la quantité agent désintégrant et/ou désagrégeant dans la deuxième phase, en particulier la deuxième couche, est au maximum de 60 %, en particulier au maximum 40 %, de préférence au maximum 20 %, de préférence au maximum 10 %, de la quantité absolue d'agent désintégrant et/ou désagrégeant dans la première phase, en particulier la première couche, et/ou est ajustée aux valeurs susmentionnées; et/ou
le rapport entre la force de compression ([N]) de la deuxième phase, en particulier de la deuxième couche, et la force de compression ([N]) de la première phase, en particulier de la première couche, étant compris entre 1,05 : 1 et 20 : 1, en particulier entre 1,1 : 1 et 10 : 1, de préférence entre 1,2 : 1 et 6 : 1, de préférence dans la plage de 1,5 : 1 à 4 : 1, et/ou est ajustée aux valeurs susmentionnées; et/ou
le rapport entre la pression de compression ([N/mm²]) de la deuxième phase, en particulier de la deuxième couche, et la pression de compression ([N/mm²]) de la première phase, en particulier de la première couche, étant compris entre 1,05 : 1 à 20 : 1, en particulier dans la plage de 1,1 : 1 à 10 : 1, de préférence dans la plage de 1,2 : 1 à 6 : 1, de préférence dans la plage de 1,5 : 1 à 4 : 1, et/ou est ajustée aux valeurs susmentionnées; et/ou
la deuxième phase, en particulier la deuxième couche, étant d'abord fabriquée et/ou comprimée, puis la première phase, en particulier la première couche, étant fabriquée et/ou comprimée, la première phase, en particulier la première couche, étant notamment fabriquée et/ou comprimée sur la deuxième phase, en particulier sur la deuxième couche, de préférence sur l'une des surfaces de base de la deuxième phase, en particulier de la deuxième couche; et/ou
la forme galénique, en particulier le comprimé, étant obtenue par compression (finale) conjointe de la première phase, en particulier de la première couche, préalablement fabriquée et/ou comprimée, et de la deuxième phase, en particulier de la deuxième couche, préalablement fabriquée et/ou comprimée; et/ou
les granulés ayant une taille moyenne de particules D₅₀ comprise entre 5 µm et 500 µm, en particulier comprise entre 10 µm et 200 µm, de préférence comprise entre 50 µm et 100 µm, de préférence comprise entre 60 µm et 85 µm, déterminée en particulier par diffractométrie laser, en particulier selon la Ph. Eur., 10e édition, méthode 2.9.31.
